# FASCICULE DE BREVET EUROPEEN

(11) **EP 0 991 654 B1**
(45) Date de publication et mention de la délivrance du brevet: **15.06.2005**
(21) Numéro de dépôt: 98932205.2
(22) Date de dépôt: 15.06.1998
(51) Int. Cl.: C07D 521/00, A61K 31/38, A61K 31/425, A61K 31/445, A61K 31/415, C07D 417/12, C07D 277/04, C07D 409/12, C07D 211/70, C07D 333/70, C07D 277/06, C07D 307/68, C07D 233/78, C07D 277/14, C07D 207/16

(54) **NOUVEAUX DERIVES DU 2-(IMINOMETHYL)AMINO-PHENYLE, LEUR PREPARATION, LEUR APPLICATION A TITRE DE MEDICAMENTS ET LES COMPOSITIONS PHARMACEUTIQUES LES CONTENANT**
2-(IMINOMETHYL)AMINOPHENYL-DERIVATE, DEREN HERSTELLUNG, VERWENDUNG ALS MEDIKAMENTE UND DIESE ENTHALTENDE PHARMAZEUTISCHE ZUBEREITUNGEN
NOVEL 2-(IMINOMETHYL)AMINO-PHENYL DERIVATIVES, PREPARATION, APPLICATION AS MEDICINES AND COMPOSITIONS CONTAINING SAME

(30) Priorité: 20.06.1997 FR 9707701
(43) Date de publication de la demande: 12.04.2000
(73) Titulaire: SOCIETE DE CONSEILS DE RECHERCHES ET D'APPLICATIONS SCIENTIFIQUES (S.C.R.A.S.), 75016 Paris (FR)
(72) Inventeur: AUVIN, Serge, F-91240 Saint-Michel-sur-Orge (FR); HARNETT, Jeremiah, F-91190 Gif-sur-Yvette (FR); BIGG, Dennis, F-91190 Gif-sur-Yvette (FR); CHABRIER DE LASSAUNIERE, Pierre-Etienne, F-75016 Paris (FR)
(74) Mandataire: Bourgouin, André
(86) Numéro de dépôt international: PCT/FR1998/001250
(87) Numéro de publication internationale: WO 1998/058934

(56) Documents cités:
- WO-A-93/13055
- WO-A-95/05363
- WO-A-96/01817
- DATABASE XFIRE BS9703PR Beilstein Informationssysteme GmbH Frankfurt DE, XP002058022 & ROBINSON ET AL.: JOURNAL OF THE CHEMICAL SOCIETY.,1931, page 980 LETCHWORTH GB
- DATABASE XFIRE BS9703PR Beilstein Informationssysteme GmbH Frankfurt DE, XP002058023 & V.G. ERMOLAEVA ET AL.: JOURNAL OF GENERAL CHEMISTRY USSR., vol. 33, 1963, pages 2646-2649, NEW YORK US
- DATABASE XFIRE BS9703PR Beilstein Informationssysteme GmbH Frankfurt DE, XP002058024 & ROUT: JOURNAL OF THE INDIAN CHEMICAL SOCIETY, vol. 33, 1956, pages 690-692,
- DATABASE XFIRE BS9703PR Beilstein Informationssysteme GmbH Frankfurt DE, XP002058025 & BALABAN ET AL.: JOURNAL OF THE CHEMICAL SOCIETY., vol. 127, 1925, page 2711 LETCHWORTH GB
- DATABASE XFIRE BS9703PR Beilstein Informationssysteme GmbH Frankfurt DE, XP002058026 & GRANT ET AL.: JOURNAL OF THE CHEMICAL SOCIETY., vol. 119, 1921, page 1899 LETCHWORTH GB
- DATABASE XFIRE BS9703PR Beilstein Informationssysteme GmbH Frankfurt DE, XP002058027 & KULEV ET AL.: JOURNAL OF GENERAL CHEMISTRY USSR., vol. 27, 1957, page 1473 NEW YORK US
- DATABASE XFIRE BS9703PR Beilstein Informationssysteme GmbH Frankfurt DE, XP002058028 & SAIKACHI: NIPPON KAGAKU KAISHI, vol. 65, 1944, pages 196-199,
- DATABASE XFIRE BS9703PR Beilstein Informationssysteme GmbH Frankfurt DE, XP002058029 & BALABAN ET AL.: JOURNAL OF THE CHEMICAL SOCIETY., vol. 127, 1925, page 2706 LETCHWORTH GB
- DATABASE XFIRE BS9703PR Beilstein Informationssysteme GmbH Frankfurt DE, XP002058030 & NIKIFOROWA: JOURNAL OF GENERAL CHEMISTRY USSR., vol. 24, 1954, page 1831 NEW YORK US
- DATABASE XFIRE BS9703PR Beilstein Informationssysteme GmbH Frankfurt DE, XP002058031 & KULEV ET AL.: JOURNAL OF GENERAL CHEMISTRY USSR., vol. 27, 1957, page 1473 NEW YORK US
- DATABASE XFIRE BS9703PR Beilstein Informationssysteme GmbH Frankfurt DE, XP002058032 & LELLMANN ET AL.: JUSTUS LIEBIGS ANNALEN DER CHEMIE., vol. 259, 1890, page 41 WEINHEIM DE

## Description

La présente invention a pour objet des nouveaux dérivés du 2-(iminométhyl)aminophényle présentant une activité inhibitrice des enzymes NO-synthases produisant le monoxyde d'azote NO et / ou une activité piégeuse des formes réactives de l'oxygène (ROS pour "*reactive oxygen species*"). L'invention concerne les dérivés correspondant à la formule générale **(I)** définie ci-après, leurs méthodes de préparation, les préparations pharmaceutiques les contenant et leur utilisation à des fins thérapeutiques, en particulier leur utilisation en tant qu'inhibiteur des NO-synthases et piégeur de formes réactives de l'oxygène de manière sélective ou non.

Compte tenu du rôle potentiel du NO et des ROS en physiopathologie, les nouveaux dérivés décrits répondant à la formule générale **(I)** peuvent produire des effets bénéfiques ou favorables dans le traitement de pathologies où ces espèces chimiques sont impliquées. Notamment :
- troubles cardio-vasculaires et cérébro-vasculaires comprenant par exemple l'athérosclérose, la migraine, l'hypertension artérielle, le choc septique, les infarctus cardiaques ou cérébraux d'origine ischémique ou hémorragiques, les ischémies et les thromboses.
- troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d'Alzheimer, la chorée de Huntington, la maladie de Parkinson, la maladie de Creutzfeld Jacob et les maladies à prions, la sclérose latérale amyotrophique mais aussi la douleur, les traumatismes cérébraux ou de la moelle épinière, l'addiction aux opiacées, à l'alcool et aux substances induisant une accoutumance, les troubles de l'érection et de la reproduction, les désordres cognitifs, les encéphalopathies, les encéphalopathies d'origine virale ou toxique.
- troubles du muscle squelettique et des jonctions neuromusculaires (myopathie, myosite) ainsi que les maladies cutanées.
- les maladies prolifératives et inflammatoires comme par exemple l'athérosclérose, l'hypertension pulmonaire, la détresse respiratoire la glomérulonéphrite, l'hypertension portale, le psoriasis, l'arthrose et l'arthrite rhumatoïde, les fibroses, les amyloïdoses, les inflammations du système gastro-intestinal (colite, maladie de Crohn) ou du système pulmonaire et des voies aériennes (asthme, sinusites, rhinites).
- les transplantations d'organes.
- les maladies auto-immunes et virales comme par exemple le lupus, le sida, les infections parasitaires et virales, le diabète, la sclérose en plaques.
- le cancer.
- les maladies neurologiques associées à des intoxications (empoisonnement au Cadmium, inhalation de n-hexane, pesticide, herbicide), à des traitements (radiothérapie) ou à des désordres d'origine génétique (maladie de Wilson).
- toutes les pathologies caractérisées par une production excessive ou un dysfonctionnement de NO et/ou des ROS.

Dans l'ensemble de ces pathologies, il existe des évidences expérimentales démontrant l'implication du NO ou des ROS (*J. Med. Chem.* (1995) **38**, 4343-4362 ; *Free Radic. Biol. Med.* (1996) **20,** 675-705 ; *The Neuroscientist* (1997) **3**, 327-333).

La demande de brevet PCT WO 95/05363 décrit certains dérivés du 2-(iminométhyl)amino-phényle. Les composés de ce document possèdent aussi une activité d'inhibition de la NO synthase et ils sont utilisés comme médicaments notamment dans le traitement des maladies neurodégénératives ou de la migraine.

Par ailleurs les inventeurs ont déjà décrit dans des brevets antérieurs des inhibiteurs de NO Synthases et leur utilisation (brevets US 5,081,148 ; US 5,360,925) et plus récemment l'association de ces inhibiteurs avec des produits possédant des propriétés antioxydantes ou antiradicalaires (demande de brevet non publiée).

Enfin, la demande de brevet WO 98/42696, antérieure à la présente demande mais non publiée à la date de dépôt de ladite demande, décrit des dérivés du 2-(iminométhyl)amino-phényle possédant la double activité d'inhibition des NO-synthases et de piégeage des ROS. Lesdits dérivés répondent à la formule générale **(A1)** 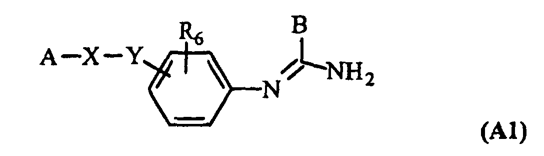 dans laquelle :
A représente notamment:
   - un radical 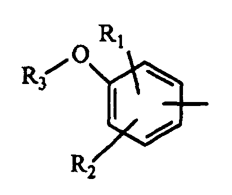
   dans lequel R₁ et R₂ représentent, indépendamment, H, un halogène, le groupe OH, un radical alkyle ou alkoxy ayant de 1 à 6 atomes de carbone,
   R₃ représente H, un radical alkyle ayant de 1 à 6 atomes de carbone ou -COR₄,
   R₄ représentant un radical alkyle ayant de 1 à 6 atomes de carbone ; ou
   - un radical 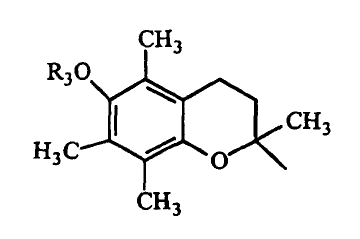
   dans lequel R₃ a la signification indiquée ci-dessus ;
B représente un radical alkyle ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy ayant de 1 à 6 atomes de carbone ;
X représente -Z₁-, -Z₁-CO-, -CH=CH-CO-, -Z₁-NR₃-CO-, -Z₁-NR₃-CS-, -Z₁-NR₃-SO₂- ou une simple liaison ;
Y représente notamment un radical choisi parmi les radicaux pipérazine, homopipérazine, 2-méthylpipérazine, 2,5-diméthyl-pipérazine et 4-aminopipéridine,
Z₁ représente une simple liaison ou un radical alkylène linéaire ou ramifié ayant de 1 à 6 atomes de carbone;
R₆ représente H ou un groupe OH.

La présente invention a pour objet de nouveaux dérivés de 2-(iminométhyl)aminophényle, leur préparation et leur application en thérapeutique.

Les composés de l'invention répondent à la formule générale **(I)** : 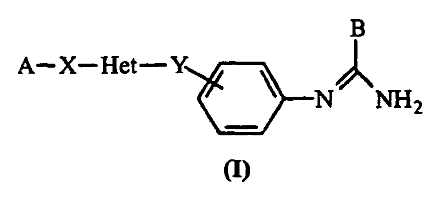 dans laquelle :
A est un aromatique correspondant aux structures : 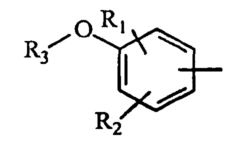
dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
   ou 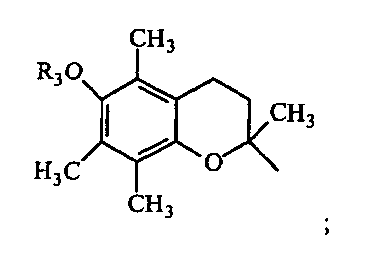
B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
   X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;

Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine.

Les composés de formule générale **(I)** comportant un ou plusieurs centres asymétriques présentent des formes isomères. Les racémiques et les énantiomères de ces composés font également partie de cette invention. De même, les composés de l'invention peuvent aussi exister à l'état de bases ou de sels d'addition à des acides.

L'invention concerne plus particulièrement des composés de formule générale **(I)** pour lesquels :
A est un aromatique correspondant à la structure : 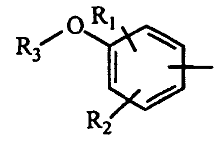 dans laquelle :
   R₁ et R₂ représentent, indépendamment un radical alkyle linéaire ou ramifié ayant de 1 à 6 atomes de carbone ou un radical alkoxy linéaire ou ramifié ayant de 1 à 6 atomes de carbone,
   R₃ représente un atome d'hydrogène ou un radical alkyle linéaire ou ramifié ayant de 1 à 6 atomes de carbone;
B représente un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -NH-CO-X'-, -CH=, -CO- ou une liaison,
   X' représentant -(CH₂)ₙ- avec n entier de 0 à 6 ;
Y représente -Y'-, -Y'-NH-CO-, -Y'-CO-, -Y'-O-, -Y'-O-Y'-, -Y'-N(R₃)-Y'- ou une liaison, Y' représentant -(CH₂)ₙ- avec n entier de 0 à 6 ;

Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine.

L'invention concerne tout particulièrement des composés de formule générale **(I)** pour lesquels :
A est un aromatique correspondant à la structure : 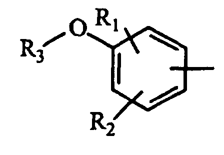 dans laquelle :
   R₁ et R₂ représentent, indépendamment un radical alkyle linéaire ou ramifié ayant de 1 à 6 atomes de carbone ou un radical alkoxy linéaire ou ramifié ayant de 1 à 6 atomes de carbone,
   R₃ représente un atome d'hydrogène ou un radical alkyle linéaire ou ramifié ayant de 1 à 6 atomes de carbone;
B représente un cycle thiophène, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -NH-CO-X'-, -CH=, -CO- ou une liaison,
   X' représentant -(CH₂)ₙ- avec n entier de 0 à 6 ;
Y représente -Y'-, -Y'-NH-CO-, -Y'-CO-, -Y'-O-, -Y'-O-Y'-, -Y'-N(R₃)-Y'- ou une liaison, Y' représentant -(CH₂)ₙ- avec n entier de 0 à 6 ;

Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants: pipérazines, homopipérazines, 4-aminopipéridine.

L'invention concerne de préférence les composés suivants :
- iodhydrate de N-(3,5-di-t-butyl-4-hydroxyphényl)-5-[4-{imino(2-thiényl)-méthylamino}phényl]-2-furanne carboxamide ;
- chlorhydrate de 3-(3,5-di-t-butyl-4-hydroxyphényl)-1-[4-{imino(2-thiényl)-méthylamino}phényl]-2,5-imidazolidinedione ;
- chlorhydrate de 2-(3,5-di-t-butyl-4-hydroxyphényl)-3-[4-{imino(2-thiényl)-méthylamino}phényl]-4-thiazolidinone ;
- fumarate de 5-[(3,5-di-t-butyl-4-hydroxyphényl)méthylène]-1-méthyl-3-[4-{imino(2-thiényl)méthylamino}phényl]-2,4-imidazolidinedione ;
- chlorhydrate de 2-(*S*)-4-(*S*)-N-[4-hydroxy-3,5-bis-(1,1-diméthyléthyl)-phényl]-4-{4-[(imino(2-thiényl)méthyl)amino]phénoxy}-prolinamide ;
- fumarate de N-[4-hydroxy-3,5-bis-(1,1-diméthyléthyl)phényl]-2-(*R*,*S*)-{4-[(imino(2-thiényl)méthyl)amino]phényl}-4-(*R*)-thiazolidine carboxamide ;
- iodhydrate de N-[3,5-bis(1,1-diméthyléthyl)-4-hydroxyphényl]-2-{4-[(imino(2-thiényl)méthyl)amino]phényl}-4-thiazolecarboxamide
- dichlorhydrate de N-[3,5-bis(1,1-diméthyléthyl)-4-hydroxyphényl]-4-(S)-{4-[(imino(2-thiényl)méthyl)amino]phénoxy}-pyrrolidine-2-(R)-carboxamide
- chlorhydrate de 1-[(3,4-dihydro-6-hydroxy-2,5,7,8-tétraméthyl-2-H-[1]-benzopyran-2-yl)carbonyl]-4-(S)-{4-[(imino(2-thiényl)méthyl)amino]-phénoxy}-pyrrolidine-2-(S)- carboxylate de méthyle
- chlorhydrate de 1-[(3,4-dihydro-6-hydroxy-2,5,7,8-tétraméthyl-2H-[1]-benzopyran-2-yl)carbonyl]-3-(S)-{4-[(imino(2-thiényl)méthyl)amino]phénoxy}-pyrrolidine
- 3- {[(3,4-dihydro-6-hydroxy-2,5,7,8-tétraméthyl-2H-[1]-benzopyran-2-yl)-carbonyl]amino}-1-{4-[(imino(2-thiényl)méthyl)amino]phényl}pyrrolidine
- chlorhydrate de 4-[3,5-bis-(1,1-diméthyléthyl)-4-hydroxyphényl]- N-{4-[(imino(2-thiényl)méthyl)amino]benzoyl}-N-méthyl-1H-imidazole-2-méthanamine
- iodhydrate de N-[3,5-bis-(1,1-diméthyléthyl)-4-hydroxyphényl]-1-{4-[(imino(2-thiényl)méthyl)amino]phényl}-1H-pyrrole-2-carboxamide
- iodhydrate de 1-[3,5-bis(1,1-diméthyléthyl)-4-hydroxyphényl]-3-{[4-[[imino(2-thiényl)méthyl]amino]phényl]carbonyl}-2-imidazolidinone
- iodhydrate de 3-[3,5-bis(1,1-diméthyléthyl)-4-hydroxyphényl]-4,5-dihydro-N-{4-[(imino(2-thiényl)méthyl)amino]phényl}-5-isoxazoleacétamide
- chlorhydrate de 4-[3,5-bis(1,1-diméthyléthyl)-4-hydroxyphényl]-N-{4-[(imino(2-thiényl)méthyl)amino]phényl}-N-méthyl-2-thiazoleméthanamine
- chlorhydrate de 4-[3,5-bis(1,1-diméthyléthyl)-4-hydroxyphényl]-N-{4-[(imino(2-thiényl)méthyl)amino]phényl}-N-méthyl-1H-imidazole-2-méthanamine
- 3-[3,5-bis(1,1-diméthyléthyl)-4-hydroxyphényl]-4,5-dihydro-5-{2-{4-[(imino(2-thiényl)méthyl)amino]phénoxy}éthyl}isoxazole
- chlorhydrate de 1-{[3,5-bis(1,1-diméthyléthyl)-4-hydroxyphényl]amino}-carbonyl}-3-{4-[(imino(2-thiényl)méthyl)amino]phénoxy}azétidine
- chlorhydrate de 1-(2-hydroxy-5-méthoxybenzoyl)-3-{4-[(imino(2-thiényl)méthyl)amino]phénoxy}azétidine
- chlorhydrate de 1-[(3,4-dihydro-6-hydroxy-2,5,7,8-tetraméthyl-2H-[1]-benzopyran-2-yl)carbonyl]-4-[4-[(imino(2-thiényl)méthyl)amino]phénoxy}-pipéridine
- chlorhydrate de 1-[(3,4-dihydro-6-hydroxy-2,5,7,8-tetraméthyl-2H-[1]-benzopyran-2-yl)carbonyl]-3-{4-[(imino(2-thiényl)méthyl)amino]-phénoxy}azétidine
ou leurs sels ou énantiomères.

L'invention concerne particulièrement les composés suivants :
- chlorhydrate de 3-(3,5-di-t-butyl-4-hydroxyphényl)-1-[4-{imino(2-thiényl)-méthylamino}phényl]-2,5-imidazolidinedione ;
- chlorhydrate de Z-(3,5-di-t-butyl-4-hydroxyphényl)-3-[4-{imino(2-thiényl)-méthylamino}phényl]-4-thiazolidinone ;
- fumarate de 5-[(3,5-di-t-butyl-4-hydroxyphényl)méthylène]-1-méthyl-3-[4-{imino(2-thiényl)méthylamino}phényl]-2,4-imidazolidinedione ;
- chlorhydrate de 2-(*S*)-4-(*S*)-N-[4-hydroxy-3,5-bis-(1,1-diméthyléthyl)-phényl]-4-{4-[(imino(2-thiényl)méthyl)amino]phénoxy}-prolinamide ;
- chlorhydrate de N-[4-hydroxy-3,5-bis-(1,1-diméthyl)éthyl-phényl]-2-{4-[(imino(2-thiényl)méthyl)amino]phényl}-4-thiazole carboxamide ;
ou leurs sels ou énantiomères.

L'invention a également pour objet, à titre de médicaments, les composés de formule générale **(I)** décrits précédemment ou leurs sels pharmaceutiquement acceptables. Elle concerne aussi des compositions pharmaceutiques contenant ces composés ou leurs sels pharmaceutiquement acceptables, et l'utilisation de ces composés ou de leurs sels pharmaceutiquement acceptables pour fabriquer des médicaments destinés à inhiber la NO synthase, à inhiber la péroxidation lipidique ou à assurer la double fonction d'inhibition de la NO synthase et d'inhibition de la péroxidation lipidique.

Par sel pharmaceutiquement acceptable, on entend notamment des sels d'addition d'acides inorganiques tels que chlorhydrate, sulfate, phosphate, diphosphate, bromhydrate, iodhydrate et nitrate ou d'acides organiques tels que acétate, maléate, fumarate, tartrate, succinate, citrate, lactate, méthane sulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J. Pharm. Sci*. **66**:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

L'invention offre aussi, à titre de produits industriels nouveaux, des intermédiaires de synthèse des produits de formule générale **(I),** à savoir les composés de formule générale **(V), (VI)** et **(VII)** 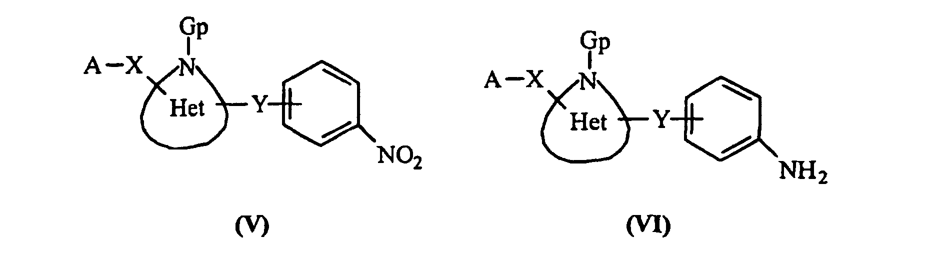 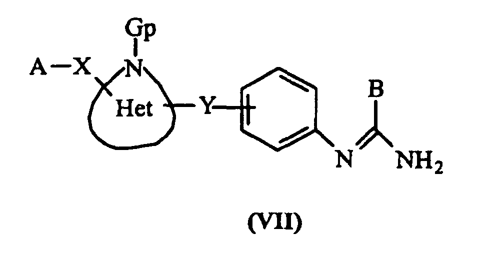 pour lesquels
A est un aromatique correspondant aux structures : 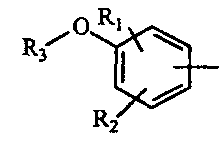 dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
   ou 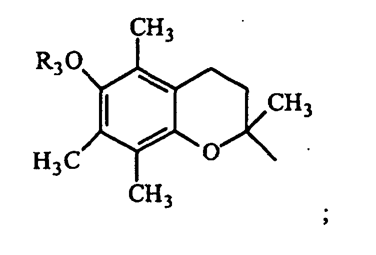
B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
   X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;

Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants: pipérazines, homopipérazines, 4-aminopipéridine ;

Gₚ représente un groupe protecteur de fonction amine clivable en milieu acide anhydre, tel que par exemple les carbamates de t-butyle, trichloroéthyle ou de triméthylsilyléthyle ou encore le groupe trityle.

Enfin, l'invention offre des procédés de préparation de composés de formule générale **(I)** tels que définis ci-dessus et consistant, par exemple, en la réaction dans un alcool inférieur, tel que méthanol, éthanol, alcool isopropylique ou t-butanol, de préférence dans l'alcool isopropylique, à une température comprise entre 20 et 90°C, par exemple à 50 °C, et durant une à 48 heures, de préférence durant 15 à 24 heures, éventuellement en présence de DMF, d'un composé de formule générale **(III)** 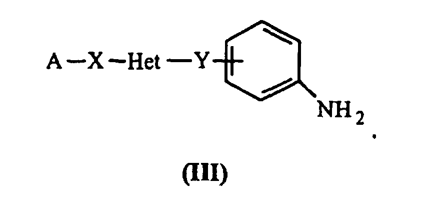 avec un composé de formule générale **(IV)** 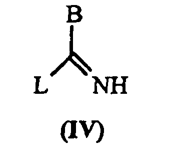 ledit composé de formule générale **(IV)** pouvant éventuellement être salifié par un acide minéral G, B ayant la signification indiquée ci-dessus et L représentant un groupe partant et notamment un radical alkoxy, thioalkyl, acide sulfonique, halogénure, alcool arylique ou tosyle (d'autres groupes partants bien connus de l'homme du métier et pouvant être éventuellement utilisés pour l'invention sont décrits dans l'ouvrage suivant: Advanced Organic Chemistry, J. March, 3rd Edition (1985), Mc Graw-Hill, p. 315). De préférence, G représente HCl, HBr ou HI.

D'autres procédés de fabrication sont envisageables et sont accessibles dans la littérature (par exemple : The Chemistry of amidines and imidates, Vol. 2, Saul PATAI and Zvi RAPPOPORT, John Wiley & Sons, 1991).

Pour les procédés ci-dessus, les composés de formule générale **(I), (III), (IV), (VI)** et **(VII)** sont tels que :
A est un aromatique correspondant aux structures : 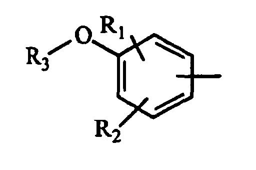
dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
   ou 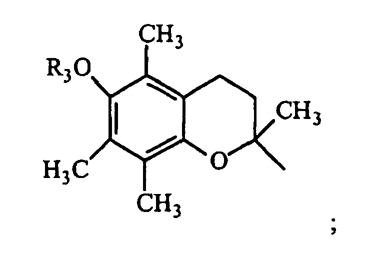
B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyl ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
   X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;

Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine ;
Gₚ représente un groupe protecteur de fonction amine de préférence clivable en milieu acide anhydre, tel que par exemple les carbamates de t-butyle, trichloroéthyle ou de triméthylsilyléthyle ou encore le groupe trityle.

Conformément à l'invention, on peut préparer les composés de formule générale **(I)** par le procédé ci-dessous.

### Préparation des composés de formule générale (I) :

Les composés de formule générale **(I)** peuvent être préparés à partir des intermédiaires de formule générale **(II), (III)** ou **(V)** selon le schéma 1.

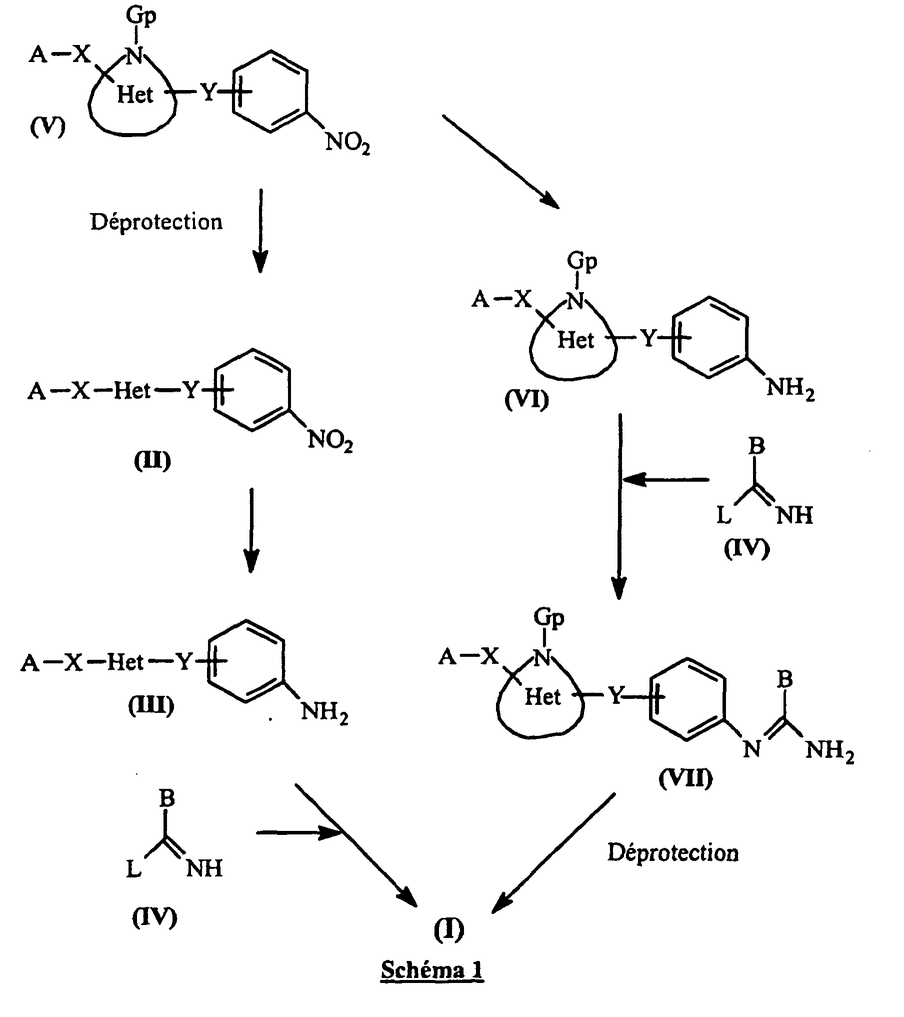

La réduction de la fonction nitro des intermédiaires de formule générale (**II**) est généralement effectuée par hydrogénation catalytique, dans l'éthanol, en présence de Pd/C, sauf dans les cas où les molécules contiennent une insaturation ou un atome de soufre, celui étant un poison pour le Pd/C. Dans ces cas, le groupement nitro est sélectivement réduit, par exemple en chauffant le produit en solution dans l'acétate d'éthyle avec un peu d'éthanol en présence de SnCl₂ (*J. Heterocyclic Chem.* (1987), **24,** 927-930 ; *Tetrahedron Letters* (1984), **25,** (8), 839-842) ou bien en utilisant du Ni Raney additionné d'hydrate d'hydrazine (*Monatshefte für Chemie,* (1995), **126,** 725-732).

Les dérivés d'aniline de formule générale **(III),** ainsi obtenus, peuvent être condensés sur des dérivés de formule générale (**IV**), par exemple des dérivés du type O-alkyl thioimidate ou S-alkyl thioimidate, pour conduire aux composés finaux de formule générale **(I)** (cf. schéma 1). Par exemple, pour B = thiophène, on peut condenser les dérivés de formule générale **(III)** sur l'iodhydrate de S-méthylthiophène thiocarboxamide, préparé selon une méthode de la littérature (*Ann. Chim*. (1962), **7**, 303-337). La condensation peut s'effectuer par chauffage dans un alcool (par exemple dans du méthanol ou de l'isopropanol), éventuellement en présence de DMF à une température de préférence comprise entre 50 et 100°C pour une durée généralement comprise entre quelques heures et une nuit.

Les molécules finales de formule générale **(I)** sont également accessibles à travers un autre chemin synthétique passant par les intermédiaires de formule générale (**V**) qui portent une fonction amine hétérocyclique protégée par un groupe protecteur " Gp ", par exemple un groupement 2-(triméthylsilyl)éthoxyméthyl (SEM) ou par un autre groupe protecteur cité dans : *Protective groups in organic synthesis*, 2d ed., (John Wiley & Sons Inc., 1991). Les étapes de réduction et condensation qui conduisent respectivement aux intermédiaires **(VI)** et **(VII)** sont effectuées dans les mêmes conditions que celles décrites précédemment. La dernière étape de la synthèse consiste à régénérer, par exemple en milieu acide ou en présence d'ion fluorure, la fonction amine hétérocyclique protégée.
Alternativement, les intermédiaires de formule générale **(V)** peuvent être transformés directement en intermédiaire de formule générale **(II)** par libération de l'amine hétérocyclique par traitement, par exemple, en milieu acide ou en présence d'ion fluorure.

### Préparation des composés de formule générale (II), (III) et (V) :

Les intermédiaires de formule générale **(II), (III)** et **(V)** peuvent être préparés par différents chemins synthétiques illustrés ci-dessous.

Lorsque :
Het = Imidazole, tétrahydropyridine, thiazolidine, dihydroimidazole-2-one
et Y = -Y' -.

Les amines de formule générale **(II)**, schéma 2, dans lesquelles A, X, Y et Het sont tels que définis ci-dessus, peuvent être obtenues par substitution nucléophile des dérivés halogénés commerciaux de formule générale **(IX)** par une amine hétérocyclique de formule générale **(VIII).** La réaction s'effectue dans l'acétonitrile, le THF ou le DMF en présence d'une base telle que K₂CO₃ à une température variant de 20 à 110° C. La synthèse des dérivés hétérocycliques de formule générale **(VIII),** non commerciaux, est décrite plus loin.

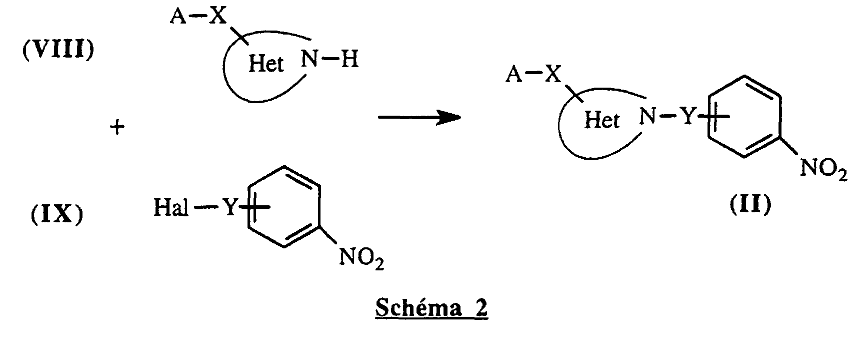

Lorsque :
Het = imidazole, thiazolidine, tétrahydropyridine
et Y = -Y'-.

Les amines hétérocycliques de formule générale **(III)**, schéma 3, dans lesquelles A, X, Y et Het sont tels que définis ci-dessus, sont préparées en deux étapes à partir des amines de formule générale **(VIII)** (voir plus loin). Le mélange d'un dérivé bromé de formule générale **(X),** dont la synthèse est détaillée plus loin, avec une amine de formule générale **(VIII)** dans un solvant tel que l'acétonitrile ou le DMF en présence d'une base conduit aux intermédiaires de formule générale **(XI).** La déprotection de la fonction amine, en milieu acide organique, permet d'obtenir les composés de formule générale **(III)**.

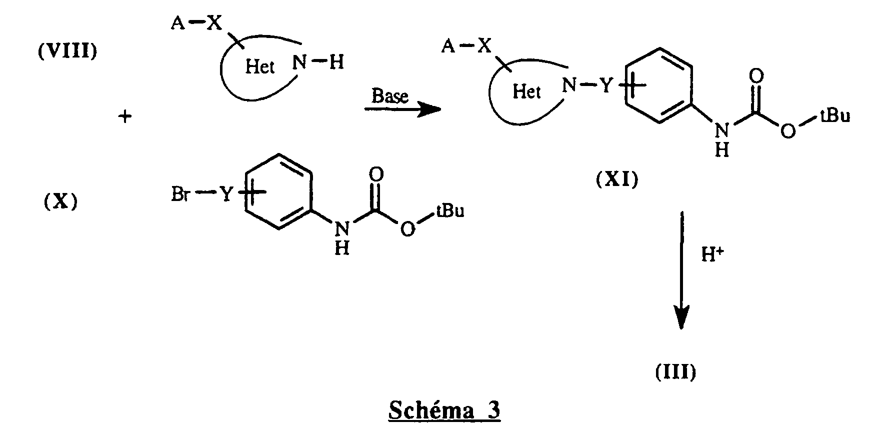

Lorsque :
Het = thiazolidine
et Y = -CO-Y'-.

Les carboxamides de formule générale **(III)**, schéma 4, dans lesquelles A, X, Y et Het sont tels que définis ci-dessus, sont préparés par condensation des amines de formule générale **(VIII)**, précédemment décrites, avec les acides carboxyliques de formule générale **(X.2)**. Les liaisons carboxamides sont formées dans des conditions classiques de synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexylcarbodiimide (DCC), le 1,1'-carbonyldiimidazole (CDI) (*J. Med. Chem*. (1992), **35** (23), 4464-4472) ou le chlorhydrate de 1-(3-diméthylaminopropyl)-3-éthylcarbodiimde (EDC ou WSCI) (John Jones, The chemical synthesis of peptides, 54 (Clarendon Press, Oxford, 1991)). La synthèse des acides carboxyliques de formule générale **(X.2)** est décrite plus loin. Les intermédiaires de formule générale **(XII)** sont ensuite déprotégés en milieu acide à l'aide, par exemple, d'acide trifluroroacétique ou d'une solution organique d'HCl.

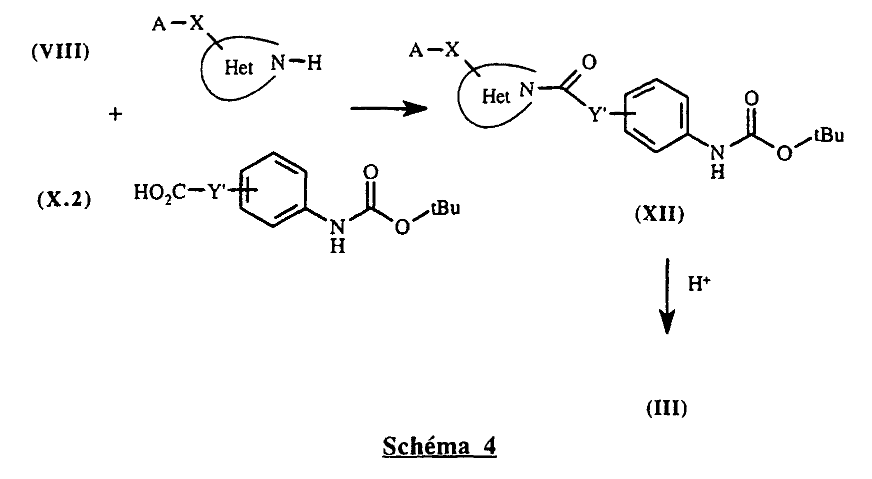

Lorsque :
Het = thiazolidine
et Y = -CO-NH-Y'-.

Les carboxamides de formule générale **(V),** schéma 5, dans lesquelles A, X, Y et Het sont tels que définis ci-dessus, sont préparés par condensation des acides carboxyliques de formule générale **(XIII)** avec les amines commerciales de formule générale **(XIV)** dans les conditions classiques de la synthèse peptidique. La synthèse des acides carboxyliques de formule générale **(XIII)** est décrite plus loin.

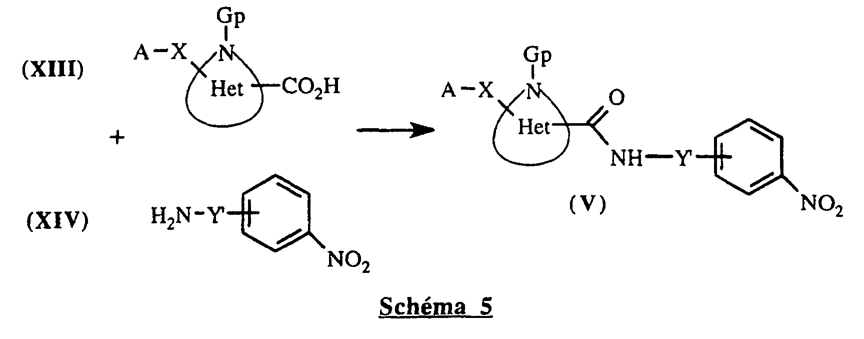

Lorsque :
Het = thiazole, furanne, pyrrole, tétrahydropyridine, pyrrolidine
et X = -NH-CO-X'-.

Les carboxamides de formule générale **(II),** schéma 6, dans lesquelles A, X, Y et Het sont tels que définis ci-dessus, sont préparés par condensation des anilines de formule générale **(XV)** avec les acides carboxyliques de formule générale **(XVI)** dans des conditions classiques de condensation peptidique. Les anilines de formule générale **(XV)** sont obtenues par hydrogènation, en présence d'une quantité catalytique de Pd/C, des dérivés nitrobenzène correspondants, eux-mêmes synthétisés selon une méthode décrite dans la littérature (*J. Org. Chem.* (1968), **33** (1), 223-226). Les acides de formule générale **(XVI),** schéma 6, non commerciaux, sont préparés selon des méthodes décrites dans la littérature.
La synthèse des pyrroles est décrite dans *Chem. Heterocycl. Compd.,* 1982, **18,** 375. Les prolines substituées sont accessibles à partir des hydroxyprolines commerciales et sont préparées selon des méthodes décrites dans *J. Org. Chem*., 1991, **56**, 3009.

La synthèse des dérivés thiazole et tétrahydropyridine est décrite plus loin.

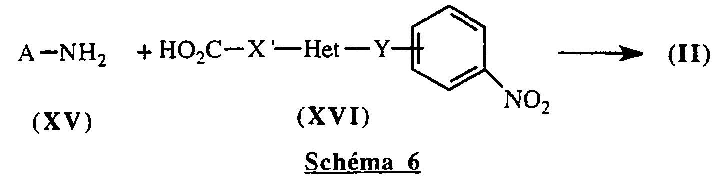

Lorsque :
Het = hydantoïne
et Y = -Y'-.

Les hydantoïnes de formule générale **(II),** schéma 7, dans lesquelles A, X, Y et Het sont tels que définis ci-dessus, sont préparées en 3 étapes à partir des anilines de formule générale **(XV)** précédemment décrites. La substitution de l'aniline par le bromoacétate d'éthyle est effectuée en présence d'acétate de sodium dans l'éthanol à une température d'environ 60-70° C. Le produit de monosubstitution de formule générale **(XVII)** est ensuite condensé sur un isocyanate de formule générale **(XVIII)** dans un solvant organique tel que, par exemple, le dichlorométhane, à une température d'environ 20°C. La cyclisation de l'urée **(XIX)** est effectuée par chauffage, à 50°C, dans l'éthanol, selon un protocole expérimental décrit dans la littérature (*J. Heterocyclic Chem.,* (1979), **16,** 607-608). Les isocyanates de formule générale **(XVIII)** sont synthétisés à partir des amines primaires commerciales correspondantes, de triphosgène et d'une amine tertiaire (*J. Org. Chem.* (1994), **59** (7), 1937-1938).

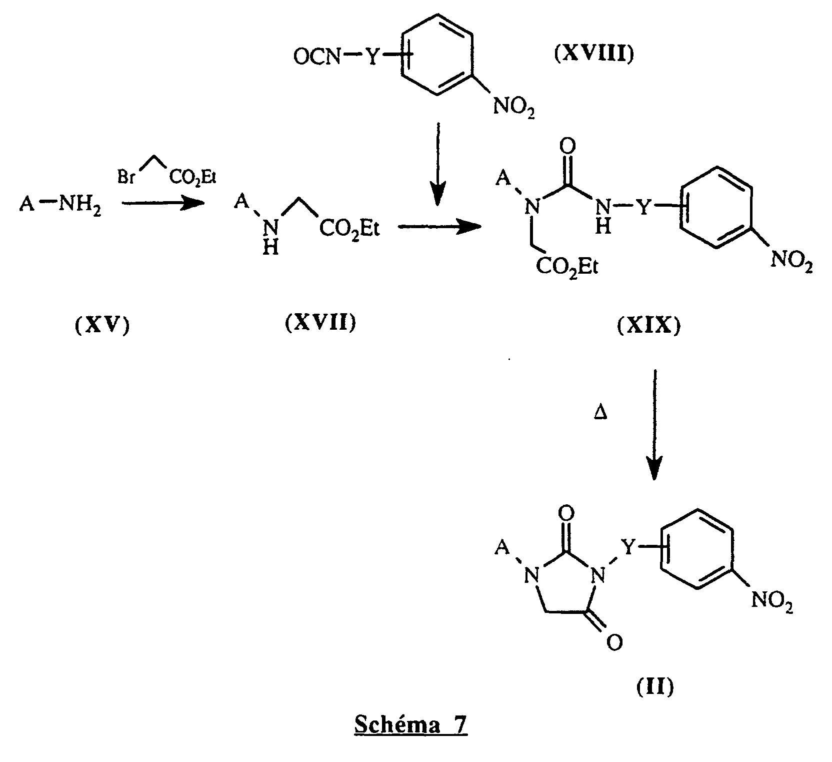

Lorsque :
Het = thiazolidinone
et Y = -Y'-.

Les thiazolidinones de formule générale **(II)**, schéma 8, dans lesquelles A, X, Y et Het sont tels que définis ci-dessus, sont préparées à partir des amines commerciales de formule générale **(XIV)** et des aldéhydes de formule générale **(XX)** en présence de l'acide mercaptoacétique selon un protocole expérimental décrit dans la littérature (*J. Med. Chem*., (1992), **35**, 2910-2912).

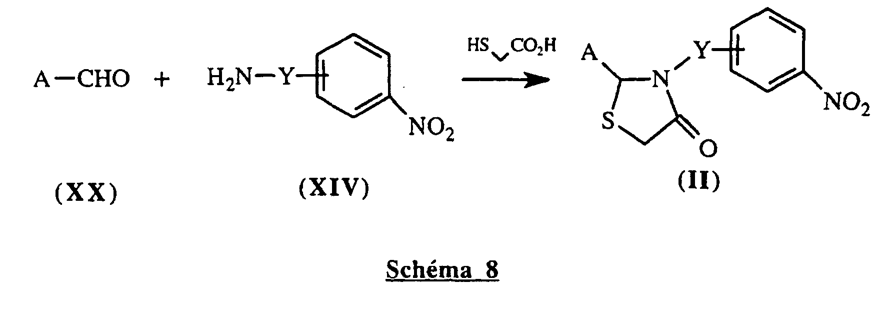

Lorsque :
Het = hydantoïne
X = -CH= et Y = -Y'-.

Les hydantoïnes de formule générale **(II)**, schéma 9, dans lesquelles A, X, Y et Het sont tels que définis ci-dessus, sont préparées en 2 étapes à partir des isocyanates de formule générale **(XVIII)** précédemment décrits. La réaction de l'ester éthylique de la sarcosine avec les isocyanates de formule générale **(XVIII),** effectuée selon un protocole expérimental décrit dans la littérature (*J. Heterocyclic Chem*., (1979), **16,** 607-608), conduit à la formation de l'hétérocycle des composés de formule générale **(XXI).** La substitution de l'hydantoïne est réalisée en présence d'une base faible, la β-alanine, et d'un aldéhyde de formule générale **(XX)** en suivant les conditions expérimentales décrites dans *J. Med. Chem*., (1994), **37**, 322-328.

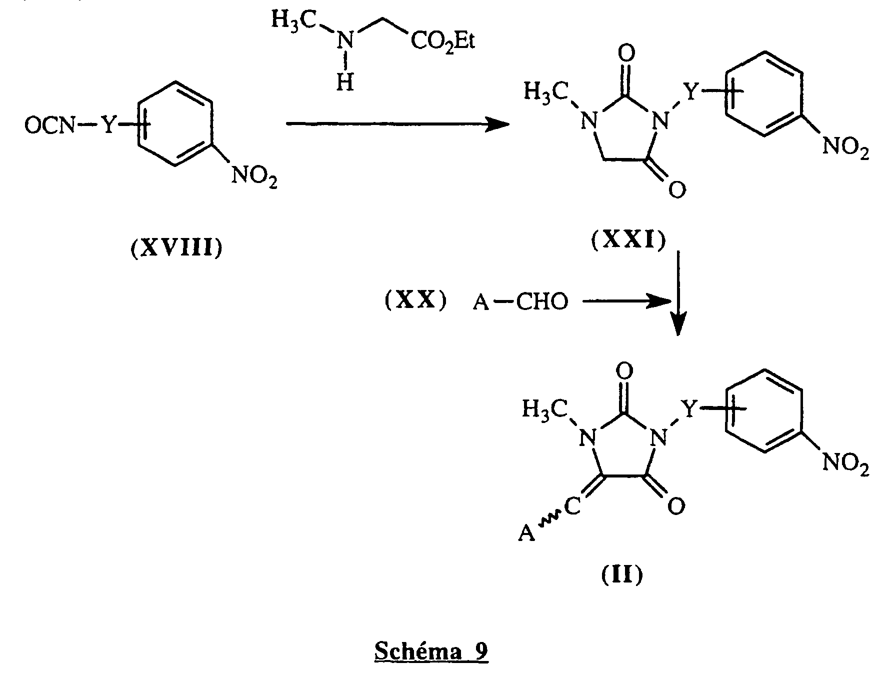

Lorsque :
Het = pyrrolidine, thiazolidine
X = -NH-CO-X'- et Y = -O-Y'- ou -Y'-.

Les carboxamides de formule générale **(V),** schéma 10, dans lesquelles A, X, Y et Het sont tels que définis ci-dessus, sont préparés par condensation des anilines de formule générale **(XV)**, précédemment décrites, avec les acides de formule générale **(XXII)** dans des conditions classiques de synthèse peptidique. Les synthèses des acides carboxyliques **(XXII)**, non commerciaux, sont décrites plus loin.

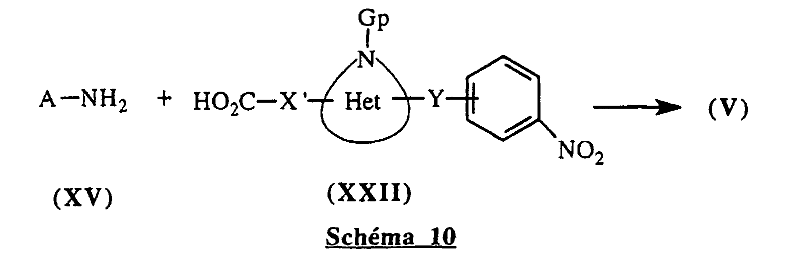

Lorsque :
Het = tétrahydropyridine
et Y = -CO-NH-Y'-.

Les urées de formule générale **(II),** schéma 11, dans lesquelles A, X, Y et Het sont tels que définis ci-dessus, sont préparées par condensation des amines hétérocycliques de formule générale **(VIII),** précédemment décrites, avec des isocyanates de formule générale **(XVIII)** (cf. plus haut) dans un solvant tel que le dichlorométhane, à 20° C, en présence d'une amine tertiaire (p. ex. diisopropyléthylamine).

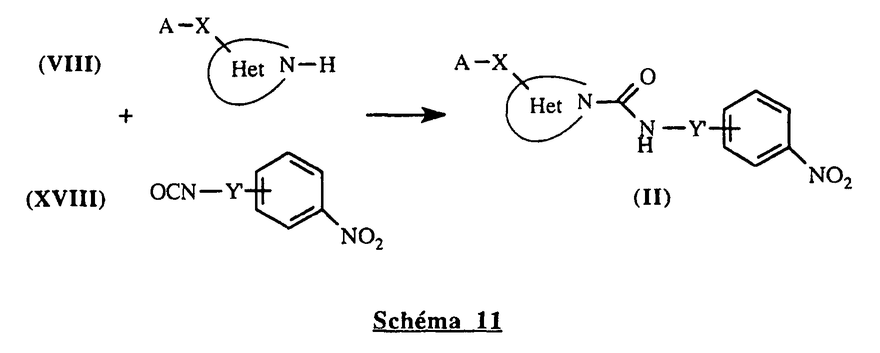

Lorsque :
Het = pyrrolidine, thiazole, thiadiazole
et X = -CO-NH-X'-.

Les carboxamides de formule générale **(II),** schéma 12, dans lesquelles A, X, Y et Het sont tels que définis ci-dessus, sont préparés par condensation des acides carboxyliques commerciaux de formule générale **(XXIII)** avec les aminés de formule générale **(XXIV)** dans les conditions classiques de la synthèse peptidique. Les synthèses des amines de formule générale **(XXIV),** non commerciales, sont décrites plus loin.

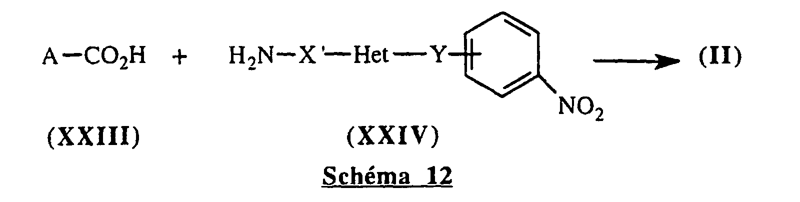

Lorsque :
Het = imidazole, oxazole et thiazole
et Y = -CH(R₃)-N(R₃)-CO-Y'-.

Les carboxamides de formule générale **(V)**, schéma 13, dans lesquelles A, X, Y et Het sont tels que définis ci-dessus, sont préparés par condensation des amines de formule générale **(XXV)** avec les acides carboxyliques commerciaux (ou les chlorures d'acide correspondants) de formule générale **(XXVI)** dans les conditions classiques de la synthèse peptidique. La synthèse des dérivés imidazoles de formule générale **(XXV)** est décrite plus loin.

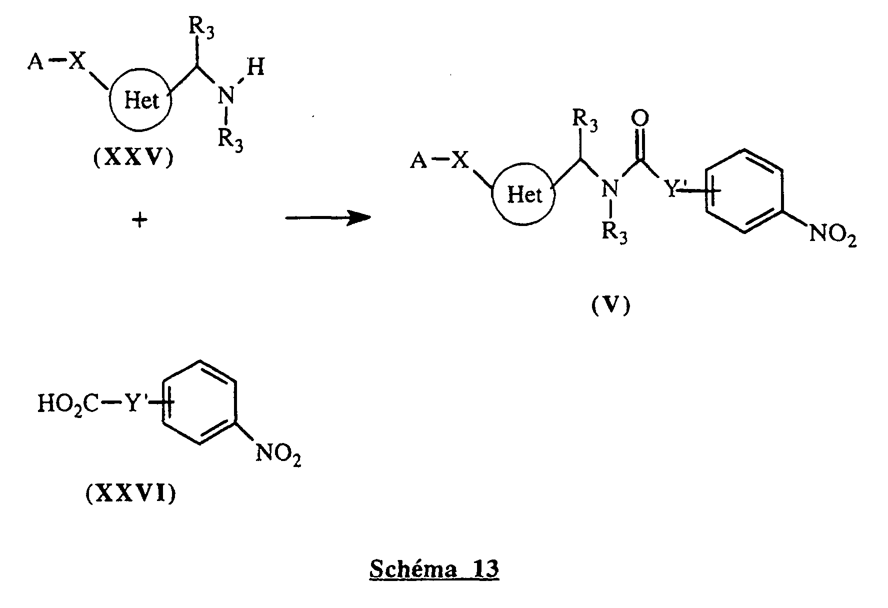

Lorsque :
Het = imidazole
et Y = -CH₂-N(R₃)-Y'-.

Les amines de formule générale **(V),** schéma 14, dans lesquelles A, X, Y et Het sont tels que définis ci-dessus, sont préparés par condensation des amines de formule générale **(XXV)** (voir plus loin) avec les dérivés halogénés commerciaux de formule générale **(IX)** dans les conditions précédemment décrites.

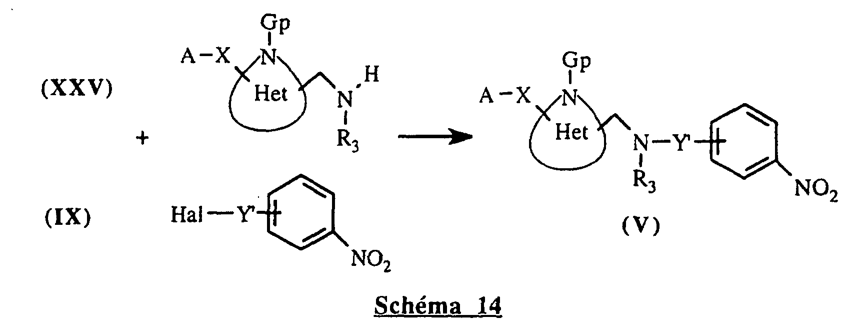

Lorsque :
Het = dihydroimidazole-2-one
et Y = -CO-Y'.

Les amines de formule générale **(II)**, schéma 15, dans lesquelles A, X, Y et Het sont tels que définis ci-dessus, sont préparés par condensation des amines de formule générale **(VIII)** (voir plus loin) avec les dérivés halogénés commerciaux de formule générale **(XXVII),** par exemple dans un mélange d'acétonitrile et de THF et en présence d'une base telle que K₂CO₃.

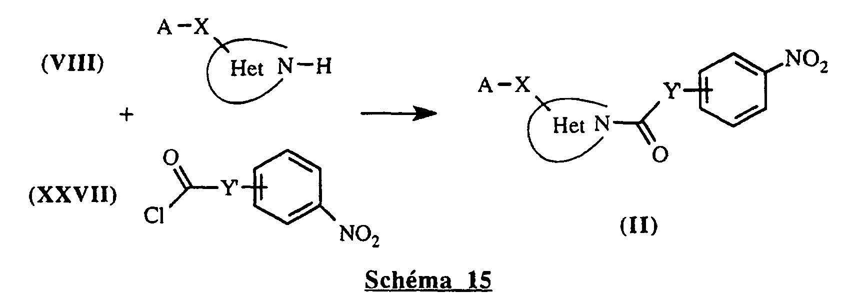

Lorsque :
Het = oxazolidinone
et Y = -Y'-O-.

Les oxazolidinones de formule générale **(II),** schéma 16, sont préparées à partir des diols de formule générale **(XXVII)** dont la synthèse est décrite dans la littérature (Daumas, M., *Tetrahedron,* 1992, **48**(12), 2373). La formation des carbonates de formule générale **(XXVIII)** est obtenue, par exemple, en présence de carbonyl di-imidazole (Kutney, J.P., *Synth. Commun.,* 1975, **5**(1), 47) ou en présence de triphosgène à basse température comme décrit dans *Synth. Commun.,* 1994, **24**(3), 305. La formation de l'oxazolidinone intervient lors du chauffage des amines de formule générale **(XV)** avec les carbonates de formule générale **(XXVIII)** en présence d'une catalyse acide, tel que ZnCl₂, à reflux du xylène pour éliminer l'eau formée au cours de la réaction (Laas, H., *Synthesis,* 1981, 958).

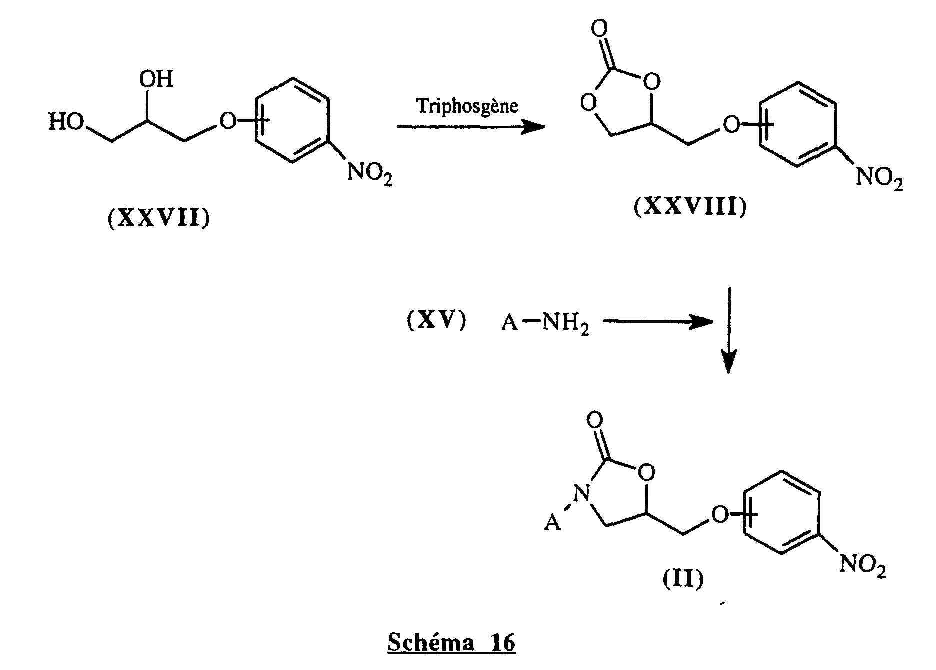

Lorsque :
Het = isoxazoline, isoxazole, oxazole, thiazole
et Y = -Y'-CO-NH-Y'-

Les carboxamides de formule générale **(II),** schéma 17, dans lesquels A, X, Y et Het sont tels que définis ci-dessus, peuvent être préparés à partir des amines commerciales de formule générale **(XIV)** et des acides carboxyliques de formule générale **(XXVIII)** par condensation en présence de chloroformiate d'isobutyle (*Org. Prep. Proced. Int.,* (1975), **7**, 215).

La préparation des oxazoles de formule générale **(XXVIII)** est effectuée en suivant un protocole expérimental décrit dans *Tetrahedron Lett.,* 1994, **35** (13), 2039. De même pour la synthèse des thiazoles de formule générale **(XXVIII)** : *J. Med. Chem.,* 1983, **26,** 884. La préparation des isoxazolines est décrite plus loin.

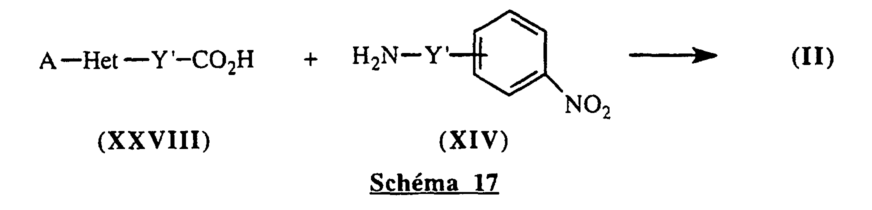

Lorsque :
Het = pyrrolidine, pipéridine
X = -CO-NH-
et Y = -O-Y'-.

Les carboxamides de formule générale **(II)**, schéma 18, dans lesquels A, X, Y et Het sont tels que définis ci-dessus, peuvent être préparés par condensation des acides carboxyliques commerciaux de formule générale **(XXIII)** avec les amines de formule générale **(XXIX)** dans les conditions classiques de la synthèse peptidique. Les synthèses des amines de formule générale **(XXIX)** sont décrites plus loin.

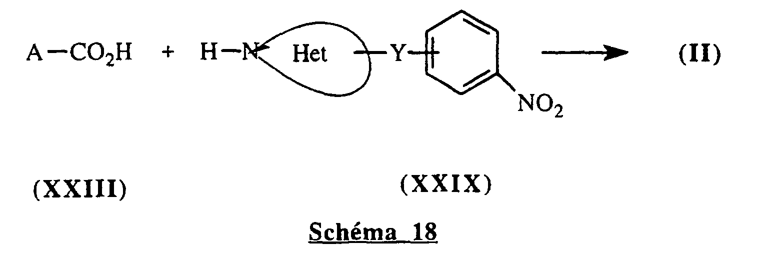

Lorsque :
Het = isoxazoline, oxazole, thiazole, imidazole
et Y = -Y'-O-Y'- ou -Y'-N(R₃)-Y'-.

Les étheroxydes de formule générale **(II)**, Schéma 19, dans lesquels A, X, Y et Het sont tels que définis ci-dessus, peuvent être préparés à partir des esters de formule générale (XXVIII.4), schéma 17.1, par réaction avec des hydrures, par exemple LiAlH₄, dans un solvant tel que, par exemple, le THF anhydre. Les alcools primaires ainsi obtenus sont ensuite condensés sur des dérivés halogénés de formule générale **(IX)** à l'aide d'une base telle que par exemple KOH en milieu organique et en présence d'un catalyseur de transfert de phase tel que par exemple l'Aliquat 336.

Les alcools primaires **(XXXI)** peuvent également être activés sous forme de dérivés sulfonate, par du chlorure de tosyle en présence de pyridine, pour conduire aux intermédiaires de formule générale **(XXXII).** La condensation des alcools de formule générale **(XXII.2)** est ensuite effectuée en présence d'une base forte, telle que, par exemple, NaH, dans un solvant aprotique (THF ou DMF) à une température comprise entre 20°C et 80°C, pour obtenir les étheroxydes de formule générale **(II)**.

De même, les amines de formule générale **(II),** schéma 19, sont obtenues par substitution de la fonction tosylate des intermédiaires de formule générale **(XXXII),** obtenus classiquement à partir des alcools de formule générale **(XXXI)** et du chlorure de tosyle en présence de pyridine, par les amines commerciales de formule générale **(XXX)** en réaction dans un solvant tel que, par exemple, l'acétonitrile ou le DMF, en présence d'une base (K₂CO₃) à une température comprise entre 20 et 85°C.

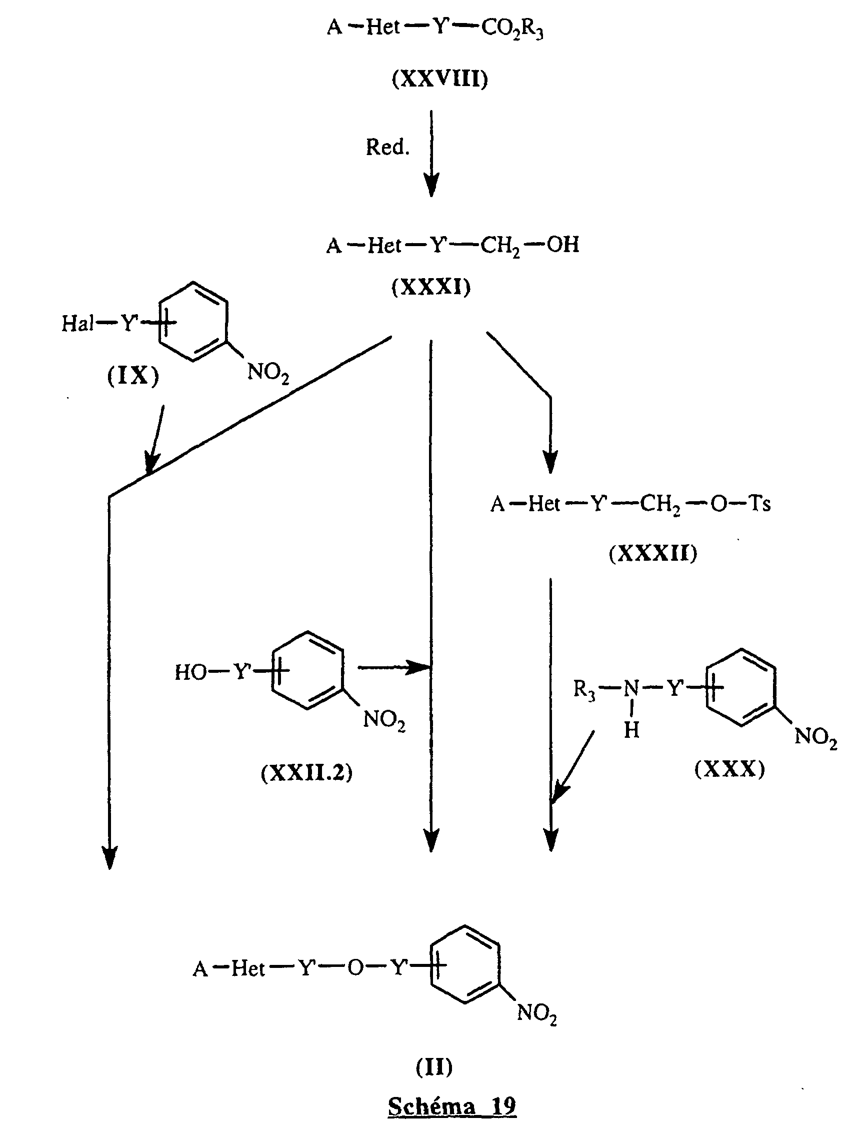

Lorsque :
Het = azétidine
X = -CO-NH-
et Y = -O-Y'-.

Les carboxamides de formule générale **(III),** schéma 20, dans lesquelles A, X, Y et Het sont tels que définis ci-dessus, peuvent être préparés par condensation des acides carboxyliques commerciaux de formule générale **(XXIII)** avec les amines de formule générale **(XXXII)** dans les conditions classiques de la synthèse peptidique. La synthèse des amines de formule générale **(XXXII)** est décrite plus loin. La déprotection de l'aniline est effectuée par un acide fort tel que, par exemple, l'acide trifluoroacétique éventuellement en présence de triéthylsilane.

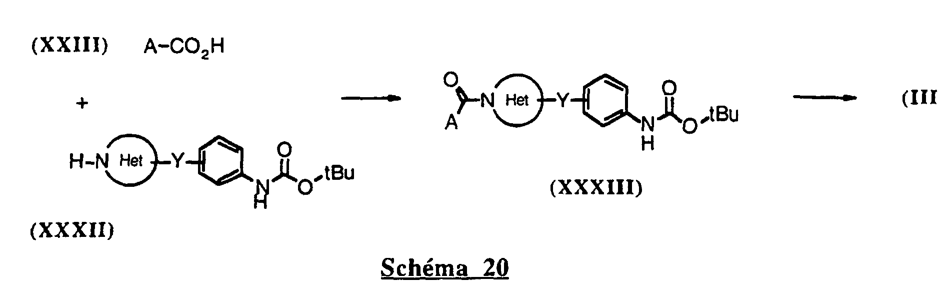

Lorsque :
Het = azétidine
X = -NH-CO-X'-
et Y = -O-Y'-.

Les urées de formule générale **(III),** schéma 21, dans lesquelles A, X, Y et Het sont tels que définis ci-dessus, peuvent être préparés par l'addition des amines de formule générale **(XXXII)** sur les isocyanates **(XXXIV)** obtenus à partir de la réaction des amines de formule générale **(XV)** avec du triphosgène en présence d'une amine tertiaire telle que par exemple la diisopropyléthylamine dans un solvant neutre tel que le dichlorométhane (*J. Org. Chem*. (1994), **59** (7), 1937-1938). Les urées de formule générale **(XXXV)** ainsi obtenues sont déprotégées par traitement en milieu acide fort comme précédemment décrit. La synthèse des amines de formule générale **(XXXII)** est décrite plus loin.

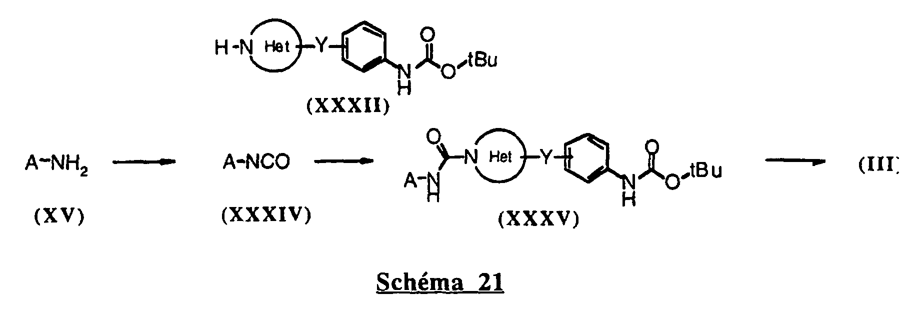

Lorsque :
Het = thiazole
et Y = -CH₂-N(R₃)-Y'-.

Les amines de formule générale **(II),** schéma 22, dans lesquelles A, X, Y et Het sont tels que définis ci-dessus, sont préparés par condensation des amines de formule générale **(XXV)** (voir plus loin) avec les dérivés halogénés commerciaux de formule générale **(IX)** dans les conditions précédemment décrites.

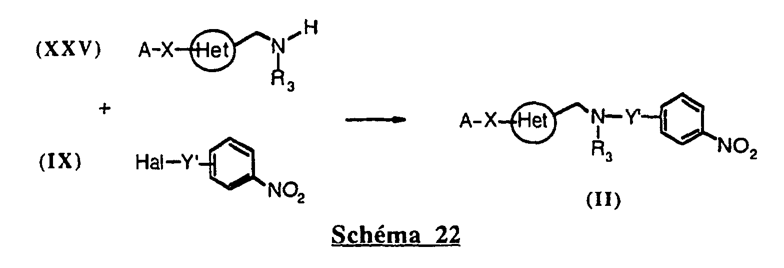

### Préparation des différents intermédiaires de synthèse :

### Synthèse des Intermédiaires (VIII) :

Les synthèses des intermédiaires de formule générale **(VIII)** sont illustrées dans les schémas 2.1 et 2.2.

Les intermédiaires de formule générale **(VIII)**, schéma 2.1, peuvent être préparés, par exemple, en 3 étapes à partir de l'acide 4-imidazole carboxylique. La protection de l'azote de l'hétérocycle est effectuée par (Boc)₂O en présence d'une base telle que K₂CO₃ dans le DMF. La condensation avec les amines de formule générale **(XV)** (voir ci-dessus) est réalisée classiquement dans des conditions de synthèse peptidique pour conduire aux intermédiaires de formule générale **(VIII.3)**. L'amine de l'hétérocycle est régénérée par traitement en milieu acide et en particulier avec l'acide trifluoroacétique pour conduire aux intermédiaires de formule générale **(VIII)**.

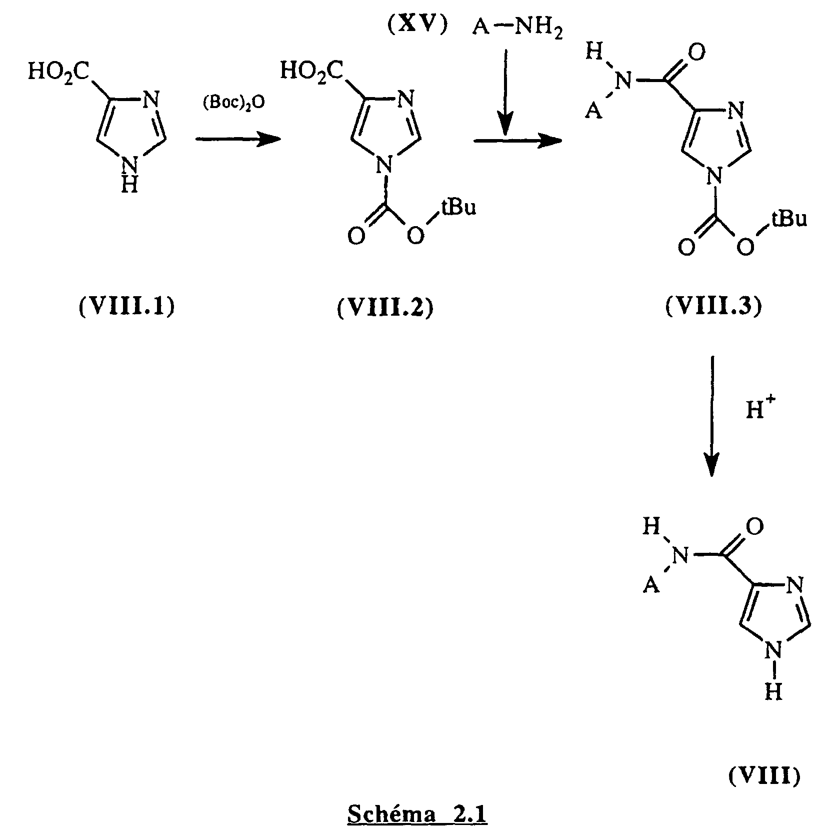

Les dihydroimidazole-2-ones de formule générale **(VIII)**, schéma 2.2, peuvent être préparés, par exemple, en 2 étapes à partir des anilines de formule générale **(XV)** (voir ci-dessus) qui sont condensées sur le 2-chloroéthyl isocyante dans le DMF à 20° C pour conduire aux urées de formule générale **(VIII.4)**. La cyclisation pour donner **(VIII)** est ensuite effectuée par traitement en milieu basique au moyen, par exemple, de tBuOK dans le DMF.

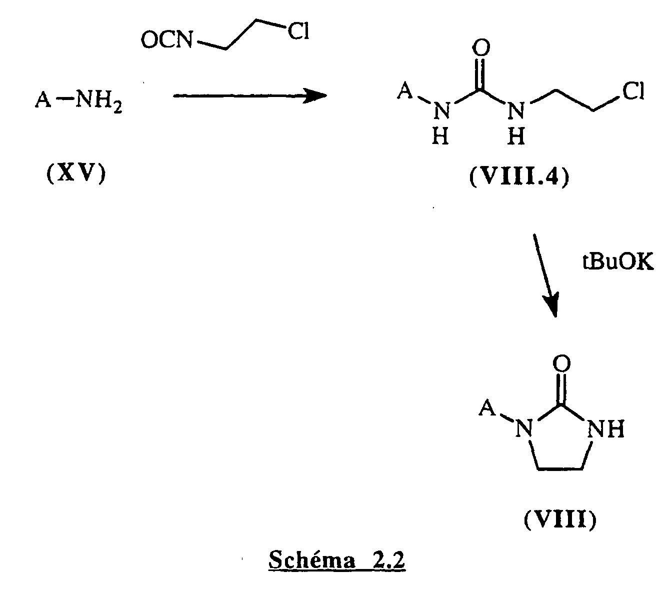

### Synthèse des Intermédiaires (X) :

Les intermédiaires de formule générale **(X),** schéma 3.1, peuvent être préparés à partir des acides carboxyliques de formule générale **(X.1)** commerciaux. La protection de la fonction amine sous forme d'un carbamate est suivie de la réduction sélective de la fonction acide carboxylique par l'hydrure de lithium et aluminium dans un solvant tel que le THF, à 20° C. L'intermédiaire **(X.3)** est ensuite bromé en présence de tétrabromure de carbone et de triphénylphosphine dans un solvant tel que le dichlorométhane.

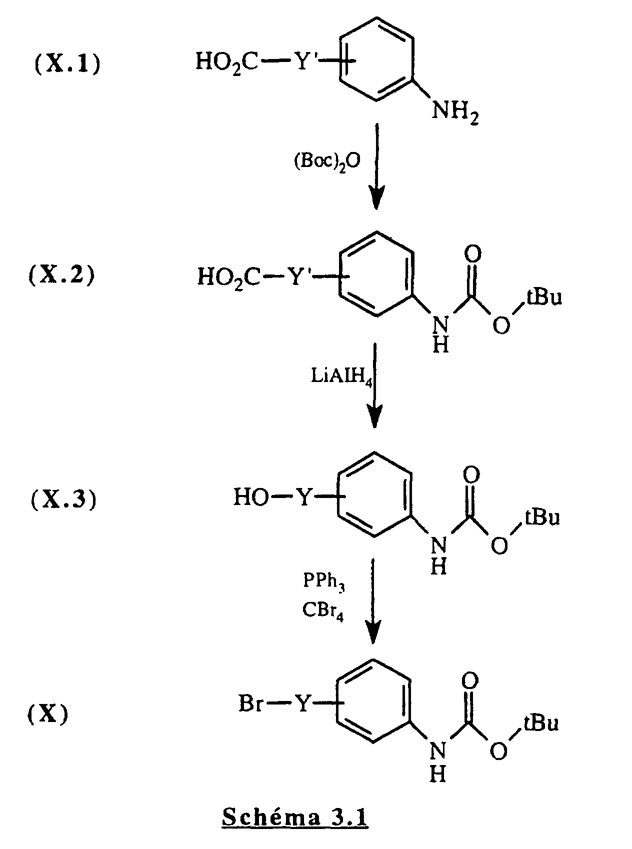

### Synthèse des Intermédiaires (XIII) :

Les intermédiaires de formule générale **(XIII),** schéma 5.1, peuvent être préparés à partir des dérivés (R ou S) des acides thiazolidine carboxyliques en présence de (Boc)₂O dans des conditions classiques.

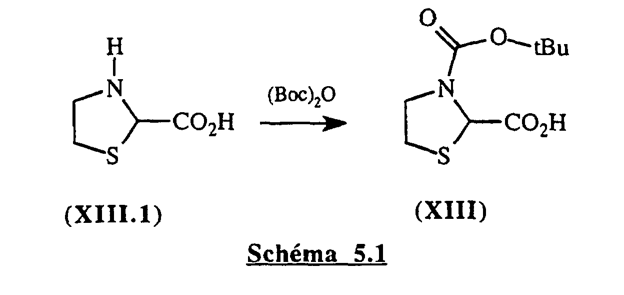

### Synthèse des Intermédiaires (XVI) :

Les intermédiaires de formule générale **(XVI),** schéma 6.1, peuvent être préparés à partir des dérivés carboxamides commerciaux de formule générale **(XVI.1).** Ces carboxamides sont traités par le réactif de Lawesson dans un solvant tel que le 1, 4-dioxanne pendant 2 à 3 heures à une température variant de **25°C au reflux du mélange**. Les thiocarboxamides de formule générale **(XVI.2)** sont ensuite traités par le bromopyruvate d'éthyle, à 20°C dans le DMF selon un protocole expérimental décrit dans *J. Med. Chem*., (1983), **26**, 884-891, pour conduire aux thiazoles de formule générale **(XVI.3).** La saponification de l'ester est effectuée en 15 heures par de la potasse aqueuse en solution dans l'acétone.

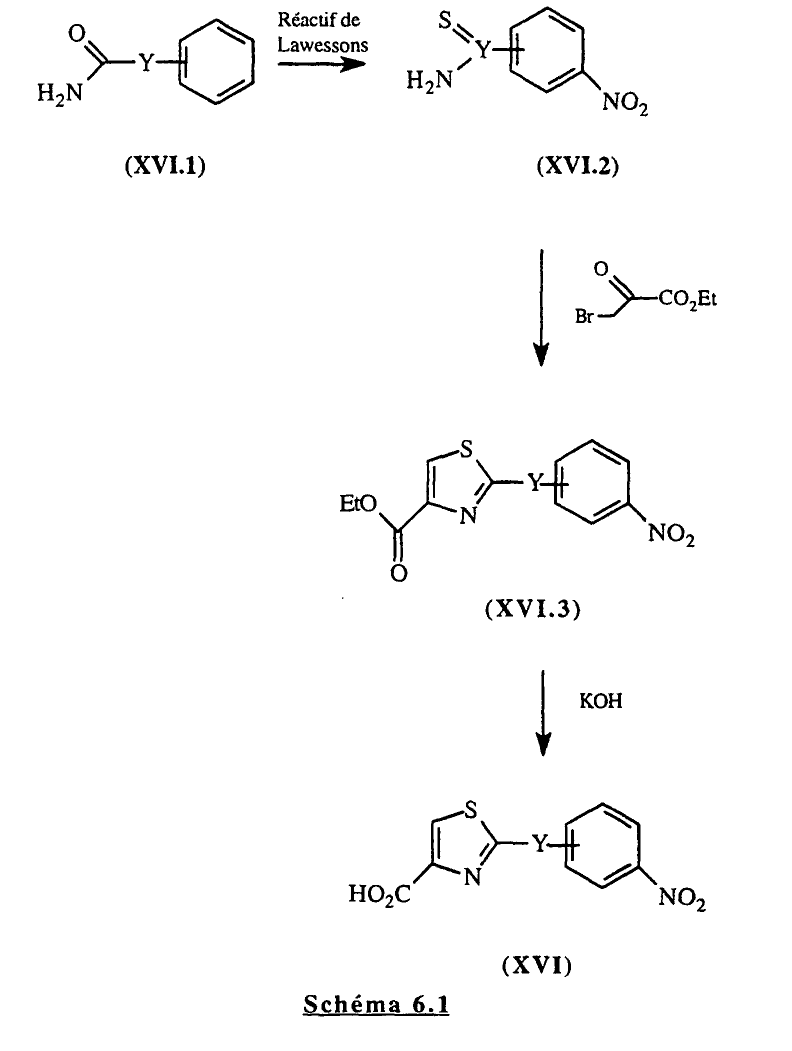

Les tétrahydropyridines de formule générale **(XVI),** schéma 6.2, peuvent être préparées à partir de l'acide tétrahydro-4-pyridine carboxylique commercial. L'estérification est effectuée classiquement en présence d'acide para-toluène sulfonique, dans le méthanol, pour conduire à l'intermédiaire **(XVI.4)** qui est ensuite condensé sur un dérivé halogéné de formule générale **(IX)** dans des conditions précédemment décrites. L'acide de formule générale **(XVI)** est obtenu par saponification en présence, par exemple, de LiOH ou de KOH.

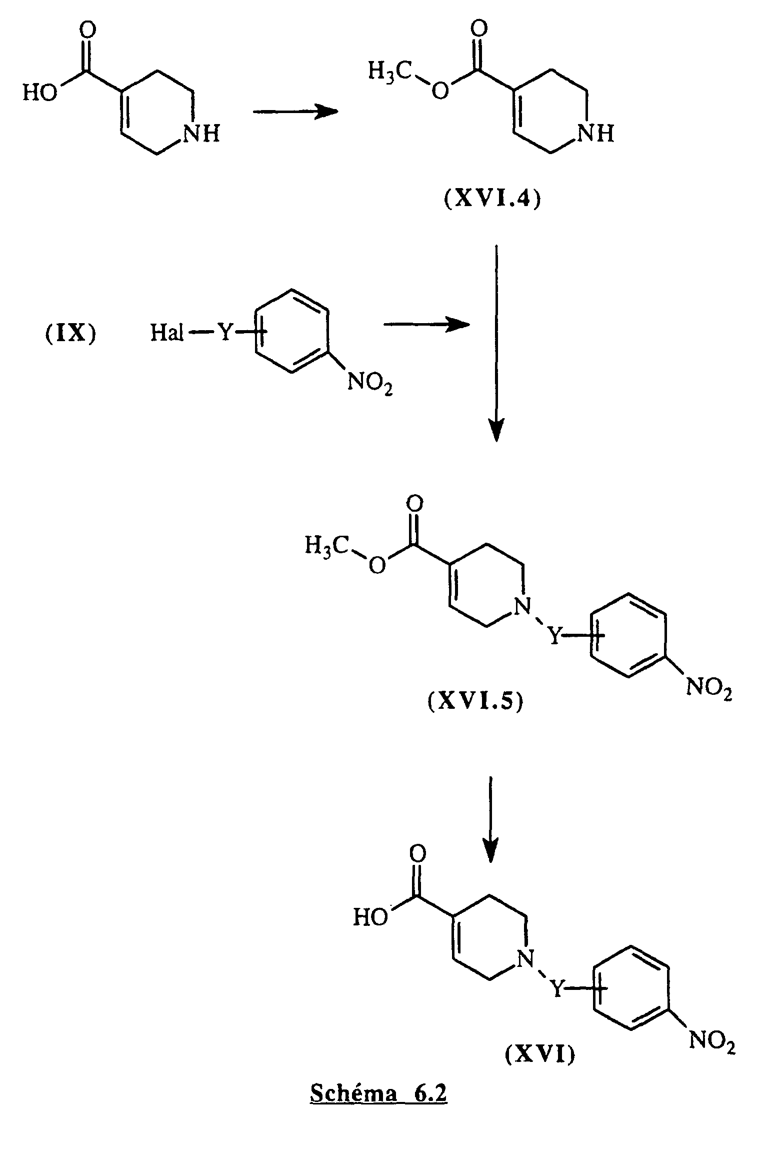

### Synthèse des Intermédiaires (XXII) :

Les synthèses des intermédiaires de formule générale **(XXII)** sont décrites dans les schémas 10.1 et 10.2.

La fonction tosylate des dérivés de la proline (L ou D) de formule générale **(XXII.1)** (Tetrahedron Lett., (1983), **24** (33), 3517-3520), schéma 10.1, est substituée par l'alcoolate des dérivés de formule générale **(XXII.2),** généré in situ par une base telle que NaH. La substitution est effectuée à 20° C dans un solvant telle que la N-methylpyrrolidinone qui conduit à une inversion propre de la configuration du carbone siège de la réaction (Tetrahedron Lett., (1983), **24** (33), 3517-3520). Les intermédiaires de formule générale **(XXII.3)** ainsi obtenus sont ensuite classiquement saponifiés par de la potasse alcoolique.

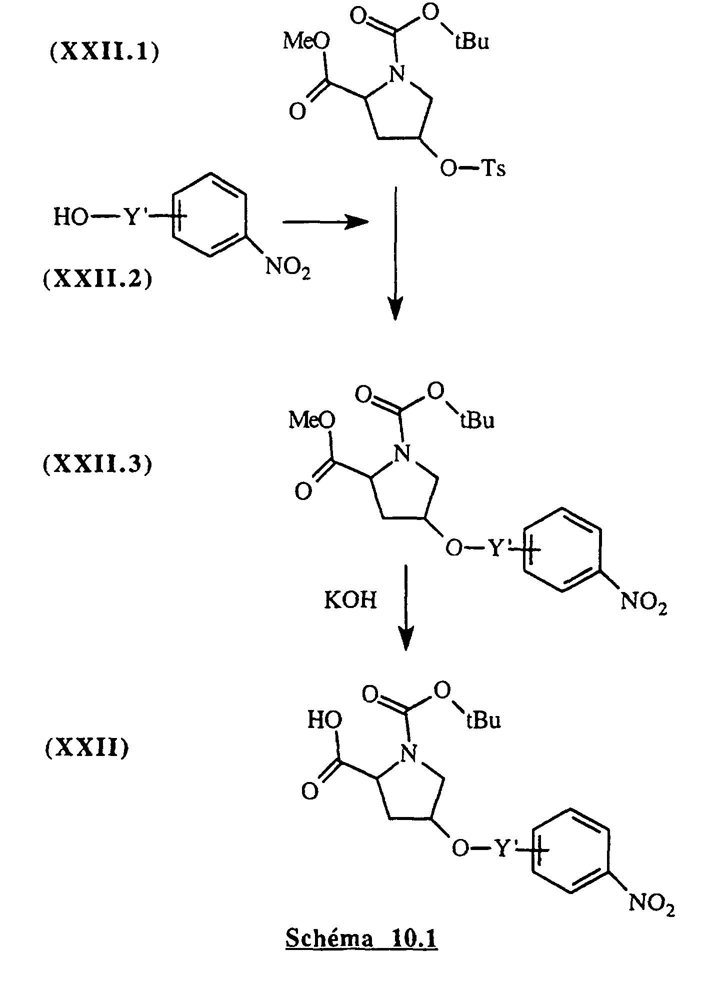

Les intermédiaires de formule générale **(XXII)** peuvent également être préparés (schéma 10.2) à partir de la condensation de la cystéine (L ou D) sur un aldéhyde de formule générale **(XXII.5)** selon un protocole expérimental décrit dans la littérature (*J. Org. Chem.,* (1957), **22**, 943-946). L'amine de l'hétérocycle est ensuite protégée sous forme de carbamate pour conduire aux intermédiaires de formule générale **(XXII).** Les aldéhydes de formule générale **(XXII.5),** non commerciaux, peuvent être préparés selon *J. Chem. Soc., Perkin Trans. I*, 1973, **1**, 35.

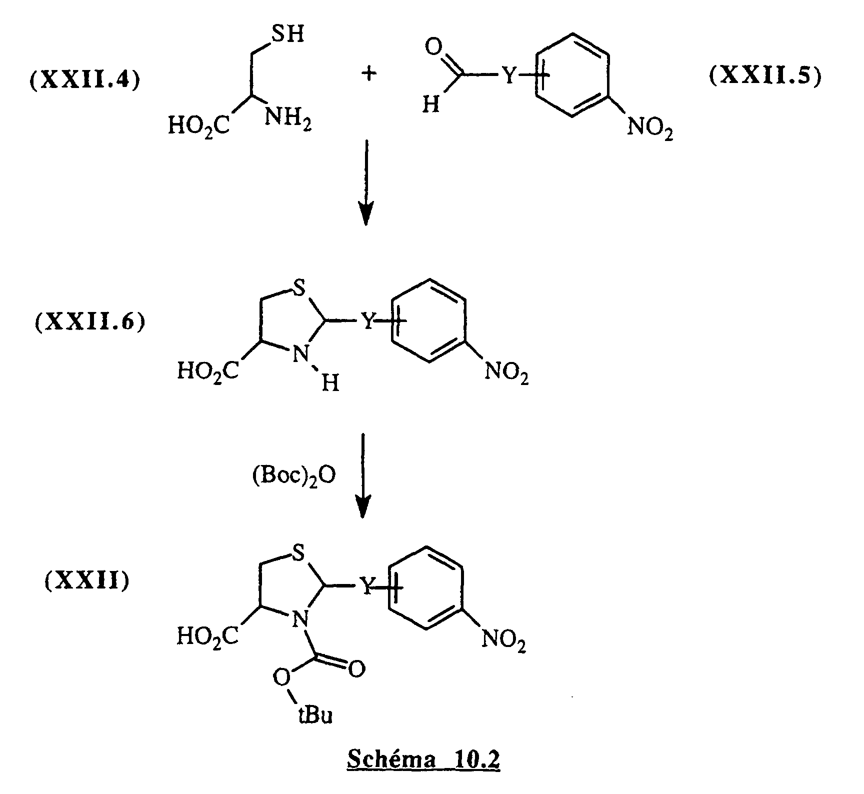

### Synthèse des Intermédiaires (XXIV) :

La synthèse des intermédiaires de formule générale **(XXIV)** est décrite dans le schéma 12.1.

La condensation des amines (R ou S) de formule générale **(XXIV.1),** schéma 12.1, sur les dérivés halogénés de formule générale **(IX)** est effectuée en présence d'une base telle que le carbonate de potassium dans un solvant comme le DMF. Le produit de condensation **(XXIV.2)** est ensuite déprotégé en milieu acide pour conduire aux intermédiaires de formule générale **(XXIV).**

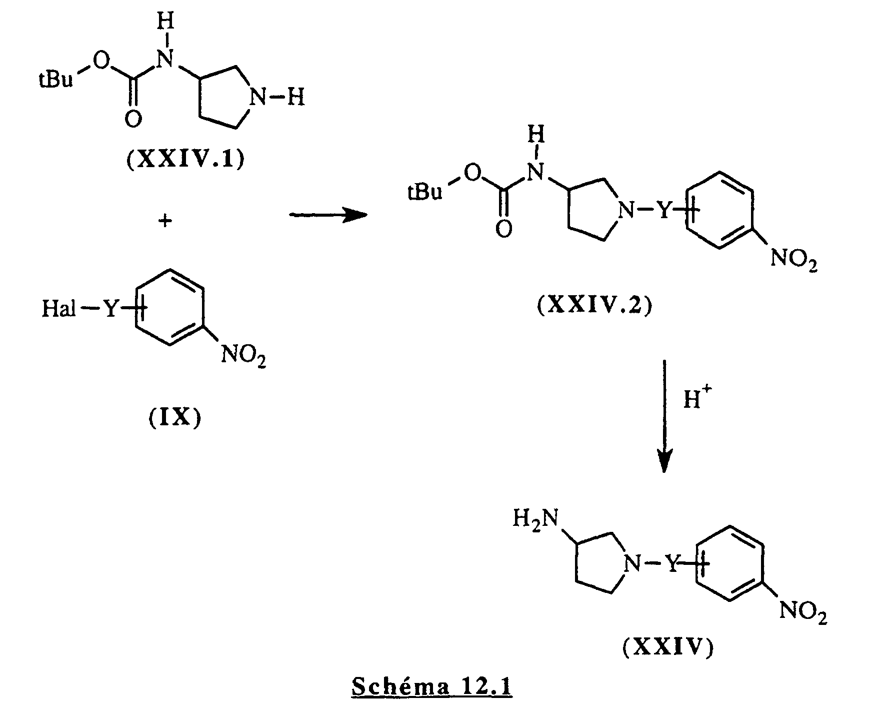

### Synthèse des Intermédiaires (XXV) :

Les synthèses des intermédiaires de formule générale **(XXV)** sont décrites dans les schémas 13.1, 13.2, 13.3 et 13.4.
Les **imidazoles** de formule générale **(XXV),** schéma 13.1, peuvent être préparés en 4 étapes à partir des composés **(XXV.1)** et **(XXV.2)** commerciaux. La condensation entre les bromoacétophénones de formule générale **(XXV.1)** et les acides carboxyliques de formule générale **(XXV.2)** est effectuée en présence de carbonate de Césium dans le DMF. Le cétoester obtenu **(XXV.3)** est cyclisé en présence de 15 équivalents d'acétate d'ammonium par chauffage dans un mélange de xylènes et élimination simultanée de l'eau formée au cours de la réaction pour conduire aux imidazoles de formule générale **(XXV.4)**. L'azote de l'hétérocycle est ensuite protégé, par exemple à **l'aide de 2-(Triméthylsilyl)éthoxyméthyl (SEM)** ou par un autre groupe protecteur cité dans : *Protective groups in organic synthesis*, 2d ed., (John Wiley & Sons Inc., 1991), pour conduire aux intermédiaires de formule générale **(XXV.5)**. La libération de l'amine de la chaîne peut être effectuée par hydrogènolyse en présence de Pd/C.

Alternativement, les intermédiaires de formule générale **(XXV.4)** peuvent être alkylés en présence d'une base telle que, par exemple, K₂CO₃, et d'un réactif comme R₃-X dans un solvant tel que le DMF ou l'acétonitrile pour conduire aux imidazoles de formule générale **(XXV.6)**. La déprotection de la chaîne latérale, comme précédemment décrite, permet d'accéder aux intermédiaires de formule générale **(XXV)**.

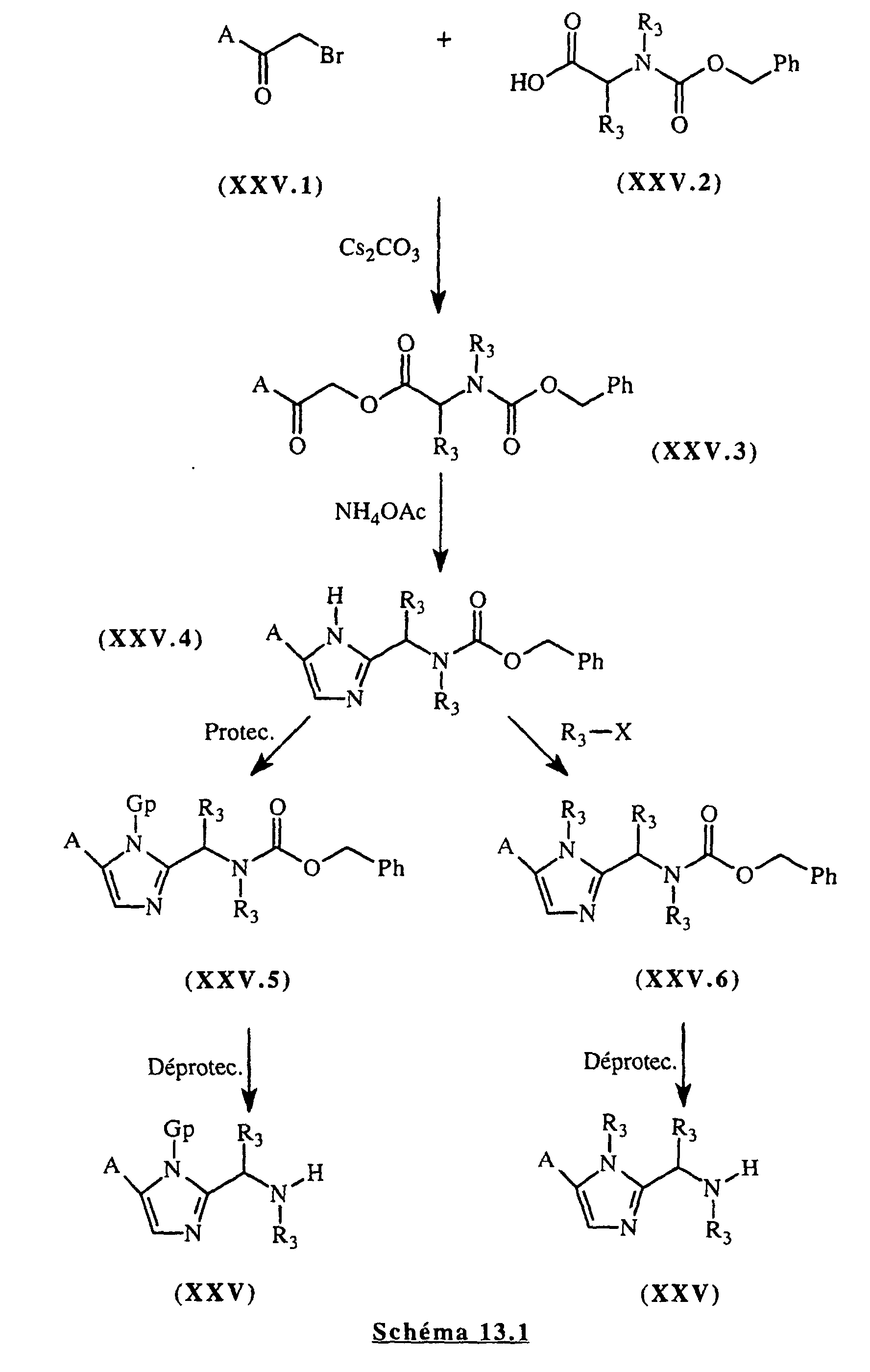

Les intermédiaires de formule générale **(XXV)** comportant un oxazole, thiazole ou un imidazole sont également accessibles à travers d'autres voies synthétiques telle que celle décrite dans *Bioorg. and Med. Chem. Lett.*,1993, **3**, 915 ou *Tetrahedron Lett.,* 1993, **34**, 1901. Les intermédiaires de formule générale **(XXV.7)** ainsi obtenus peuvent être modifiés, schéma 13.2, par saponification suivi d'une décarboxylation, par exemple thermique, pour conduire aux hétérocycles disubstitués de formule générale **(XXV.9)**. La libération de l'amine de la chaîne latérale, comme précédemment décrite, permet d'accéder aux intermédiaires de formule générale **(XXV).**

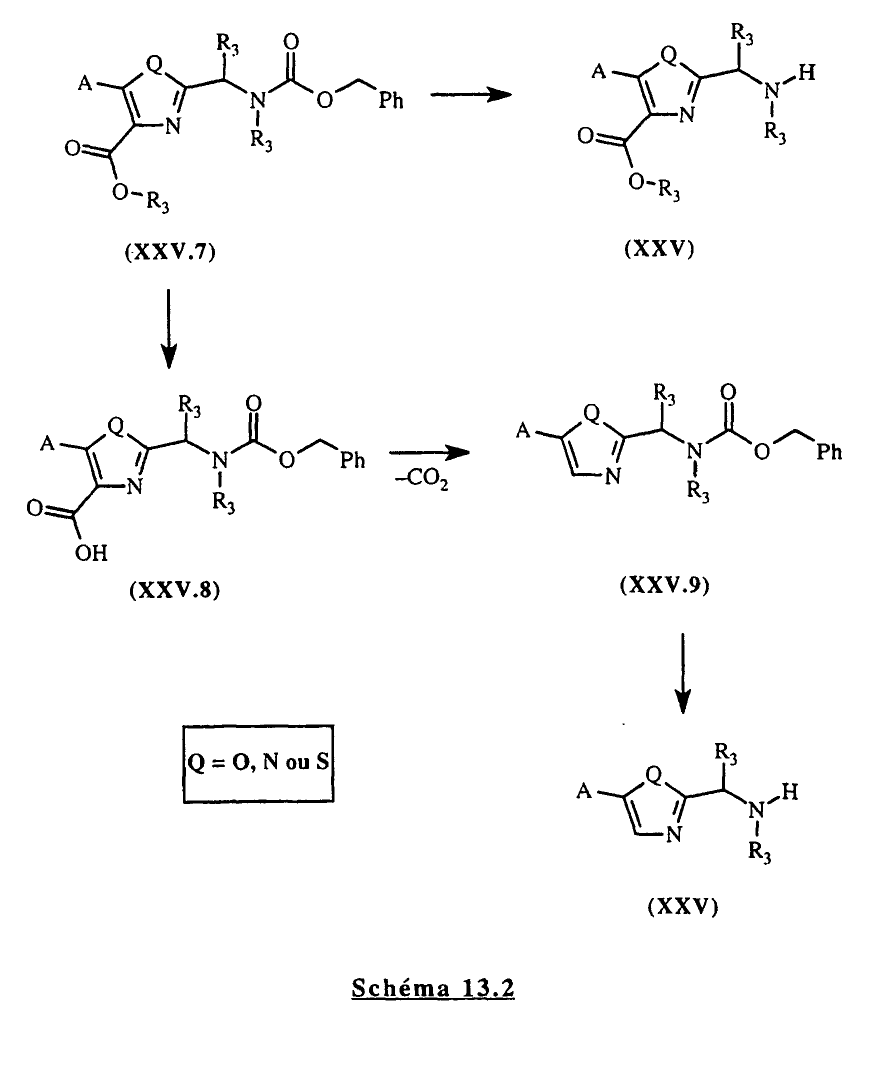

Alternativement, la fonction carboxylique des hétérocycles de formule générale **(XXV.7)**, peut être réduite, par exemple par NaBH₄, pour conduire aux dérivés alcooliques de formule générale **(XXV.10)**, schéma 13.3, qui peuvent être alkylés en présence de R₃-X et d'une base telle que K₂CO₃ dans un solvant tel que l'acétonitrile ou le DMF. La libération de l'amine de la chaîne latérale, comme précédemment décrite, permet d'accéder aux intermédiaires de formule générale **(XXV).**

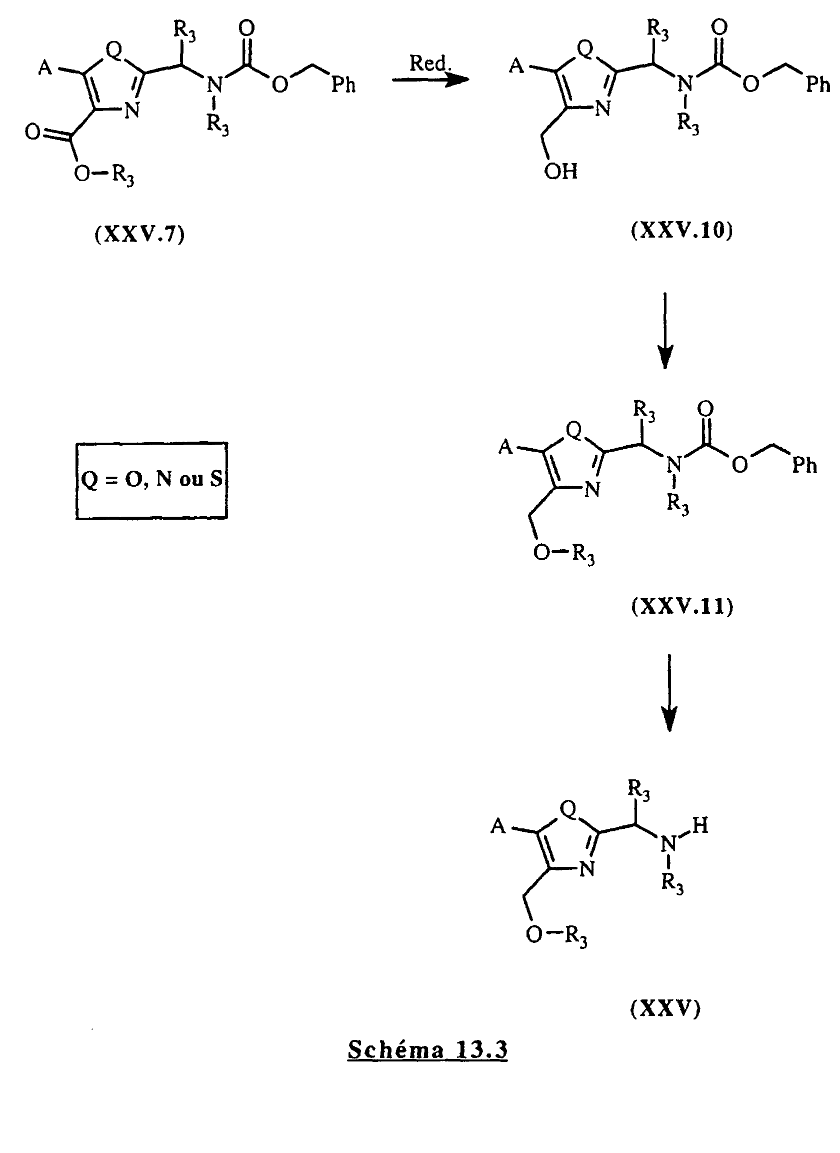

Les thiazoles de formule générale **(XXV),** schéma 13.4, peuvent également être préparés en 4 étapes à partir du chlorhydrate de sarcosinamide commercial. L'amine est d'abord classiquement protégée sous forme de carbamate de tBu et la fonction carboxamide est transformée en thiocarboxamide en présence du réactif de Lawesson. La formation du cycle thiazole est effectuée par réaction du thiocarboxamide avec l'intermédiaire de formule générale **(XXV.1)** selon un protocole expérimental décrit dans la littérature (*J. Org. Chem.,* (1995), **60,** 5638-5642). La fonction amine est régénérée par traitement de l'intermédiaire de formule générale **(XXV.12)** en milieu acide fort tel que, par exemple, l'acide trifluoroacétique.

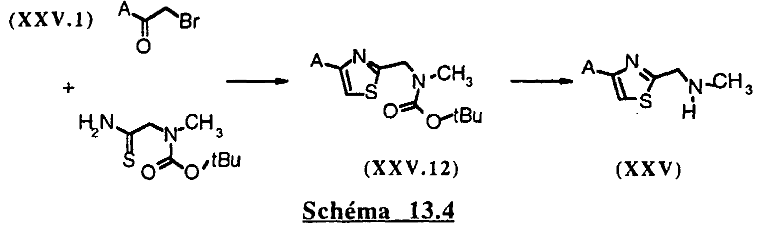

### Synthèse des Intermédiaires (XXVIII) :

Les isoxazolines et isoxazoles de formule générale **(XXVIII),** Schéma 17.1, sont préparées par réaction des aldéhydes commerciaux de formule générale **(XX)** avec le chlorhydrate d'hydroxylamine. L'oxime de formule générale **(XXVIII.1)** ainsi obtenue est activée sous forme de chlorure d'oxime, de formule générale **(XXVIII.2),** par réaction avec la N-chlorosuccinimide dans le DMF avant de réagir avec les esters de formule générale **(XXVIII.3)** pour conduire aux dérivés isoxazolines ou bien avec les esters de formule générale **(XXVIII.4)** pour conduire aux dérivés isoxazoles selon un protocole expérimental décrit dans la littérature (*Tetrahedron Lett.,* 1996, **37** (26), 4455 ; *J. Med. Chem*., 1997, **40**, 50-60 et 2064-2084). La saponification des isoxazolines ou isoxazoles de formule générale **(XXVIII.5)** est ensuite effectuée classiquement dans des conditions précédemment décrites.

Les esters insaturés de formule générale **(XXVIII.3)** et **(XXVIII.4),** non commerciaux, peuvent être préparés selon des méthodes décrites dans la littérature (*J. Med. Chem.,* 1987, **30**, 193 ; *J. Org. Chem.,* 1980, **45**, 5017).

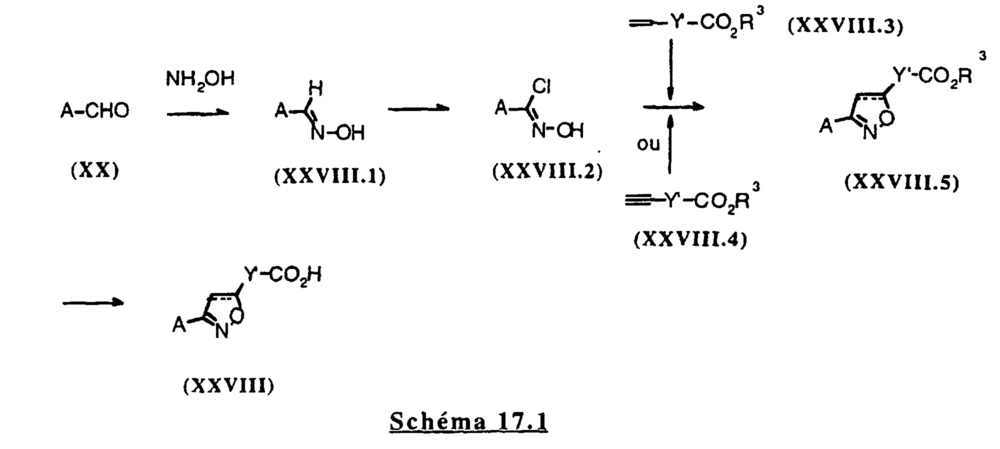

### Synthèse des Intermédiaires (XXIX) :

Les synthèses des intermédiaires de formule générale **(XXIX)** sont décrites dans les schémas 18.1,18.2,18.3 et 18.4

Les intermédiaires de formule générale **(XXIX)** peuvent être préparés, schéma 18.1, à partir des intermédiaires de formule générale **(XXII.3),** précédemment décrits, par traitement en milieu acide fort pour régénérer la fontion amine hétérocyclique. La réduction sélective de la fonction carboxylique en présence, par exemple, de borohydrure de sodium dans un solvant tel que, par exemple, le THF anhydre, permet d'obtenir l'intermédiaire de formule générale **(XXIX)** portant une fonction alcool primaire sans toucher le groupement nitro (Rao, A. V. R., *J. Chem. Soc. Chem. Commun*., 1992, **11**, 859).

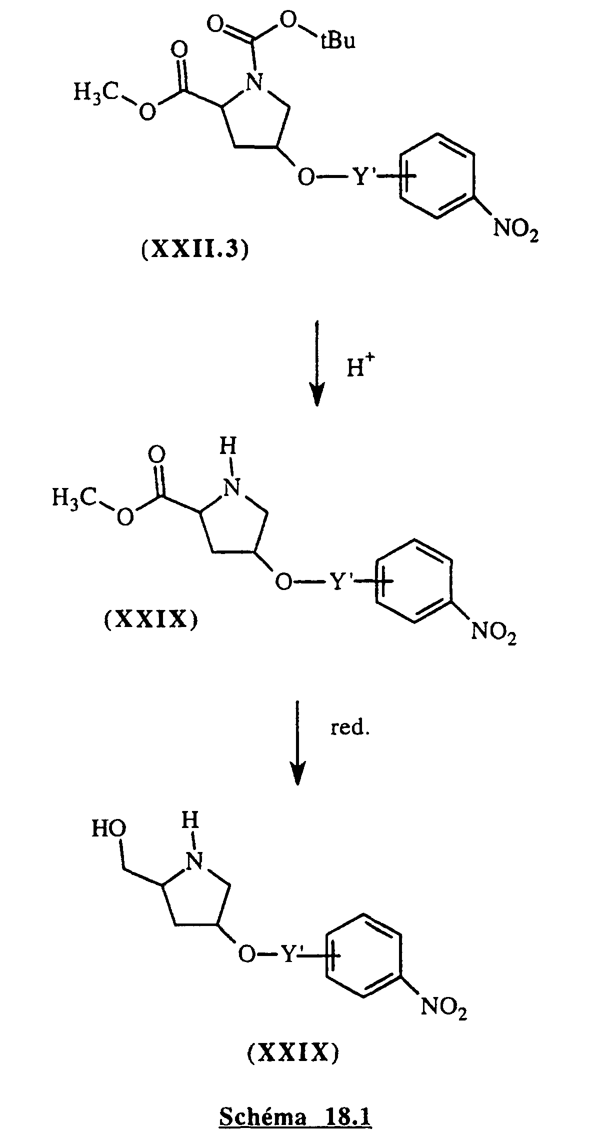

Les intermédiaires de formule générale **(XXIX)** peuvent également être préparés, schéma 18.2, à partir des intermédiaires de formule générale **(XXIX.1)** (R ou S) dont la préparation est analogue à celle des composés de formule générale **(XXII.1)**. La condensation des dérivés alcooliques de formule générale **(XXII.2)** sur les intermédiaires de formule générale **(XXIX.1)** est également décrite ci-dessus. La libération de l'amine hétérocyclique est effectuée en présence d'une solution organique d'acide fort, par exemple, l'acide trifluoroacétique.

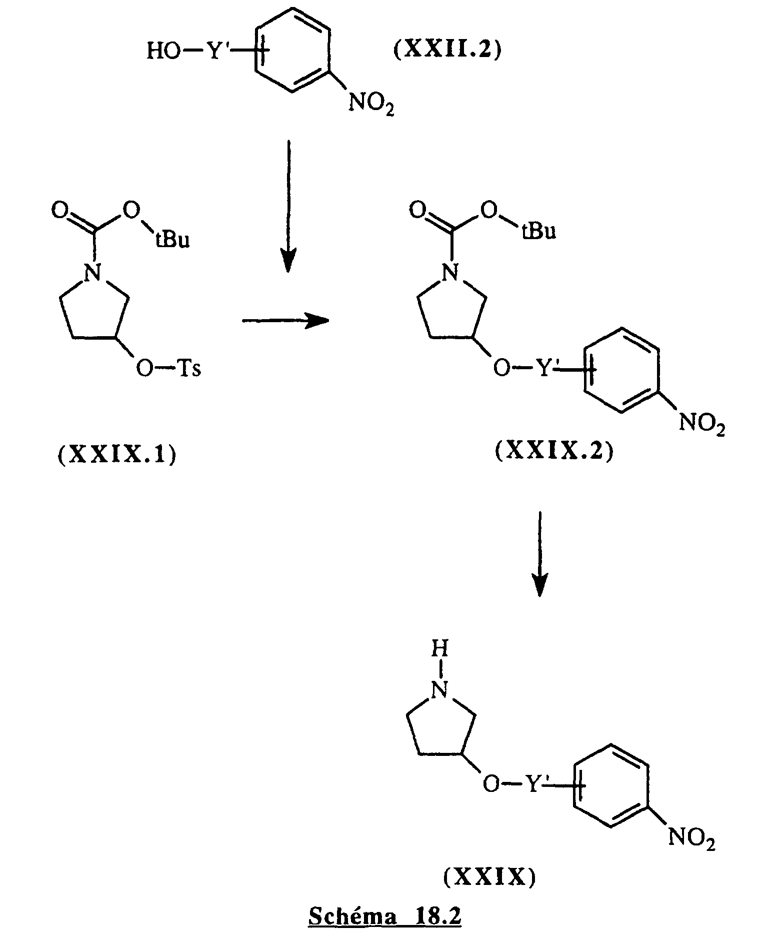

Les amines de formule générale **(XXIX)**, schéma 18.3, sont également accessibles à partir de la substitution des dérivés tosylés de formule générale **(XXIX.1)** par les amines commerciales de formule générale **(XXX)**. La coupure de la fonction carbamate des intermédiaires de formule générale **(XXIX.3)** est effectuée comme précédemment décrite.

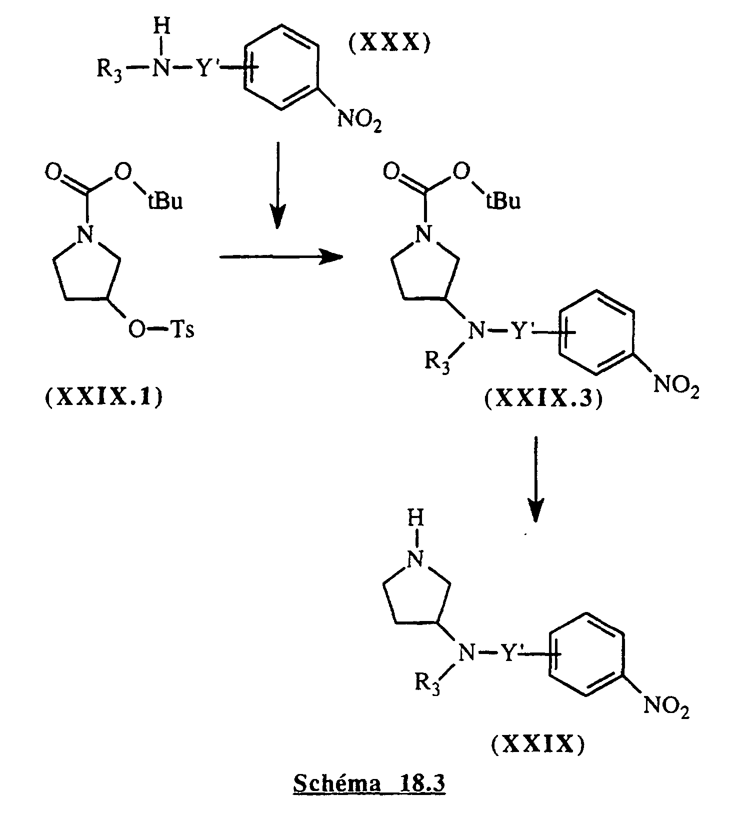

Les intermédiaires de formule générale **(XXIX)** peuvent également être préparés, schéma 18.4, par réaction des dérivés halogénés de formule générale **(IX)** avec un alcool de formule générale **(XXIX.4)** en présence d'une base telle que par exemple tBuO⁻K⁺ dans un solvant anhydre tel que le THF. L'intermédiaire de formule générale **(XXIX.5)** ainsi obtenu est ensuite déprotégé en milieu acide fort (HCl ou TFA).

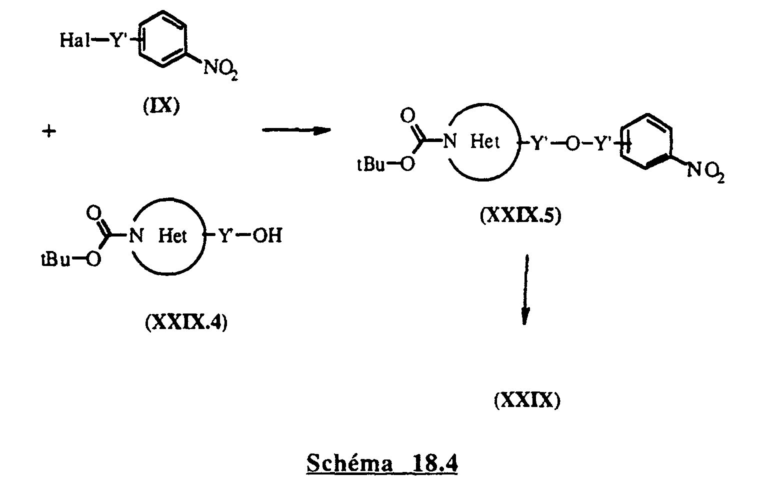

### Synthèse des intermédiaires (XXXII) :

Les intermédiaires de formule générale **(XXXII)** peuvent être préparés, schéma 20.1, par réaction des dérivés halogénés de formule générale **(IX)** avec la 1-(diphénylméthyl)-3-hydroxyazétidine commercial **(XXXII.1)** en présence d'une base telle que par exemple NaH dans un solvant anhydre tel que le THF. Dans ce cas, le groupement nitro de l'intermédiaire de formule générale **(XXXII.2)** est réduit en présence de SnCl₂, comme précédemment décrit, pour conduire à l'intermédiaire de formule générale **(XXXII.3)** dont l'amine est ensuite protégée sous forme d'un carbamate de tButyl. La coupure du groupe protecteur diphénylméthyl est ensuite effectuée classiquement par hydrogènolyse en présence de Pd(OH)₂ pour conduire à l'intermédiaire de formule générale **(XXXII).**

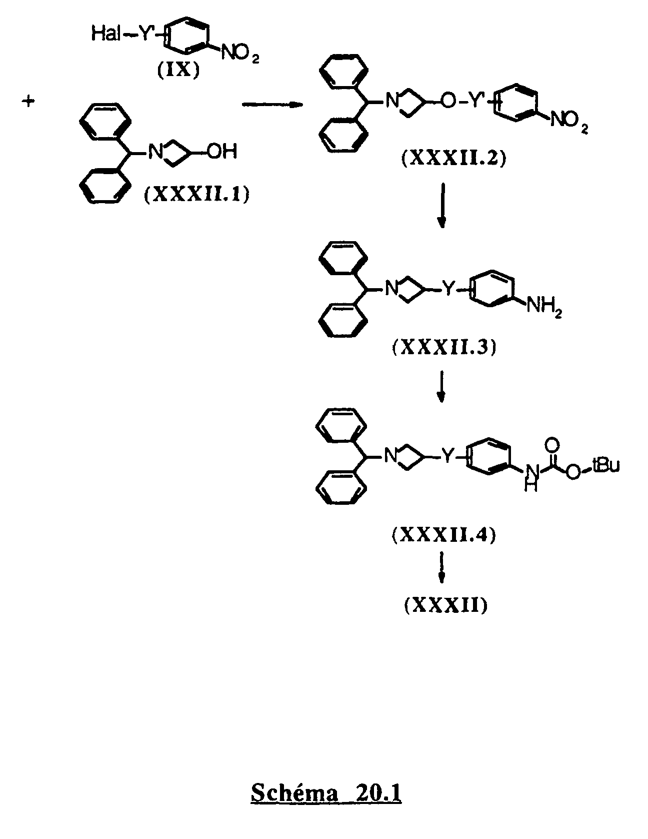

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES :

### Exemple 1 : Iodhydrate de N-[4-(1H-imidazol-1-yl)phényl]-2-thiophènecarboximidamide (1) :

### 1.1 1-(4-nitrophényl)-1H-imidazole :

9 g (64,5 mmoles) de carbonate de potassium et 5 g (3,75 ml ; 35,2 mmoles) de 1-fluoro-4-nitrobenzène sont ajoutés à une solution de 2 g d'imidazole (29,4 mmoles) dans 14 ml de DMF. Le mélange réactionnel est agité pendant 1,5 heure à 110° C. De l'acétate d'éthyle (50 ml) est ajouté dans le milieu qui est lavé par 3 fois 50 ml d'eau. Les phases organiques sont séchées sur sulfate de magnésium et concentrées sous vide. 4,4 g de produit sont ainsi obtenus (rendement = 80%) sous forme d'huile incolore et utilisés sans autre purification dans les étapes suivantes.
RMN ¹H (CDCl₃, 100 MHz, δ) : 6,92 (t, 1H, H arom. imidazole), 7,16 (s, 1H, H arom. imidazole), 7,24-7,32-8,18-8,27 (4s, 4H, H arom.), 7,59 (s, 1H, H arom. imidazole).

### 1.2 1-(4-aminophényl)-1H-imidazole :

Le 1-(4-nitrophényl)-1H-imidazole (4,4 g ; 23,5 mmoles) est mis en solution dans du méthanol anhydre (140 ml) et du palladium sur charbon (0,44 g) est ajouté dans le milieu. L'ensemble est placé sous hydrogène pendant 4 heures. Le catalyseur est filtré et le solvant évaporé à sec. Le produit attendu est obtenu quasi-pur avec un rendement de 89 % (3,3 g).
RMN ¹H (CDCl₃, 100 MHz, δ) : 6,61-6,69-6,95-7,05 (4s, 4H, H arom.), 6,88 (t, 1H, H arom. imidazole), 7,07 (s, 1H, H arom. imidazole), 7,52 (s, 1H, H arom. imidazole).

### 1.3 Iodhydrate de N-[4-(1H-imidazol-1-yl)phényl]-2-thiophènecarboximidamide (1) :

Le 1-(4-aminophényl)-1H-imidazole (0,3 g ; 1,7 mmoles) et l'iodhydrate de S-méthyl-2-thiophènethiocarboximide (0,5 g ; 1,75 mmoles) sont mis en solution dans 1 ml d'isopropanol et 1 ml de DMF et le mélange réactionnel est agité pendant 18 heures à 25° C. Le précipité formé est filtré et lavé par 15 ml de dichlorométhane et 15 ml d'éthanol. Le produit attendu est ainsi obtenu (0,48 g ; 73 %) sous forme salifiée (iodhydrate). Point de fusion : 252-253° C (décomposition).
RMN ¹H (DMSO, 400 MHz, δ): 7,24 (s, 1H, H arom.), 7,38 (t, 1H, H arom.), 7,55-7,57-7,85-7,87 (4s, 4H, H arom.), 7,89 (s, 1H, H arom.), 8,10 (m, 2H, H arom.), 8,50 (s, 1H, H arom.).
IR : ν_{C=N} (amidine) : 1585 cm⁻¹.

### Exemple 2 : N-[4-(3-thiazolidinylméthyl)phényl]-2-thiophènecarboximidamide (2) :

### 2.1 1-bromométhyl-4-nitrobenzène :

L'alcool 4-nitrobenzylique (6 g, 39 mmoles) est mis en solution dans du dichlorométhane (100 ml) et additionné de tétrabromure de carbone (14,9 g, 45 mmoles). La triphénylphosphine (11,8 g, 45 mmoles) est ajoutée par parties dans le milieu à 0° C. Puis le mélange est agité pendant 2 heures à température ambiante. Le solvant est évaporé et le produit obtenu purifié sur gel de silice dans un mélange acétate d'éthyle / heptane (1/2). Il est obtenu sous forme de cristaux blancs en aiguilles (7,2 g ; 85 %). Point de fusion : 97-98° C.
RMN ¹H (CDCl₃, 100 MHz, δ) : 4,53 (s, 2H, CH₂), 7,53-7,61-8,18-8,27 (4 s, 4H, H arom.).

### 2.2 3-(4-nitrobenzyl)-thiazolidine :

Un mélange de thiazolidine (0,9 g, 10 mmoles) et de carbonate de potassium (2,5 g, 18 mmoles) dans l'acétonitrile (10 ml) est porté à 70° C. Le 1-bromométhyl-4-nitrobenzène (2 g, 9,2 mmoles) en solution dans de l'acétonitrile (25 ml) est ajouté goutte à goutte et la réaction est maintenue 2 heures au reflux.

Le précipité formé est filtré, les eaux-mères sont évaporées et le résidu est repris par 50 ml de dichlorométhane et lavé par 3 fois 50 ml d'eau. Les phases organiques sont séchées, évaporées et purifiées sur gel de silice dans un mélange acétate d'éthyle / heptane (1/2). Le produit attendu est obtenu sous forme d'huile incolore (1,5 g, 72 %).
RMN ¹H (CDCl₃, 100 MHz, δ) : 3,05 (m, 4H, 2CH₂), 3,68 (s, 2H, CH₂-S), 4,04 (s, 2H, CH₂), 7,53-7,62-8,17-8,26 (4s, 4H, H arom.).

### 2.3 3-(4-aminobenzyl)-thiazolidine :

La 3-(4-nitrobenzyl)-thiazolidine (1,1 g, 5 mmoles) est mise en solution dans 10 ml d'acide chlorhydrique concentré à 0°C. Le chlorure d'étain dihydraté (7,7 g, 34 mmoles) est ajouté par parties. Le mélange est chauffé pendant 2 heures au reflux et l'acide est évaporé sous pression réduite. Le résidu est ensuite repris par 20 ml d'eau et neutralisé par une solution de soude 2N (environ 100 ml). 100 ml de dichlorométhane sont ajoutés au milieu et l'ensemble est filtré sur célite afin d'éliminer les sels en suspension. La phase organique est extraite, lavée par 3 fois 50 ml d'eau, séchée filtrée et évaporée à sec sous pression réduite. Le produit attendu est purifié sur gel de silice dans un mélange dichlorométhane / méthanol (98/2) et obtenu sous forme de poudre beige (0,6 g , 63 %). Point de fusion : 73-74° C.
RMN ¹H (CDCl₃, 100 MHz, δ) : 3,02 (m, 4H, 2CH₂), 3,44 (s, 2H, CH₂), 3,66 (s large, 2H, NH₂), 4,07 (s, 2H, CH₂), 6,62-6,71-7,10-7,27 (4 s, 4H, H arom.).

### 2.4 [4-(3-thiazolidinylméthyl)phényl]-2-thiophènecarboximidamide (2) :

La 3-(4-aminobenzyl)-thiazolidine (0,6 g, 3 mmoles) et l'iodhydrate de S-méthyl-2-thiophènethiocarboximide (1,14 g, 4 mmoles) sont mis en solution dans 7 ml d'un mélange isopropanol / DMF (2/5). L'ensemble est agité 18 heures à température ambiante. On ajoute ensuite 10 ml d'acétate d'éthyle dans le milieu et on extrait le produit de la réaction par 3 fois 10 ml d'eau. La phase aqueuse est collectée et basifiée par une solution saturée d'hydrogénocarbonate de sodium, puis le produit est extrait par 3 fois 10 ml d'acétate d'éthyle. Il est purifié sur gel de silice dans un mélange dichlorométhane / méthanol (95/5) et obtenu sous forme de poudre blanche (0,6 g, 65 %). Point de fusion : 161,5-163,5° C.
RMN ¹H (CDCl₃, 400 MHz, δ) : 2,98 (t, 2H, CH₂), 3,14 (t, 2H, CH₂), 3,54 (s, 2H, CH₂), 4,10 (s, 2H, CH₂), 4,85 (s large, 2H, NH₂), 6,98 (s, 1H, H arom.), 7,00 (s, 1H, H arom.), 7,10 (t, 1H, thiophène), 7,34 (s, 1H, H arom.), 7,36 (s, 1H, H arom.), 7,42 (t, 1H, thiophène), 7,45 (m, 1H, thiophène).
IR : ν_{C=N} (amidine) : 1593 cm⁻¹.

### Exemple 3 : Fumarate de N-[4-(1,2,3,6-tétrahydropyridin-1-yl)phényl]-2-thiophènecarboximidamide (3) :

### 3.1 1-(4-nitrophényl)-1,2,3,6-tétrahydropyridine :

Le protocole expérimental utilisé est le même que celui décrit pour l'intermédiaire 1.1, la 1,2,3,6-tétrahydropyridine remplaçant l'imidazole. Huile incolore.
RMN ¹H (CDCl₃, 100 MHz, δ) : 2,33 (m, 2H, CH₂), 3,59 (t, 2H, CH₂), 3,90 (m, 2H, CH₂), 5,90 (m, 2H, CH=CH), 6,75-6,82-8,07-8,18 (m, 4H, H arom.).

### 3.2 1-(4-aminophényl)-1,2,3,6-tétrahydropyridine :

Le protocole expérimental utilisé est le même que celui décrit pour l'intermédiaire 2.3, la 1-(4-nitrophényl)-1,2,3,6-tétrahydropyridine remplaçant la 3-(4-nitrobenzyl)-thiazolidine. Huile incolore.
RMN ¹H (CDCl₃, 100 MHz, δ) : 2,31 (m, 2H, CH₂), 3,21 (t, 2H, CH₂), 3,43 (m, 2H, NH₂), 3,56 (m, 2H, CH₂), 5,84 (m, 2H, CH=CH), 6,75 (m, 4H, H arom.).

### 3.3 Fumarate de N-[4-(1,2,3,6-tétrahydropyridin-1-yl)phényl]-2-thiophènecarboximidamide (3) :

Le protocole expérimental utilisé est le même que celui décrit pour l'intermédiaire 1.3, la 1-(4-aminophényl)-1,2,3,6-tétrahydropyridine remplaçant le 1-(4-aminophényl)-1H-imidazole. Poudre beige. Point de fusion : 193-194° C.
RMN ¹H (DMSO, 400 MHz, δ) : 2,23 (m, 2H, CH₂), 3,29 (m, 2H, CH₂), 3,61 (m, 2H, CH₂), 5,84 (m, 2H, CH=CH), 6,56 (s, 1H, acide fumarique), 6,89 (m, 4H, H arom.), 7,13 (m, 1H, H arom.), 7,67 (m, 1H, H arom.), 7,77 (m, 1H, H arom.).
IR : ν_{C=N} (amidine) : 1560 cm⁻¹.

### Exemple 4 : Chlorhydrate de N-[4-(1H-imidazol-1-yl méthyl)phényl]-2-thiophènecarboximidamide (4) :

### 4.1 1-(4-nitrobenzyl)-1H-imidazole :

Le protocole expérimental utilisé est le même que celui décrit pour l'intermédiaire 1.1, le 1-bromométhyl-4-nitrobenzène remplaçant le 1-fluoro-4-nitrobenzène. Huile incolore.
RMN ¹H (CDCl₃, 100 MHz, δ) : 5,26 (s, 2H, CH₂), 6,92 (m, 1H, H imidazole), 7,16 (m, 1H, H imidazole), 7,59 (m, 1H, H imidazole), 7,24-7,32-8,18-8,27 (4s, 4H, H arom.).

### 4.2 1-(4-aminobenzyl)-1H-imidazole :

Le protocole expérimental utilisé est le même que celui décrit pour l'intermédiaire 1.2, le 1-(4-nitrobenzyl)-1H-imidazole remplaçant le 1-(4-aminophényl)-1H-imidazole. Poudre jaune pâle. Point de fusion : 121-122° C.
RMN ¹H (CDCl₃, 100 MHz, δ) : 2,87 (s large, 2H, NH₂), 4,98 (s, 2H, CH₂), 6,88 (m, 1H, H imidazole), 7,06 (m, 1H, H imidazole), 7,52 (m, 1H, H imidazole), 6,60-6,69-6,95-7,05 (4s, 4H, H arom.).

### 4.3 Chlorhydrate de N-[4-(1H-imidazol-1-yl méthyl)phényl]-2-thiophènecarboximidamide (4) :

Le protocole expérimental utilisé est le même que celui décrit pour l'intermédiaire 2.4, le 1-(4-aminobenzyl)-1H-imidazole remplaçant la 3-(4-aminobenzyl)-thiazolidine. Après salification par une solution molaire de HCl dans l'éther diéthylique anhydre, on obtient une poudre beige. Point de fusion : 261-263° C.
RMN ¹H (DMSO, 400 MHz, δ): 5,12 (s, 2H, CH₂), 6,46 (s large, 2H, NH₂), 6,83-6,85-7,22-7,24 (4s, 4H, H arom.), 6,90 (s, 1H, H arom.), 7,09 (t, 1H, H arom.), 7,20 (s, 1H, H arom.), 7,60 (d, 1H, H arom.), 7,74 (s, 2H, H arom.).
IR : ν_{C=N} (amidine) : 1599 cm⁻¹.

### Exemple 5 : N-[4-{2-(3-thiazolidinyl)éthyl}phényl]-2-thiophènecarboximidamide (5) :

### 5.1 Acide 4-(t-butoxycarbonylamino)-benzèneacétique :

L'acide para-aminophénylacétique (3 g, 20 mmoles) est dissous dans 60 ml d'un mélange THF /H₂O (2/1). On ajoute 11 ml de soude à 10% puis 6 g de di-t-butyl-dicarbonate (28 mmoles) en solution dans 50 ml du mélange THF / H₂O (2/1). On agite 18 heures à température ambiante, puis on évapore le THF sous pression réduite. Le milieu est ensuite acidifié (pH = 2) par une solution à 10% de sulfate acide de potassium (45 ml environ) et le produit de la réaction est extrait par 3 lavages à l'acétate d'éthyle (3 fois 50 ml). Les phases organiques sont séchées et évaporées pour donner 4,32 g (87 %) d'acide 4-(t-butoxycarbonylamino)-benzèneacétique pur sous forme de poudre beige. Point de fusion.: 149-150° C.
RMN ¹H (CDCl₃, 100 MHz, δ) : 1,52 (s, 9H, tBu), 3,60 (s, 2H, CH₂), 4,12 (s large, 1H, COOH), 6,55 (s, 1H, NH), 7,21 (m, 4H, H arom.).

### 5.2 (t-butoxycarbonylamino)-benzène éthanol :

L'acide 4-(t-butoxycarbonylamino)-benzèneacétique (2,9 g, 11,4 mmoles) est dissous dans 10 ml de THF anhydre à 0°C et ajouté à une suspension de LiAlH₄ (0,52 g, 13,6 mmoles) dans 30 ml de THF. Le mélange réactionnel est agité à température ambiante pendant 1,5 heure. On ajoute 50 ml d'acétate d'éthyle puis 20 ml de soude 2N dans le milieu. On extrait le produit attendu de la phase organique, qui est ensuite lavée par 3 fois 15 ml d'eau. La phase organique est séchée et le solvant évaporé sous pression réduite, puis le produit de la réaction est purifié sur gel de silice dans un mélange dichlorométhane / méthanol (95/5). 1,1 g (40%) sont ainsi obtenus sous forme d'huile incolore.
RMN ¹H (CDCl₃, 100 MHz, δ): 1,53 (s, 9H, tBu), 2,82 (t, 2H, CH₂), 3,83 (q, 2H, CH₂-OH), 6,47 (s, 1H, NH), 7,23 (m, 4H, H arom.).

### 5.3 (2-bromoéthyl-4-t-butoxycarbonylamino)benzène :

Le 4-(t-butoxycarbonylamino)-benzène éthanol (0,75 g, 3,1 mmoles) et le tétrabromure de carbone (1,2 g, 3,6 mmoles) sont dissous dans 20 ml de dichlorométhane à 0° C. La triphénylphosphine (0,94 g, 3,6 mmoles) est ajoutée par parties et l'ensemble est agité pendant 1 heure à température ambiante. Le solvant est évaporé sous pression réduite et le produit obtenu est purifié sur gel de silice dans un mélange acétate d'éthyle / heptane (1/2). Le 1-(2-bromoéthyl-4-t-butoxycarbonylamino)benzène est obtenu sous forme de poudre blanche (0,8 g, 84%). Point de fusion : 129-130° C.
RMN ¹H (CDCl₃, 100 MHz, δ) : 1,52 (s, 9H, tBu), 3,11 (t, 2H, CH₂), 3,54 (t, 2H, CH₂Br), 6,45 (s, 1H, NH), 7,22 (m, 4H, H arom.).

### 5.4 3-{2-[4-(t-butoxycarbonylamino)phényl]éthyl}thiazolidine :

Le protocole expérimental utilisé est le même que celui décrit pour l'intermédiaire 2.2, le (2-bromoéthyl-4-t-butoxycarbonylamino)benzène remplaçant le 1-bromométhyl-4-nitrobenzène. Huile incolore.
RMN ¹H (CDCl₃, 100 MHz, δ) : 1,52 (s, 9H, tBu), 2,90 (m, 8H, 4CH₂), 4,10 (s, 2H, N-CH₂-S), 6,46 (s, 1H, NH), 7,25 (m, 4H, H arom.).

### 5.5 3-{2-[4-aminophényl]éthyl}thiazolidine :

Dans un ballon de 100 ml contenant une solution de 616 mg (2 mmoles) de l'intermédiaire 5.4 dans 10 ml de dichlorométhane, on ajoute 2,3 g (20 mmoles) d'acide trifluoroacétique. Après une heure d'agitation, à 20° C, le mélange réactionnel est concentré à sec sous vide. Le résidu est dilué par un mélange de 20 ml de dichlorométhane et 20 ml de soude 4 N. Après décantation, la phase organique est lavée successivement par 3 x 20 ml d'eau suivi de 20 ml de saumure. La solution organique est séchée sur sulfate de sodium, filtrée et le solvant évaporé sous pression réduite pour obtenir une huile incolore avec un rendement de 72 %.
RMN ¹H (CDCl₃, 100 MHz, δ) : 2,85 (m, 8H, 4CH₂), 4,15 (s, 2H, N-CH₂-S), 7,25 (m, 4H, H arom.).

### 5.6 [4-{2-(3-thiazolidinyl)éthyl}phényl]-2-thiophènecarboximidamide (5) :

Le protocole expérimental utilisé est le même que celui décrit pour l'intermédiaire 2.4, la 3-{2-[4-aminophényl]éthyl}thiazolidine remplaçant la 3-(4-aminobenzyl)-thiazolidine. Poudre beige. Point de fusion : 60,5-61,5° C.
RMN ¹H (DMSO, 400 MHz, δ) : 2,65 (t, 2H, CH₂), 2,82 (t, 2H, CH₂), 2,91 (t, 2H, CH₂), 3,13 (t, 2H CH₂), 4,13 (s, 2H, N-CH₂-S), 6,93-6,95-7,19-7,21 (4s, 4H, H arom.), 7,09 (t, 1H, H thiophène), 7,44 (m, 2H, H thiophène).
IR : ν_{C=N} (amidine) : 1591 cm⁻¹.

### Exemple 6 : Iodhydrate de N-{4-[2-(1H-imidazol-1-yl)éthyl]phényl}-2-thiophènecarboximidamide (6) :

### 6.1 1-{2-[4-(t-butoxycarbonylamino)phényl]éthyl}-1H-imidazole :

Dans un ballon de 100 ml, on mélange 2,5 g (18 mmoles) de K₂CO₃ avec 680 mg (10 mmoles) d'imidazole dilué dans 10 ml d'acétonitrile. Le mélange réactionnel est chauffé à 70° C avant addition, goutte à goutte, d'une solution de 2 g (9,2 mmoles) de 1-bromométhyl-4-nitrobenzène en solution dans 25 ml d'acétonitrile. Après deux heures d'agitation à cette température, le mélange réactionnel est refroidi et filtré pour éliminer l'insoluble. Le filtrat est concentré sous vide et le résidu dilué dans 50 ml de dichlorométhane. La solution organique est successivement lavée par 3 x 50 ml d'eau et 50 ml de saumure. Après séchage sur Na₂SO₄, filtration, la phase organique est concentrée sous vide et le résidu purifié sur colonne de silice (éluant : dichlorométhane/méthanol : 95/5). Huile marron.
RMN ¹H (CDCl₃, 100 MHz, δ) : 1,50 (s, 9H, tBu), 2,90 (t, 2H, CH₂), 4,10 (t, 2H, CH₂), 6,50 (s, 1H, NH), 7,05 (m, 4H, H arom.), 6,85 (m, 1H, H imidazole.), 7,03 (s, 1H, H imidazole.), 7,32 (m, 1H, H imidazole).

### 6.2 1-[2-(4-aminophényl)éthyl]-1H-imidazole :

Le protocole expérimental utilisé est le même que celui décrit pour l'intermédiaire 5.5, la 1-{2-[4-(t-butoxycarbonylamino)phényl]éthyl}-1H-imidazole remplaçant la 3-{2-[4-(t-butoxycarbonylamino)phényl]éthyl}thiazolidine. Huile incolore.
RMN ¹H (CDCl₃, 100 MHz, δ) : 2,90 (t, 2H, CH₂), 3,35 (s large, 2H, NH₂), 4,10 (t, 2H, CH₂), 6,70 (m, 4H, H arom.), 6,85 (m, 1H, H imidazole.), 7,03 (s, 1H, H imidazole.), 7,32 (m, 1H, H imidazole).

### 6.3 Iodhydrate de N-{4-[2-(1H-imidazol-1-yl)éthyl]phényl}-2-thiophènecarboximidamide (6) :

Le protocole expérimental utilisé est le même que celui décrit pour l'intermédiaire 1.3, la 1-[2-(4-aminophényl)éthyl]-1H-imidazole remplaçant la 1-(4-aminophényl)-1H-imidazole. Poudre beige. Point de fusion : 214-215° C.
RMN ¹H (DMSO, 400 MHz, δ) : 3,11 (t, 2H, CH₂), 4,33 (t, 2H, CH₂), 7,29 (m, 6H, H arom.), 7,99 (m, 1H, H arom.), 8,70 (s large, 2H, NH₂).
IR : ν_{C=N} (amidine) : 1597 cm⁻¹.

### Exemple 7: Fumarate de N-{4-[2-(1,2,3,6-tétrahydropyridin-1-yl)éthyl]phényl}-2-thiophènecarboximidamide (7) :

### 7.1 1-{2-[4-(t-butoxycarbonylamino)phényl]éthyl}-1,2,3,6-tétrahydropyridine :

Le protocole expérimental utilisé est le même que celui décrit pour l'intermédiaire 6.1, la 1,2,3,6-tétrahydropyridine remplaçant la thiazolidine. Huile incolore.
RMN ¹H (CDCl₃, 100 MHz, δ) : 1,57 (s, 9H, tBu), 2,10 (m, 2H, CH₂), 2,70 (m, 6H, 3CH₂), 3,00 (m, 2H, CH₂), 5,72 (m, 2H, CH=CH), 6,48 (s, 1H, NH), 7,10 (m, 4H, H arom.).

### 7.2 1-[2-(4-aminophényl)éthyl]-1,2,3,6-tétrahydropyridine :

Le protocole expérimental utilisé est le même que celui décrit pour l'intermédiaire 5.5, la 1-{2-[4-(t-butoxycarbonylamino)phényl]éthyl}-1,2,3,6-tétrahydropyridine remplaçant la 3-{2-[4-aminophényl]éthyl}thiazolidine. Huile incolore.
RMN ¹H (CDCl₃, 100 MHz, δ) : 3,20 (m, 2H, CH₂), 3,80 (m, 6H, 3CH₂), 4,10 (m, 2H, CH₂), 4,57 (s large, 2H, NH₂), 6,90 (m, 2H, CH=CH), 8,00 (m, 4H H arom.).

### 7.3 Fumarate de N-{4-[2-(1,2,3,6-tétrahydropyridin-1-yl)éthyl]phényl}-2-thiophènecarboximidamide (7) :

Le protocole expérimental utilisé est le même que celui décrit pour l'intermédiaire 1.3, la 1-[2-(4-aminophényl)éthyl]-1,2,3,6-tétrahydropyridine remplaçant la 1-(4-aminophényl)-1H-imidazole. Poudre blanche. Point de fusion : 128-129° C.
RMN ¹H (DMSO, 400 MHz, δ) : 2,19 (m, 2H, CH₂), 2,83 (m, 6H, 3CH₂), 3,25 (m, 2H, CH₂), 5,72 (m, 2H, CH=CH), 6,58 (s, 3H, acide fumarique), 6,81-6,83-7,18-7,20 (4s, 4H, H arom.), 7,10 (t, 1H, H thiophène), 7,63 (m, 1H, H thiophène), 7,75 (m, 1H, H thiophène).
IR : ν_{C=N} (amidine) : 1620 cm⁻¹.

### Exemple 8 : N-[4-(3-thiazolidinylcarbonylméthyl)phényl]-2-thiophènecarboximidamide (8) :

### 8.1 3-[{4-(t-butoxycarbonylamino)phényl}méthylcarbonyl]thiazolidine :

L'acide 4-(t-butoxycarbonylamino)-benzèneacétique (1,4 g, 5,6 mmoles), intermédiaire 5.1, et le carbonyldiimidazole (0,9 g, 5,6 mmoles) sont dissous dans 15 ml de THF. La réaction est maintenue pendant 1 heure à température ambiante, puis la thiazolidine (0,5 g, 5,6 mmoles), en solution dans le THF (5 ml), est ajoutée dans le milieu. L'ensemble est agité à nouveau pendant 2 heures à température ambiante. On évapore les solvants sous pression réduite, puis on reprend le résidu avec 25 ml de dichlorométhane et on lave par 3 fois 15 ml d'eau. La phase organique est séchée et concentrée sous pression réduite. La 3-[{4-(t-butoxycarbonylamino)phényl}méthylcarbonyl]thiazolidine est obtenue sous forme de poudre blanche (1,43 g, 79 %) et sera utilisée sans autre purification dans les étapes suivantes. Point de fusion : 223-224° C.
RMN ¹H (CDCl₃, 100 MHz, δ) : 1,51 (s, 9H, tBu), 3,00 (m, 2H, CH₂-S), 3,67 (s, 2H, N-CH₂-S), 3,88 (m, 2H, CH₂-N), 4,52 (d, J = 16 Hz, 2H, CH₂-CO), 6,52 (s large, 1H, NH), 7,26 (m, 4H, H arom.).

### 8.2 3-[(4-aminophényl)méthylcarbonyl]thiazolidine :

La 3-[(4-aminophényl)méthylcarbonyl]thiazolidine est obtenue sous forme d'huile incolore avec un rendement de 44 % en suivant le mode opératoire décrit pour l'intermédiaire 5.5.
RMN ¹H (CDCl₃, 100 MHz, δ) : 1,62 (s large, 2H, NH₂), 2,98 (m, 2H, CH₂-S), 3,61 (s, 2H, N-CH₂-S), 3,80 (m, 2H, CH₂-N), 4,52 (d, J = 16 Hz, 2H, CH₂-CO), 6,61-6,69-7,01-7,09 (4 s, 4H, H arom.).

### 8.3 [4-(3-thiazolidinylcarbonylméthyl)phényl]-2-thiophènecarboximidamide (8) :

Le mode opératoire utilisé est le même que celui décrit pour l'intermédiaire 2.4, la 3-[(4-aminophényl)méthylcarbonyl]thiazolidine remplaçant la 3-(4-aminobenzyl)-thiazolidine. La base libre est obtenue avec un rendement de 64 %. Point de fusion : 163,0-163,5° C.
RMN ¹H (CDCl₃, 400 MHz, δ) : 3,01 (m, 2H, CH₂-S), 3,69 (d, J = 6 Hz, 2H, N-CH₂-S), 3,75-3,88 (2 t, 2H, CH₂-N), 4,55 (d, 2H, CH₂-CO), 4,87 (s, 2H, NH₂), 6,95-6,97-7,22-7,24 (4 s, 4H, H arom.), 7,08 (t, 1H, thiophène), 7,43 (m, 2H, thiophène).
IR: ν_{C=O} (amide) : 1630 cm⁻¹; ν_{C=N} (amidine) : 1577 cm⁻¹.

### Exemple 9 : Fumarate de N-(4-{[2-thiazolidinyl]carbonylaminométhyl}phényl)-2-thiophènecarboximidamide (9) :

### 9.1 Acide 3-(t-butoxycarbonyl)thiazolidine-2-carboxylique :

L'acide thiazolidine-2-carboxylique (2 g, 15 mmoles) est agité en présence de di-t-butyl dicarbonate selon le mode opératoire décrit pour l'intermédiaire 5.1. L'acide 3-(t-butoxycarbonyl)thiazolidine-2-carboxylique est obtenu sous forme d'huile jaune pâle avec un rendement de 97 % (3,4 g) et sera utilisé tel quel dans les étapes suivantes.
RMN ¹H (CDCl₃, 100 MHz, δ) : 1,46 (s, 9H, tBu), 3,10 (m, 3H, CH₂-S, CH-S), 3,85 (m, 2H, CH₂-N).

### 9.2 (4-nitrobenzyl)-3-(t-butoxycarbonyl)thiazolidine-2-carboxamide :

L'acide 3-(t-butoxycarbonyl)thiazolidine-2-carboxylique (1 g, 4,3 mmoles) et le carbonyldiimidazole (0,7 g, 4,3 mmoles) sont dissous dans du THF (10 ml). Le mélange est agité pendant 1 heure à température ambiante. Le chlorhydrate de la 4-nitrobenzylamine (0,81 g, 4,3 mmoles) et la triéthylamine (0,6 ml, 0,43 g, 4,3 mmoles) en suspension dans 10 ml d'un mélange THF et DMF (1/1) sont ajoutés à la solution précédente et l'ensemble est chauffé au reflux pendant 5 heures. Les solvants sont ensuite évaporés sous pression réduite. Le résidu est repris par 25 ml d'acétate d'éthyle et lavé par 3 fois 15 ml d'eau. La phase organique est séchée et le solvant évaporé sous pression réduite. Le produit obtenu est purifié sur gel de silice dans un mélange dichlorométhane / méthanol (95/5). Le N-(4-nitrobenzyl)-3-(t-butoxycarbonyl)thiazolidine-2-carboxamide est obtenu sous forme d'huile jaune pâle avec un rendement de 80 % (1,25 g).
RMN ¹H (CDCl₃, 100 MHz, δ) : 1,45 (s, 9H, tBu), 3,09 (m, 3H, CH₂-S, CH-S), 3,86 (m, 2H, CH₂-CH₂-N), 4,57 (m, 2H, CH₂-NH), 6,60 (s large, 1H, NH), 7,41-7,50-8,14-8,23 (4s, 4H, H arom.).

### 9.3 (4-aminobenzyl)-3-(t-butoxycarbonyl)thiazolidine-2-carboxamide :

On ajoute une pointe de spatule de Nickel de Raney à une solution de 1,25 g (3,4 mmoles) de N-(4-nitrobenzyl)-3-(t-butoxycarbonyl)thiazolidine-2-carboxamide dans 2,5 ml de méthanol. L'ensemble est porté au reflux et l'hydrate d'hydrazine (1,75 ml) est ajouté goutte à goutte dans le milieu. La réaction est maintenue 1 heure au reflux, puis, près retour à température ambiante, le catalyseur est filtré et abondamment rincé par du méthanol. Le solvant est évaporé sous pression réduite, puis le résidu est repris par du dichlorométhane (20 ml) et lavé par 3 fois 15 ml d'eau. La phase organique est séchée et le solvant évaporé sous pression réduite. Le N-(4-aminobenzyl)-3-(t-butoxycarbonyl)thiazolidine-2-carboxamide est obtenu sous forme d'un solide inerte jaune (0,815 g, 71 %) ; il sera utilisé dans les étapes suivantes sans autre purification.
RMN ¹H (CDCl₃, 100 MHz, δ) : 1,43 (s, 9H, tBu), 3,08 (m, 2H, CH₂-S), 3,67 (m, 3H, CH₂-CH₂-N, CH-S), 4,36 (m, 2H, CH₂-NH), 6,05 (s large, 1H, NH), 6,60-6,69-7,04-7,12 (4 s, 4H, H arom.).

### 9.4 [4-{[3-(t-butoxycarbonyl)-2-thiazolidinyl]carbonylaminométhyl}phényl]-2-thiophènecarboximidamide :

Le protocole expérimental utilisé est le même que celui décrit pour l'intermédiaire 2.4, la N-(4-aminobenzyl)-3-(t-butoxycarbonyl)thiazolidine-2-carboxamide remplaçant la 3-(4-aminobenzyl)-thiazolidine. Le composé attendu est obtenu avec un rendement de 77 %.
RMN ¹H (CDCl₃, 100 MHz, δ) : 1,45 (s, 9H, tBu), 3,14 (m, 3H, CH₂-S, CH-S), 3,84 (m, 2H, CH₂-CH₂-N), 4,46 (m, 2H, CH₂-NH), 4,83 (s large, 2H, NH₂), 6,27 (s large, 1H, NH), 7,22 (m, 7H, H arom.).

### 9.5 Fumarate de N-(4-{[2-thiazolidinyl]carbonylaminométhyl}phényl)-2-thiophènecarboximidamide (9) :

Le protocole expérimental utilisé est le même que celui décrit pour l'intermédiaire 5.5, la [4-{[3-(t-butoxycarbonyl)-2-thiazolidinyl]carbonylaminométhyl}phényl]-2-thiophènecarboxi-midamide remplaçant la 3-{2-[4-aminophényl]éthyl}thiazolidine. Le composé attendu est obtenu sous forme de base libre avec un rendement de 34 %. Il est salifié par un équivalent d'acide fumarique dans l'éthanol à reflux. Point de fusion : 167-168° C.
RMN ¹H (DMSO, 400 MHz, δ): 2,78 (t, 2H, CH₂-S), 3,06 (m, 2H, CH₂-CH₂-N), 3,28 (s large, 1H, CH-S), 4,26 (m, 2H, CH₂-NH), 4,86 (s large, 1H, NH), 6,45 (s large, 2H, NH₂), 6,81-6,83-7,19-7,21 (4 s, 4H, H arom.), 7,10 (t, 1H, thiophène), 7,61 (d, 1H, thiophène), 7,74 (m, 1H, thiophène), 8,53 (t, 1H, NH-CO).
IR : ν_{C=O} (amide) : 1624 cm⁻¹; ν_{C=N} (amidine) : 1584 cm⁻¹.

### Exemple 10 : Iodhydrate de N-(3,5-di-t-butyl-4-hydroxyphényl)-5-[4-{imino(2-thiényl)-méthylamino}phényl]-2-furanne carboxamide (10) :

### 10.1 2,6-di-t-butyl-4-nitrophénol :

Le 2,6-di-t-butylphénol (8 g, 39 mmoles) est dissous dans 25 ml de cyclohexane à 10° C. Un mélange (1/1) acide nitrique / acide acétique (5 ml) est ajouté goutte à goutte dans le milieu réactionnel maintenu à cette température. On agite ensuite pendant 15 minutes à température ambiante, puis on filtre le précipité formé, on le rince par de l'eau et du pentane. Le 2,6-di-t-butyl-4-nitrophénol obtenu (6,34 g, 65 %) est séché à l'étuve et sera utilisé sans autre purification dans les étapes suivantes. Poudre jaune pâle. Point de fusion : 167-168° C.
RMN ¹H (CDCl₃, 100 MHz, δ) : 1,48 (s, 18H, 2tBu), 5,93 (s, 1H, OH), 8,13 (s, 2H, H arom.).

### 10.2 2,6-di-t-butyl-4-aminophénol :

Le 2,6-di-t-butyl-4-nitrophénol (6,3 g, 25 mmoles) est dissous dans du méthanol (100 ml). On ajoute 0,6 g de palladium sur charbon (10 %) et on place l'ensemble sous atmosphère d'hydrogène sous 2 bar de pression. Le catalyseur est filtré et le solvant évaporé sous pression réduite. Le résidu est repris dans l'heptane et filtré. On obtient ainsi le 2,6-di-t-butyl-4-aminophénol (2,7 g, 48 %) qui sera utilisé sans autre purification dans les étapes suivantes. Poudre rose. Point de fusion : 123-124° C.
RMN ¹H (CDCl₃, 100 MHz, δ) : 6,60 (s, 2H, Ph) ; 4,65 (s large, 1H, OH) ; 3,15 (s large, 2H, NH₂) ; 1,42 (s, 18H, 2 tBu).

### 10.3 N-(3,5-di-t-butyl-4-hydroxyphényl)-5-(4-nitrophényl)-2-furanne carboxamide :

Le protocole expérimental utilisé est le même que celui décrit pour l'intermédiaire 8.1, le 2,6-di-t-butyl-4-aminophénol et l'acide 5-(4-nitrophényl)-2-furanne carboxylique remplaçant respectivement la thiazolidine et l'acide 4-(t-butoxycarbonylamino)-benzèneacétique. Le composé attendu est obtenu sous forme d'une huile incolore avec un rendement de 56 %.
RMN ¹H (DMSO, 100 MHz, δ): 1,41 (s, 18H, 2tBu), 6,91 (s, 1H, OH), 7,42 (m, 4H, H arom.), 7,54 (s, 2H, H arom.), 8,30 (m, 4H, H arom.), 10,11 (s, 1H, NH).

### 10.4 N-(3,5-di-t-butyl-4-hydroxyphényl)-5-(4-aminophényl)-2-furanne carboxamide :

Le protocole expérimental utilisé est le même que celui décrit pour l'intermédiaire 1.2, le N-(3,5-di-t-butyl-4-hydroxyphényl)-5-(4-nitrophényl)-2-furanne carboxamide remplaçant le 1-(4-nitrophényl)-1H-imidazole. Le composé attendu est obtenu sous forme d'une huile incolore avec un rendement de 59 %.
RMN ¹H (DMSO, 100 MHz, δ) : 1,41 (s, 18H, 2 tBu), 4,70 (s large, 2H, NH₂), 6,91 (s, 1H, OH), 7,50 (m, 4H, H arom.), 7,54 (s, 2H, H arom.), 8,20 (m, 4H, H arom.).

### 10.5 Iodhydrate de N-(3,5-di-t-butyl-4-hydroxyphényl)-5-[4-{imino(2-thiényl)-méthylamino}phényl]-2-furanne carboxamide (10) :

Le protocole expérimental utilisé est le même que celui décrit pour l'intermédiaire 1.3, le N-(3,5-di-t-butyl-4-hydroxyphényl)-5-(4-aminophényl)-2-furanne carboxamide remplaçant le 1-(4-aminophényl)-1H-imidazole. Le produit attendu est obtenu sous forme salifiée avec un rendement de 27 %. Point de fusion : 273-274° C.
RMN ¹H (DMSO, 400 MHz, δ) : 1,40 (s, 18H, 2tBu), 6,90 (s, 1H, OH), 7,45 (m, 5H, H arom,), 7,54 (s, 2H, H arom.), 8,15 (m, 4H, H arom.), 9,05-9,90 (2 s larges, 2H, NH₂), 10,01 (s, 1H, NH-CO), 11,57 (s, 1H, HI).
IR : ν_{OH} : 3423-3242 cm⁻¹; ν_{C=O} (amide) : 1646 cm⁻¹; ν_{C=N} (amidine) : 1554 cm⁻¹.

### Exemple 11 : Chlorhydrate de 3-(3,5-di-t-butyl-4-hydroxyphényl)-1-[4-{imino(2-thiényl)-méthylamino}phényl]-2,5-imidazolidinedione (11) :

### 11.1 (3,5-di-t-butyl-4-hydroxyphényl)aminoacétate d'éthyle :

1 g (4,5 mmoles) de 2,6-di-t-butyl-4-aminophénol (intermédiaire 10.2) et 0,65 g d'acétate de sodium (7,9 mmoles) sont mis en suspension dans 1 ml d'éthanol. On ajoute ensuite le bromoacétate d'éthyle (0,94 g, 5,65 mmoles) dans le milieu et on chauffe l'ensemble à 65° C pendant 2 heures. On verse le mélange réactionnel sur 20 ml d'eau glacée et on extrait le produit de la réaction par du dichlorométhane (3 fois 15 ml). Les phases organiques sont séchées et le solvant évaporé sous pression réduite. Le résidu est passé sur gel de silice dans du dichlorométhane. On obtient une huile incolore constituée d'un mélange de 2 composés : le produit de mono et double substitution. Le mélange de ces 2 composés est engagé sans autre purification dans l'étape suivante.

### 11.2 (3,5-di-t-butyl-4-hydroxyphényl)-(4-nitrophénylcarbamoyl)aminoacétate d'éthyle :

1,13 g (4,2 mmoles) de l'intermédiaire 11.1 et 0,69 g (4,23 mmoles) de 4-nitrophénylisocyanate sont dissous dans 9 ml de dichlorométhane. Le mélange réactionnel est agité pendant 2,5 heures à température ambiante. Le solvant est évaporé sous pression réduite et le résidu passé sur gel de silice dans du dichlorométhane. On isole ainsi 0,66 g de (3,5-di-t-butyl-4-hydroxyphényl)-(4-nitrophénylcarbamoyl)aminoacétate d'éthyle pur sous forme d'huile incolore. (Rendement sur les 2 étapes : 31 %).
RMN ¹H (CDCl₃, 100 MHz, δ) : 1,30 (t, 3H, CH₃), 1,46 (s, 18H, 2tBu), 4,23 (q, 2H, CH₂-CH₃), 4,38 (s, 2H, CH₂-CO), 5,50 (s, 1H, OH), 6,75 (s large, 1H, NH), 7,28 (s, 2H, H arom.), 7,40-7,50-8,10-8,20 (4s, 4H, H arom.).

### 11.3 (3,5-di-t-butyl-4-hydroxyphényl)-1-(4-nitrophényl)-2,5-imidazolidinedione :

On dissout 0,66 g (1,4 mmole) de l'intermédiaire 11.2 dans 10 ml d'éthanol à 50° C et l'ensemble est chauffé à cette température pendant 2 heures. Le précipité formé est filtré et lavé par de l'éthanol froid. Le composé obtenu est investi directement dans l'étape suivante sans purification supplémentaire.
RMN ¹H (CDCl₃, 100 MHz, δ) : 1,47 (s, 18H, 2tBu), 4,51 (s, 2H, N-CH₂-CO), 5,27 (s, 1H, OH), 7,33 (s, 2H, H arom.), 7,77-7,86-8,32-8,41 (4s, 4H, H arom.).

### 11.4 (3,5-di-t-butyl-4-hydroxyphényl)-1-(4-aminophényl)-2,5-imidazolidinedione :

Le protocole expérimental utilisé est le même que celui décrit pour l'intermédiaire 1.2, le 3-(3,5-di-t-butyl-4-hydroxyphényl)-1-(4-nitrophényl)-2,5-imidazolidinedione remplaçant le 1-(4-nitrophényl)-1H-imidazole. Le composé attendu est obtenu sous forme d'un précipité blanc avec un rendement de 87 %. Il est investi sans purification supplémentaire dans l'étape suivante.
RMN ¹H (CDCl₃, 100 MHz) : 1,47 (s, 18H, 2tBu), 4,45 (s, 2H, N-CH₂-CO), 5,18 (s, 1H, OH), 6,70-6,80-7,16-7,23 (4s, 4H, H arom.), 7,39 (s, 2H, H arom.).

### 11.5 Chlorhydrate de 3-(3,5-di-t-butyl-4-hydroxyphényl)-1-[4-{imino(2-thiényl)-méthylamino}phényl]-2,5-imidazolidinedione (11) :

Le protocole expérimental utilisé est le même que celui décrit pour l'intermédiaire 2.4, le 3-(3,5-di-t-butyl-4-hydroxyphényl)-1-(4-aminophényl)-2,5-imidazolidinedione remplaçant la 3-(4-aminobenzyl)-thiazolidine. La base libre est salifiée par traitement avec une solution 1N d'éther chlorhydrique. On obtient le chlorhydrate avec un rendement de 53 %. Point de fusion : 258-265° C.
RMN ¹H (DMSO, 400 MHz, δ) : 1,40 (s, 18H, 2 tBu), 4,65 (s, 2H, CH₂), 7,08 (s, 1H, OH), 7,40 (m, 3H, H arom.), 7,61 (s, 4H, H arom.), 8,21 (m, 2H, H arom.), 9,20-9,95 (2s larges, 2H, NH₂), 11,75 (s, 1H, HCl).
IR : ν_{OH} : 3637-3437 cm⁻¹ ; ν_{C=O} (imidazolidinedione) : 1712 cm⁻¹; ν_{C=O} (amidine) : 1598 cm⁻¹.

### Exemple 12 : Chlorhydrate de 2-(3,5-di-t-butyl-4-hydroxyphényl)-3-[4- {imino(2-thiényl)-méthylamino}phényl]-4-thiazolidinone (12) :

### 12.1 2-(3,5-di-t-butyl-4-hydroxyphényl)-3-(4-nitrophényl)-4-thiazolidinone :

5 g de 3,5-di-t-butyl-4-hydroxybenzaldéhyde (21 mmoles) et 2,95 g de para-nitroaniline (21 mmoles) sont dissous dans 50 ml de toluène anhydre. On ajoute 0,5 ml d'acide acétique glacial et on porte l'ensemble au reflux pendant 24 heures. On ajoute ensuite 1,96 g d'acide mercaptoacétique (21 mmoles) dans le milieu et on poursuit le reflux encore 24 heures. Après retour à température ambiante, on lave le mélange réactionnel par de l'eau (3 fois 30 ml), après décantation, on sèche la phase organique sur sulfate de sodium et on évapore le solvant sous pression réduite. Le résidu est purifié sur gel de silice dans un mélange acétate d'éthyle / heptane (1/4) et on obtient 1,33 g de 2-(3,5-di-t-butyl-4-hydroxyphényl)-3-(4-nitrophényl)-4-thiazolidinone pure sous forme d'huile incolore (15 %).
RMN ¹H (CDCl₃, 100 MHz, δ) : 1,36 (s, 18H, 2tBu), 3,91 (s, 2H, CH-S), 5,28 (s, 1H, CH-S), 6,20 (s, 1H, OH), 7,03 (s, 2H, H arom.), 7,38-7,48-8,11-8,20 (4 s, 4H, H arom.).

### 12.2 2-(3,5-di-t-butyl-4-hydroxyphényl)-3-(4-aminophényl)-4-thiazolidinone :

1,3 g de 2-(3,5-di-t-butyl-4-hydroxyphényl)-3-(4-nitrophényl)-4-thiazolidinone (3 mmoles) et 3,4 g (15 mmoles) de chlorure d'étain dihydraté sont dissous dans 25 ml d'acétate d'éthyle. La réaction est maintenue pendant 2 heures à 70° C. Après retour à température ambiante, le mélange est versé sur une solution saturée d'hydrogénocarbonate de sodium. Le produit attendu est ensuite extrait dans la phase organique puis celle-ci est lavée par 3 fois 10 ml d'eau. La 2-(3,5-di-t-butyl-4-hydroxyphényl)-3-(4-aminophényl)-4-thiazolidinone est purifiée sur gel de silice dans un mélange acétate d'éthyle / heptane (1/1) et obtenue sous forme d'huile beige avec un rendement de 69 % (0,82 g).
RMN ¹H (CDCl₃, 100 MHz, δ): 1,37 (s, 18H, 2tBu), 3,64 (s large, 2H, NH₂), 3,89 (s, 2H, CH₂-S), 5,22 (s, 1H, CH-S), 5,91 (s, 1H, OH), 6,51-6,59-6,78-6,86 (4 s, 4H, H arom.), 7,04 (s, 2H, H arom.).

### 12.3 Chlorhydrate de 2-(3,5-di-t-butyl-4-hydroxyphényl)-3-[4-{imino(2-thiényl)-méthylamino}phényl]-4-thiazolidinone (12) :

Le protocole expérimental utilisé est le même que celui décrit pour l'intermédiaire 2.4, la 2-(3,5-di-t-butyl-4-hydroxyphényl)-3-(4-aminophényl)-4-thiazolidinone remplaçant la 3-(4-aminobenzyl)-thiazolidine. Le composé attendu est obtenue sous forme salifiée (chlorhydrate) par traitement de la base libre par une solution 1N d'éther chlorhydrique avec un rendement de 43 %. Point de fusion : 58-61° C.
RMN ¹H (DMSO, 400 MHz, δ) : 1,32 (s, 18H, 2tBu), 3,93 (m, 2H, CH-S), 6,57 (s, 1H, CH-S), 7,08 (s, 2H, H arom.), 7,41 (m, 5H, H arom.), 8,15 (m, 2H, H arom.), 9,10-9,90 (2s larges, 2H, NH₂), 11,45 (s large, 1H, HCl).
IR : ν_{OH} : 3624-3423 cm⁻¹; ν_{C=O} (thiazolidinone) : 1679-1658 cm⁻¹; ν_{C=N} (amidine) : 1568 cm⁻¹.

### Exemple 13 : Fumarate de 5-[(3,5-di-t-butyl-4-hydroxyphényl)méthylène]-1-méthyl-3-[4-{imino(2-thiényl)méthylamino}phényl]-2,4-imidazolidinedione (13) :

### 13.1 1-Méthyl-3-(4-nitrophényl)-2,4-imidazolidinedione :

0,47 g d'ester éthylique de sarcosine, HCl (3 mmoles) est dissous dans 5 ml de dichlorométhane et additionné de 0,42 ml (3 mmoles) de triéthylamine. 0,5 g de 4-nitrophénylisocyanate (3 mmoles) en solution dans 5 ml de dichlorométhane est ajouté goutte à goutte dans le mélange précédent et l'ensemble est maintenu 30 minutes à température ambiante. La solution organique est ensuite lavée par de l'eau (3 fois 10 ml) puis séchée et le solvant évaporé sous pression réduite.

Le résidu est repris par 10 ml d'éthanol et l'ensemble est chauffé au reflux pendant 2 heures. Après retour à température ambiante, le précipité formé est filtré. La 1-méthyl-3-(4-nitrophényl)-2,4-imidazolidinedione est ainsi obtenue avec un rendement de 72% (0,5 g) et sera utilisée sans autre purification dans l'étape suivante.
RMN ¹H (CDCl₃, 100 MHz, δ) : 3,11 (s, 3H, CH₃), 4,09 (s, 2H, CH₂), 7,70-7,79-8,27-8,37 (4 s, 4H, H arom.).

### 13.2 5-[(3,5-di-t-butyl-4-hydroxyphényl)méthylène]-1-méthyl-3-(4-nitrophényl)-2,4-imidazolidinedione :

L'intermédiaire 13.1 (0,5 g, 2,13 mmoles), le 3,5-di-t-butyl-4-hydroxybenzaldéhyde (0,5 g, 2,13 mmoles) et la β-alanine (0,123 g, 1,4 mmoles) sont dissous dans de l'acide acétique (10 ml). La réaction est maintenue au reflux pendant 24 heures. Après retour à température ambiante, 40 ml d'eau sont ajoutés dans le milieu et l'ensemble est agité pendant 1 heure. Le précipité formé est filtré et lavé par de l'eau. Le filtrat est concentré sous vide et le résidu d'évaporation purifié sur gel de silice (éluant : heptane/acétate d'éthyle : 4/1). Les fractions pures sont collectées et concentrées à sec pour conduire au produit attendu avec un rendement de 32 % (0,3 g).
RMN ¹H (CDCl₃, 100 MHz, δ) : 1,49 (s, 18H, 2tBu), 3,35 (s, 3H, CH₃), 5,59 (s, 1H, OH), 6,40 (s, 1H, CH=C), 7,75-7,84-8,31-8,40 (4 s, 4H, H arom.), 7,92 (s, 2H, H arom.).

### 13.3 5-[(3,5-di-t-butyl-4-hydroxyphényl)méthylène]-1-méthyl-3-(4-aminophényl)-2,4-imidazolidinedione :

Le protocole expérimental utilisé est le même que celui décrit pour l'intermédiaire 12.2, la 5-[(3,5-di-t-butyl-4-hydroxyphényl)méthylène]-1-méthyl-3-(4-nitrophényl)-2,4-imidazolidinedione remplaçant la 2-(3,5-di-t-butyl-4-hydroxyphényl)-3-(4-nitrophényl)-4-thiazolidinone. Le composé attendu est obtenu avec un rendement de 45 %.
RMN ¹H (CDCl₃, 100 MHz, δ) : 1,47 (s, 18H, 2tBu), 3,30 (s, 3H, CH₃), 5,51 (s, 1H, OH), 6,28 (s, 1H, CH=C), 6,69-6,78-7,12-7,21 (4 s, 4H, H arom.), 7,91 (s, 2H, H arom.).

### 13.4 Fumarate de 5-[(3,5-di-t-butyl-4-hydroxyphényl)méthylène]-1-méthyl-3-[4-{imino(2-thiényl)méthylamino}phényl]-2,4-imidazolidinedione (13) :

Le protocole expérimental utilisé est le même que celui décrit pour l'intermédiaire 2.4, la 5-[(3,5-di-t-butyl-4-hydroxyphényl)méthylène]-1-méthyl-3-(4-aminophényl)-2,4-imidazolidinedione remplaçant la 3-(4-aminobenzyl)-thiazolidine. Le composé attendu est obtenu sous forme salifiée (fumarate) par traitement de la base libre par un équivalent d'acide fumarique dans l'éthanol à chaud avec un rendement de 35%. Point de fusion : 54,5-57,5° C.
RMN ¹H (DMSO, 400 MHz, δ) : 1,40 (s, 18H, 2tBu), 3,22 (s, 3H, CH₃), 6,59 (s, 1H, CH=C), 6,61 (s, acide fumarique), 6,97-6,99-7,30-7,32 (4 s, 4H, H arom.), 7,11 (t, 1H, thiophène), 7,64 (d, 1H, thiophène), 7,79 (m, 1H, thiophène), 7,96 (s, 2H, H arom.).
IR : ν_{OH} : 3618-3433 cm⁻¹; ν_{C=O} (imidazolidinedione) : 1711 cm⁻¹; ν_{C=N} (amidine) : 1585 cm⁻¹.

### Exempte 14 : Chlorhydrate de 2-(S)-4-(S)-N-[4-hydroxy-3,5-bis-(1,1-diméthyléthyl)-phényl]-4-{4-[(imino(2-thiényl)méthyl)amino]phénoxy}-prolinamide (14) :

### 14.1 Ester méthylique de la 2-(S)-4-(S)-1-[(1,1-diméthyléthoxy)carbonyl]-4-(4-nitrophénoxy)-proline :

A une suspension, refroidie à 0°C, de 1,23 g (30,7 mmoles) de NaH à 60 % en suspension dans 30 ml de N-méthyl-2-pyrrolidinone anhydre, sous atmosphère inerte, on additionne lentement une solution de 4,37 g (30,7 mmoles) de 4-nitrophénol dans 30 ml de N-méthyl-2-pyrrolidinone anhydre. Après une heure d'agitation à 0° C, on ajoute le dérivé de la proline (6g, 15 mmoles) en une portion. Le mélange réactionnel est agité à 20° C pendant 15 heures suivi de chauffage à 80°C pendant 2 heures pour achever la réaction. Après retour à 20° C, 200 ml d'acétate d'éthyle et 100 ml de soude 1N sont ajoutés au milieu. Aprés décantation, la phase organique est lavée successivement par des solutions diluées de soude 1N jusqu'à extraction complète du dérivé phénolique n'ayant pas réagi, 2 x 100 ml d'eau et 100 ml de saumure. La solution organique est séchée sur sulfate de sodium, filtrée et concentrée à sec, sous pression réduite, pour conduire à une huile jaune claire qui cristallise spontanément à l'air. Les cristaux sont collectés et lavés par 3 x 50 ml d'éther éthylique. Après séchage, on obtient des cristaux incolores avec un rendement de 63 %. Point de fusion : 155-157° C.
RMN ¹H (DMSO, 400 MHz, δ): 1,34-1,40 (2s, 9H, tBu) ; 2,45 (m, 2H, CH₂); 3,60 (m, 2H, CH₂-N); 3,58-3,63 (2s, 3H, O-CH₃); 4,40 (m, 1H, CH-CO₂); 5,22 (m, 1H, HC-O) ; 7,63 (m, 4H, Ph).

### 14.2 2-(S)-4-(S)-1-[(1,1-diméthyléthoxy)carbonyl]-4-(4-nitrophénoxy)-proline :

Dans un ballon de 100 ml contenant 2,87 g (7,84 mmoles) du composé 14.1 dans 40 ml d'éthanol on ajoute 730 mg (environ 16 mmoles) de potasse diluée dans 5 ml d'eau, à 20° C. Après 15 heures d'agitation, le mélange réactionnel est dilué par 100 ml d'acétate d'éthyle, acidifié à 0° C par une solution 12N d'HCl et décanté. La phase organique est lavée par 50 ml d'eau suivie de 50 ml de saumure. Après séchage sur sulfate de sodium, la solution organique est filtrée et concentrée à sec sous vide. On obtient 2,67 g d'une poudre blanche qui est utilisée directement dans l'étape suivante sans purification supplémentaire.
RMN ¹H (CDCl₃, 100 MHz, δ): 1,50 (s, 9H, tBu) ; 2,60 (m, 2H, CH₂); 3,80 (m, 2H, CH₂-N) ; 4,60 (m, 1H, CH-CO₂); 5,07 (m, 1H, HC-O); 7,58 (m, 4H, Ph) ; 8,95 (s large, 1H, CO₂H).

### 14.3 2-(S)-4-(S)-1-[(1,1-diméthyléthoxy)carbonyl]-N-[4-hydroxy-3,5-bis-(1,1-diméthyléthyl)phényl]-4-(4-nitrophénoxy)-prolinamide :

A une solution de 1,99 g (5,64 mmoles) de l'intermédiaire 14.2, de 1,25 g (5,64 mmoles) de l'intermédiaire 10.2 et de 845 mg (6,20 mmoles) d'hydroxybenzotriazole dans 25 ml de DMF, à 0° C, on ajoute 1,28 g (6,20 mmoles) de dicyclohexylcarbodiimide. Après 24 heures d'agitation à 20° C, le mélange réactionnel est filtré et le précipité lavé par de l'acétate d'éthyle. Le filtrat est dilué par 100 ml d'acétate d'éthyle et lavé successivement par 2 x 40 ml de soude 1N, 2 x 40 ml d'eau et 40 ml de saumure. Après séchage sur sulfate de sodium, la solution organique est filtrée et concentrée à sec sous vide pour donner une huile marron qui est purifiée sur colonne de silice (éluant heptane/acétate d'éthyle : 1/1). Les fractions pures sont collectées et après concentration sous vide, on obtient 1,35 g (43 %) d'une poudre beige. Point de fusion : 117-120° C.
RMN ¹H (CDCl₃, 100 MHz, δ) : 1,20-1,70 (m, 27 H, 3 x tBu) ; 2,68 (m, 2H, CH₂) ; 3,80 (m, 2H, CH₂-N) ; 4,58 (m, 1H, CH-CO₂) ; 5,10 (m, 2H, OH, HC-O) ; 7,25-7,28 (2s, 2H, Ph-OH) ; 7,51 (m, 4H, Ph-NO₂) ; 8,00 (s large, 1H, NHCO).

### 14.4 2-(S)-4-(S)-1-[(1,1-diméthyléthoxy)carbonyl]-N-[4-hydroxy-3,5-bis-(1,1-diméthyléthyl)phényl]-4-(4-aminophénoxy)-prolinamide :

Dans un autoclave équipé d'un barreau magnétique, on dissout 1,35 g (2,4 mmoles) de l'intermédiaire 14.3 dans 30 ml d'éthanol en présence d'1/2 spatule de Pd/C à 10 %. Le mélange réactionnel est agité sous 1,5 bar d'hydrogène pendant 3 heures. Après filtration sur célite, le filtrat est concentré sous vide. Le résidu est repris par un mélange 1/1 éther éthylique /heptane et après cristallisation, il est filtré et rincé à l'aide d'heptane. On obtient une poudre beige avec un rendement de 60 %. Point de fusion : 112-113° C.
RMN ¹H (CDCl₃, 100 MHz, δ): 1,20-1,70 (m, 27 H, 3 x tBu) ; 2,55 (m, 2H, CH₂); 3,50 (s large, 2H, NH₂); 3,75 (m, 2H, CH₂-N); 4,48 (m, 1H, CH-CO₂) ; 4,80 (m, 1H, HC-O); 5,10 (s, 1H, OH) ; 6,65 (m, 4H, Ph-NH₂); 7,28 (m, 2H, Ph-OH) ; 8,00 (s large, 1H, NHCO).

### 14.5 Chlorhydrate de 2-(S)-4-(S)-N-[4-hydroxy-3,5-bis-(1,1-diméthyléthyl)phényl]-4-{4-[(imino(2-thiényl)méthyl)amino]phénoxy}-prolinamide (14) :

On chauffe à 50° C, pendant 48 heures, le mélange de 694 mg (1,32 mmole) de l'intermédiaire 14.4 en présence de 376 mg (1,32 mmole) de iodhydrate de S-méthyl-2-thiophènethiocarboximide en solution dans 15 ml d'isopropanol. Le mélange réactionnel est ensuite concentré à sec sous vide et le résidu d'évaporation suspendu dans 50 ml d'acétate d'éthyle. Après addition de 50 ml d'une solution saturée de Na₂CO₃ la phase organique est décantée et successivement lavée par 25 ml d'une solution saturée de Na₂CO₃, 50 ml d'eau et 50 ml de saumure. Après séchage sur sulfate de sodium, la solution organique est filtrée et concentrée à sec sous vide pour donner une poudre jaune qui est purifiée sur colonne de silice (éluant : acétate d'éthyle). Les fractions pures sont collectées et après concentration sous vide, on obtient 686 mg (82 %) d'une poudre beige qui est immédiatement dissoute dans 5 ml d'une solution 4M d'HCl dans du 1,4-dioxanne. Après 15 heures d'agitation à 20° C, on ajoute 20 ml d'éther éthylique sec au mélange réactionnel. Le précipité apparu est alors filtré, rincé par 2 x 25 ml d'éther éthylique sec et séché à l'étuve pour conduire à 270 mg d'une poudre beige. Point de fusion : 233,5-235° C.
RMN ¹H (DMSO, 400 MHz, δ) : 1,37 (s, 18H, 2 x tBu) ; 2,61 (m, 2H, CH₂); 3,60 (m, 2H, CH₂-N); 4,56 (m, 1H, CH-CO₂) ; 5,25 (m, 1H, HC-O) ; 6,92 (s, 1H, OH) ; 7,21 (m, 4H, Ph-N) ; 7,38 (m, 1H, thiophène) ; 7,45 (s, 2H, Ph-OH) ; 8,18 (m, 2H, thiophène) ; 8,78 (s large, 1H, NH⁺); 9,09 (s large, 1H, NH⁺) ; 9,80 (s large, 1H, NH⁺); 10,68 (c, 1H, CONH) ; 11,42 (s large, 1H, NH⁺).
IR: ν_{OH} : 3624-3420 cm⁻¹; ν_{C=O} (amide) : 1653 cm⁻¹; ν_{C=N} (amidine): 1610 cm⁻¹.

### Exemple 15 : Chlorhydrate de 5,6-dihydro-N-{4-[(imino(2-thiényl)méthyl)amino]phényl}-1-(2H)-pyridine carboxamide (15) :

### 15.1 5,6-dihydro-N-(4-nitrophényl)-1-(2H)-pyridine carboxamide :

Sous atmosphère d'argon, dans un tricol de 100 ml, on dissout 900 mg (5 mmoles) de 4-nitrophénylisocyanate dans 17 ml de DMF sec. A cette solution, on ajoute en une seule fois, 0,45 ml (5 mmoles) de 1,2,3,6-tétrahydropyridine et on maintient l'agitation pendant 15 heures. Le mélange réactionnel est alors concentré à sec sous vide et le résidu d'évaporation déposé sur une colonne de gel de silice. Après élution par un mélange heptane/acétate d'éthyle : 4/6, les fractions pures sont collectées et concentrées sous pression réduite pour conduire à 860 mg (70 %) d'une poudre jaune vif. Point de fusion : 169-170° C.
RMN ¹H (DMSO, 100 MHz, δ): 2,29 (m, 2H, =CH-CH₂); 3,69 (m, 2H, CH₂-N); 4,10 (m, 2H, =CH-CH₂-N) ; 5,91 (m, 2H, CH=CH) ; 8,09 (m, 4H, Ph) ; 9,32 (s large, 1H, NHCO).

### 15.2 N-(4-aminophényl)-5,6-dihydro-1-(2H)-pyridine carboxamide :

Le protocole expérimental utilisé est le même que celui décrit pour l'intermédiaire 12.2, la 5,6-dihydro-N-(4-nitrophényl)-1-(2H)-pyridine carboxamide remplaçant la 2-(3,5-di-t-butyl-4-hydroxyphényl)-3-(4-nitrophényl)-4-thiazolidinone. On obtient une huile brune avec un rendement de 36 %.
RMN ¹H (CDCl₃ + D₂O, 400 MHz, δ): 2,20 (m, 2H, =CH-CH₂); 3,59 (m, 2H, CH₂-N); 3,95 (m, 2H, =CH-CH₂-N); 5,84 (m, 2H, CH=CH) ; 6,90 (m, 4H, Ph) ; 9,32 (s large, 1H, NHCO).

### 15.3 Chlorhydrate de 5,6-dihydro-N-{4-[(imino(2-thiényl)méthyl)amino]phényl}-1-(2H)-pyridine carboxamide (15) :

Le protocole expérimental utilisé est le même que celui décrit pour l'intermédiaire 14.5, la N-(4-aminophényl)-5,6-dihydro-1-(2H)-pyridine carboxamide remplaçant la 2-(S)-4-(S)-1-[(1,1-diméthyléthoxy)carbonyl]-N-[4-hydroxy-3,5-bis-(1,1-diméthyléthyl)phényl]-4-(4-aminophénoxy)-prolinamide . On obtient après salification, au moyen d'une solution d'HCl 1M dans l'éther éthylique, une poudre jaune pâle avec un rendement de 55 %. Point de fusion : 230-231° C.
RMN ¹H (DMSO, 400 MHz, δ) : 2,16 (m, 2H, =CH-CH₂); 3,59 (m, 2H, CH₂-N); 3,98 (m, 2H, =CH-CH₂-N); 5,80 (m, 2H, CH=CH) ; 7,52 (m, 4H, Ph) ; 7,38 (s, 1H, thiophène) ; 8,16 (m, 2H, thiophène); 8,78 (s large, 1H, NH⁺); 8,81 (s, 1H, CONH) ; 9,73 (s large, 1H, NH⁺); 11,41 (s large, 1H, NH⁺).
IR : ν_{C=O} (urée) : 1637 cm⁻¹; v_{C=N} (amidine) : 1583 cm⁻¹.

### Exemple 16 : Fumarate de N-[4-hydroxy-3,5-bis-(1,1-diméthyléthyl)phényl]-2-(R,S)-{4-[(imino(2-thiényl)méthyl)amino]phényl}-4-(R)-thiazolidine carboxamide (16) :

### 16.1 Acide 2-(R,S)-(4-nitrophényl)-4-(R)-thiazolidine carboxylique :

On dissout 3 g (17,08 mmoles) du chlorhydrate de L-Cystéine et 2,18 g (22,2 mmoles) d'acétate de sodium dans 75 ml d'eau. La solution est agitée vigoureusement pendant l'addition, en une portion, de 3,10 g (20,5 mmoles) de 4-nitrobenzaldéhyde en solution dans 80 ml d'éthanol à 95 %. Dans cette solution jaune pâle apparaît rapidement un précipité blanc qui se forme en abondance. L'agitation est maintenue pendant une heure, le mélange réactionnel est ensuite refroidi à 0° C et filtré. Le précipité est successivement rincé par 200 ml d'eau, 100 ml d'éthanol froid et 100 ml d'éther éthylique. Après séchage, on obtient une poudre blanche avec un rendement de 87%. Point de fusion : 120-121° C.
RMN ¹H (Acétone D6, 100 MHz, δ) : 3,50 (m, 2H, CH₂-S) ; 4,25 (m, 1H, CH-CO) ; 4,75 (bosse, 2H, CO₂H + NH) ; 5,86 (s, 1H, N-CH-S) ; 8,20 (m, 4H, Ph).

### 16.2 Acide 3-[(1,1-diméthyléthoxy)carbonyl]-2-(R,S)-(4-nitrophényl)-4-(R)-thiazolidine carboxylique :

Le protocole expérimental utilisé est le même que celui décrit pour l'intermédiaire 5.1, l'acide 2-(*R*,*S*)-(4-nitrophényl)-4-(*R*)-thiazolidine carboxylique remplaçant l'acide 4-(t-butoxy-carbonylamino)-benzèneacétique. On obtient une poudre jaune pâle avec un rendement de 59 %. Point de fusion : 145-146° C.
RMN ¹H (CDCl₃, 100 MHz, δ) : 1,35 (m, 9H, tBu) 3,40 (m, 2H, CH₂-S) ; 4,95 (m, 1H, CH-CO) ; 6,10 (m, 1H, N-CH-S); 8,00 (m, 4H, Ph) ; 10,00 (s large, 1H, CO₂H).

### 16.3 3-[(1,1-diméthyléthoxy)carbonyl]-N-[4-hydroxy-3,5-bis-(1,1-diméthyléthyl)phényl]-2-(R,S)-(4-nitrophényl)-4-(R)-thiazolidine carboxamide :

Le protocole expérimental utilisé est le même que celui décrit pour l'intermédiaire 14.3, l'acide 3-[(1,1-diméthyléthoxy)carbonyl]-2-(*R*,*S*)-(4-nitrophényl)-4-(*R*)-thiazolidine carboxylique remplaçant la 2-(*S*)-4-(*S*)-1-[(1,1-diméthyléthoxy)carbonyl]-4-(4-nitrophénoxy)-proline. On obtient une poudre blanche avec un rendement de 41 %. Point de fusion : 226-227° C.
RMN ¹H (CDCl₃, 100 MHz, δ) : 1,45 (m, 27H, 3 x tBu) ; 3,52 (m, 2H, CH₂-S); 5,00 (m, 1H, CH-CO); 5,15 (s, 1H, OH); 6,10 (s large, 1H, N-CH-S) ; 7,30 (s, 2H, Ph-OH) ; 7,92 (m, 4H, Ph-NO₂); 8,60 (s large, 1H, CONH).

### 16.4 3-[(1,1-diméthyléthoxy)carbonyl]-N-[4-hydroxy-3,5-bis-(1,1-diméthyléthyl)phényl]-2-(R,S)-(4-aminophényl)-4-(R)-thiazolidine carboxamide :

Le protocole expérimental utilisé est le même que celui décrit pour l'intermédiaire 12.2, la 3-[(1,1-diméthyléthoxy)carbonyl]-N-[4-hydroxy-3,5-bis-(1,1-diméthyléthyl)phényl]-2-(*R,S*)-(4-nitrophényl)-4-(*R*)-thiazolidine carboxamide remplaçant la 2-(3,5-di-t-butyl-4-hydroxyphényl)-3-(4-nitrophényl)-4-thiazolidinone. Le produit attendu est obtenu sous forme d'une poudre jaune pâle avec un rendement de 21 %. Point de fusion : 196-198° C.
RMN ¹H (CDCl₃, 100 MHz, δ) : 1,40 (m, 27H, 3 x tBu); 3,50 (m, 4H, CH₂-S + NH₂) ; 5,00 (m, 1H, CH-CO) ; 5,10 (s, 1H, OH) ; 6,01 (s large, 1H, N-CH-S) ; 6,98 (m, 4H, Ph-NH₂) ; 7,25 (s, 2H, Ph-OH) ; 8,50 (s large, 1H, CONH).

### 16.5 Fumarate de N-[4-hydroxy-3,5-bis-(1,1-diméthyléthyl)phényl]-2-(R,S)-{4-[(imino(2-thiényl)méthyl)amino]phényl}-4-(R)-thiazolidine carboxamide (16) :

Le protocole expérimental utilisé est le même que celui décrit pour l'intermédiaire 14.5, l'intermédiaire 16.4 remplaçant la 2-(*S*)-4-(*S*)-1-[(1,1-diméthyléthoxy)carbonyl]-N-[4-hydroxy-3,5-bis-(1,1-diméthyléthyl)phényl]-4-(4-aminophénoxy)-prolinamide. Le composé 16.5 obtenu sous forme de base libre est ensuite salifié en présence d'acide fumarique à reflux de l'éthanol pendant 30 minutes. On obtient une poudre jaune avec un rendement global de 30 %. Point de fusion : 201-204° C.
RMN ¹H (DMSO, 400 MHz, δ) : 1,37 (s, 18H, 2 x tBu) ; 3,17 (m, 2H, CH₂-S); 3,29 (s large, 1H, NH thiazolidine) ; 3,91 (m, _H, CH-CO) ; 4,31 (m, _H, CH-CO) ; 5,59 (s, _H, N-CH-S) ; 5,67 (s, _H, N-CH-S) ; 6,61 (s, 2H, fum.) ; 6,74 (m, 2H, NH₂ amidine) ; 7,11 (m, 1H, thiophène) ; 7,19 (m, 4H, Ph-N) ; 7,42 (s, 2H, Ph-OH) ; 7,62 (m, 1H, thiophène) ; 7,73 (s large, 1H, thiophène) ; 9,69 (s, _H, CONH) ; 9,95 (s, _H, CONH).
IR: ν_{OH} : 3625-3421 cm⁻¹; ν_{C=O} (amide) : 1652 cm⁻¹; ν_{C=N} (amidine) : 1604 cm⁻¹.

### Exemple 17 : Iodhydrate de N-[3,5-bis(1,1-diméthyléthyl)-4-hydroxyphényl]-2-{4-[(imino(2-thiényl)méthyl)amino]phényl}-4-thiazolecarboxamide (17) :

### 17.1 4-nitrobenzène-carbothioamide :

A une solution de 4,15 g (25 mmoles) de 4-nitrobenzamide dans 100 ml de dioxanne-1,4, on ajoute 6,06 g (15 mmoles) de réactif de Lawesson. Le mélange réactionnel est chauffé à reflux pendant deux heures. Après retour à température ambiante, la solution est versée dans 150 ml d'eau et extraite par 5 fois 100 ml d'acétate d'éthyle. La solution organique est séchée sur sulfate de magnésium, filtrée et concentrée sous vide pour conduire à une huile jaune qui est purifiée sur une colonne de gel de silice (éluant : heptane/acétate d'éthyle 1/1). Les fractions pures sont collectées et concentrées sous vide, on obtient 3,26 g d'une poudre jaune avec un rendement de 72%. Point de fusion : 165-167°C.

### 17.2 2-(4-nitrophényl)-4-thiazolecarboxylate d'éthyle :

Dans un ballon contenant 100 ml de DMF, on introduit successivement 3,26 g (17,9 mmoles) de l'intermédiaire 17.1 et 2,26 ml (18 mmoles) de bromopyruvate d'éthyle. Après agitation du mélange réactionnel à 23°C, pendant 1 heure, la solution est concentrée sous vide. Le résidu d'évaporation est dissous dans 150 ml de dichlorométhane et lavé successivement par 100 ml d'eau et 100 ml de saumure. Après séchage sur sulfate de magnésium et filtration, la solution organique est concentrée sous vide. La poudre obtenue est alors agitée en présence de 100 ml d'un mélange (3/1) de toluène et d'éthanol, filtrée et rincée par 25 ml du même mélange de solvants. On obtient 3,2 g (60%) d'une poudre beige. Point de fusion : 156-158°C.

### 17.3 Acide 2-(4-nitrophényl)-4-thiazolecarboxylique :

A une solution de l'intermédiaire 17.2 (2,15g, 7,25 mmoles) dans 100 ml d'acétone, à 23°C, on ajoute goutte-à-goutte une solution de 0,82 g (14,5 mmoles) de KOH dans 5 ml d'eau. Après agitation pendant une nuit, le précipité formé est filtré et rincé par 10 ml d'acétone. Ce précipité est repris par un mélange de 100 ml d'acétate d'éthyle et 100 ml d'une solution 1M d'HCl. Après décantation, la phase aqueuse est réextraite par 25 ml d'acétate d'éthyle. Les phases organiques sont collectées et lavées successivement par 25 ml d'eau et 50 ml de saumure. La solution organique est séchée sur sulfate de sodium, filtrée et concentrée sous vide pour conduire à une poudre jaune avec un rendement de 93%. Point de fusion : 250-252°C.

### 17.4 N-[3,5-bis(1,1-diméthyléthyl)-4-hydroxyphényl]-2-(4-nitrophényl)-4-thiazolecarboxamide :

Le protocole expérimental utilisé est le même que celui décrit pour l'intermédiaire 14.3, l'intermédiaire 17.3 remplaçant l'intermédiaire 14.2. Le composé attendu est obtenu sous forme d'une poudre jaune avec un rendement de 51%. Point de fusion : 262-264°C. RMN ¹H (acétone d6, 100 MHz, δ) : 1,60 (s, 18H, 2 tBu), 6,12 (s, 1H, OH), 8,21 (m, 2H, H arom.), 8,50 (s, 4H, H arom.), 8,60 (s, 1H, thiazole), 9,93 (s large, 1H, CO-NH).

### 17.5 N-[3,5-bis(1,1-diméthyléthyl)-4-hydroxyphényl]-2-(4-aminophényl)-4-thiazolecarboxamide :

A une solution de l'intermédiaire 17.4 (1,50g, 3,18 mmoles) dans 50 ml d'un mélange acétate d'éthyle/éthanol/acétone (2/1/2), on introduit 3,59 g (16 mmoles) de SnCl₂, 2H₂O. Le mélange réactionnel est chauffé à reflux pendant 5 heures et finalement après refroidissement, concentré de moitié sous vide. Le résidu d'évaporation est ensuite versé dans 50 ml d'eau froide, le précipité qui se forme est dilué par 100 ml d'acétate d'éthyle et 25 ml d'une solution saturée de NaHCO₃. Le mélange trouble est filtré sur célite et le filtrat est décanté. La phase organique est lavée successivement par 50 ml d'eau et 50 ml de saumure. Après séchage sur sulfate de magnésium et filtration, la solution organique est concentrée sous vide pour conduire à une poudre jaune vif qui est purifiée par lavage à l'aide d'un mélange Et₂O/Heptane (90/10). Le composé attendu est obtenu sous forme d'une poudre jaune pâle avec un rendement de 55%. Point de fusion : 267-268°C. RMN ¹H (CDCl₃, 100 MHz, δ) : 1,49 (s, 18H, 2 tBu), 4,00 (s large, 2H, NH₂), 5,11 (s, 1H, OH), 6,72 (m, 2H, H arom.), 7,60 (s , 2H, H arom.), 7,81 (m, 2H, H arom.), 8,05 (s, 1H, thiazole), 9,10 (s large, 1H, CO-NH).

### 17.6 Iodhydrate de N-[3,5-bis(1,1-diméthyléthyl)-4-hydroxyphényl]-2-{4-[(imino(2-thiényl)méthyl)amino]phényl}-4-thiazolecarboxamide (17) :

Le protocole expérimental utilisé est le même que celui décrit pour l'intermédiaire 1.3, l'intermédiaire 17.5 remplaçant l'intermédiaire 1.2. On obtient une poudre jaune avec un rendement de 27%. Point de fusion : 270-272°C. RMN ¹H (DMSO d6, 400 MHz, δ) : 1,40 (s, 18H, 2 tBu), 6,89 (s, 1H, OH), 7,41 (m, 1H, H arom.), 7,63 (m, 4H, H arom.), 8,11 (m, 1H, H arom.), 8,20 (m, 1H, H arom.), 8,36 (m, 2H, H arom.), 8,48 (s, 1H, H arom.), 9,19 (s large, 1H, NH⁺), 9,90 (s large, 1H, NH⁺), 10,02 (s, 1H, CO-NH), 11,50 (s, 1H, NH⁺). IR: νC=O (amide) : 1660 cm⁻¹ ; νC=N (amidine) : 1646 cm⁻¹.

### Exemple 18 : Dichlorhydrate de N-[3,5-bis(1,1-diméthyléthyl)-4-hydroxyphényl]-4-(S)-{4-[(imino(2-thiényl)méthyl)amino]phénoxy}-pyrrolidine-2-(R)-carboxamide (18) :

### 18.1 4-(S)-(4-nitrophénoxy)-1,2-(R)-pyrrolidinedicarboxylate de 1-(1,1-diméthyléthyle) et 2-méthyle :

Dans un tricol refroidi à 0°C, sous atmosphère inerte, on ajoute goutte-à-goutte 4,38 g (31,5 mmoles) de 4-nitrophénol en solution dans 40 ml de N-méthyl-2-pyrrolidinone anhydre à une suspension de 1,26 g (31,5 mmoles) de NaH à 60% dans 60 ml de N-méthyl-2-pyrrolidinone anhydre. La réaction s'accompagne d'un dégagement important d'hydrogène. Après une heure d'agitation à 0°C, on ajoute en une portion 6 g (15 mmoles) de 4-(R)-{[(4-méthylphényl)sulfonyl]oxy}-1,2-(R)-pyrrolidinedicarboxylate de 1-(1,1-diméthyléthyle) et 2-méthyle, l'agitation est maintenue 15 heures supplémentaires à 23°C et la réaction est achevée par 5 heures de reflux. Après retour à 23°C, le mélange réactionnel est dilué par 150 ml d'acétate d'éthyle et 100 ml d'une solution 1M de soude. Après décantation, la phase aqueuse est réextraite par 2 fois 50 ml d'acétate d'éthyle. Les phases organiques sont collectées et lavées successivement par de la soude 1N (jusqu'à disparition de l'excès de 4-nitrophénol de la phase organique), de l'eau jusqu'à neutralité et finalement 100 ml de saumure. Après séchage sur sulfate de magnésium et filtration, la solution organique est concentrée sous vide pour conduire à un résidu huileux brun qui est purifié sur colonne de silice (éluant : heptane/acétate d'éthyle : 8/2). Les fractions pures sont collectées et concentrées sous vide pour conduire à une huile jaune pâle avec un rendement de 83%. RMN ¹H (CDCl₃, 100 MHz, δ) : 1,41 (s, 9H, tBu), 2,40 (m, 2H, CH₂), 3,80 (s, 5H, CH₃ + CH₂), 4,50 (m, 1H, CH-N), 5,03 (m, 1H, CH-O), 6,95 (m, 2H, H arom.), 8,22 (m, 2H, H arom.).

### 18.2 2-(R)-carboxy-4-(S)-(4-nitrophénoxy)-1-pyrrolidinecarboxylate de 1,1-diméthyléthyle :

A une solution de 7 g (19 mmoles) de l'intermédiaire 18.1 dans 100 ml de méthanol, on ajoute goutte-à-goutte, à 0°C, une solution de 2,14 g (38 mmoles) de KOH dans 15 ml d'eau. Le mélange réactionnel est agité à 23°C pendant 15 heures et finalement concentré de moitié sous vide. Après dilution par 50 ml d'acétate d'éthyle et 50 ml de soude 1N, le mélange est décanté. La phase organique est éliminée et la phase aqueuse est acidifiée à froid par HCl 1M. Le produit est alors extrait par 100 ml d'acétate d'éthyle. La solution organique est ensuite lavée par 50 ml d'eau et 50 ml de saumure. Après séchage sur sulfate de magnésium et filtration, la solution est concentrée sous vide. On obtient une huile jaune pâle avec un rendement de 66%. RMN ¹H (CDCl₃, 100 MHz, δ): 1,45 (s, 9H, tBu), 2,52 (m, 2H, CH₂), 3,80 (m, 2H, CH₂), 4,48 (m, 1H, CH-N), 5,03 (m, 1H, CH-O), 5,92 (s large, CO₂H), 6,92 (m, 2H, H arom.), 8,20 (m, 2H, H arom.).

### 18.3 2-(R)-{[[3,5-bis(1,1-diméthyléthyl)-4-hydroxyphényl]amino]carbonyl}-4-(S)-(4-nitrophénoxy)-pyrrolidine-1-carboxylate de 1,1-diméthyléthyle :

Le protocole expérimental utilisé est le même que celui décrit pour l'intermédiaire 14.3, l'intermédiaire 18.2 remplaçant l'intermédiaire 14.2. On obtient une poudre beige avec un rendement de 43%. Point de fusion : 140-142°C. RMN ¹H (CDCl₃, 100 MHz, δ) : 1,45 (s, 18H, 2 tBu), 1,50 (s, 9H, tBu), 2,30 (m, 1H, 1/2 CH₂), 2,95 (m, 1H, 1/2 CH₂), 3,75 (m, 2H, CH₂), 4,65 (m, 1H, CH-N), 5,10 (m, 2H, CH-O + OH), 6,98 (m, 2H, H arom.), 7,31 (s, 2H, H arom.), 8,22 (m, 2H, H arom.), 9,10 (s large, 1H, CO-NH).

### 18.4 2-(R)-{[[3,5-bis(1,1-diméthyléthyl)-4-hydroxyphényl]amino]carbonyl}-4-(S)-(4-aminophénoxy)-pyrrolidine-1-carboxylate de 1,1-diméthyléthyle :

Le protocole expérimental utilisé est le même que celui décrit pour l'intermédiaire 14.4, l'intermédiaire 18.3 remplaçant l'intermédiaire 14.3. Après purification sur une colonne de silice (éluant : heptane/acétate d'éthyle : 1/1) et concentration des fractions pures, on obtient le composé attendu sous forme d'une poudre beige avec un rendement de 70%. Point de fusion : 104-106°C. RMN ¹H (CDCl₃, 100 MHz, δ) : 1,45 (s, 18H, 2 tBu), 1,50 (s, 9H, tBu), 1,60 (s, 2H, NH₂), 2,10 (m, 1H, 1/2 CH₂), 2,80 (m, 1H, 1/2 CH₂), 3,60 (m, 2H, CH₂), 4,60 (m, 1H, CH-N), 4,85 (m, 1H, CH-O), 5,04 (s, 1H, OH), 6,70 (m, 4H, H arom.), 7,34 (s, 2H, H arom.), 9,10 (s large, 1H, CO-NH).

### 18.5 Dichlorhydrate de N-[3,5-bis(1,1-diméthyléthyl)-4-hydroxyphényl]-4-(S)-{4-[(imino(2-thiényl)méthyl)amino]phénoxy}-pyrrolidine-2-(R)-carboxamide (18) :

Le protocole expérimental utilisé est le même que celui décrit pour l'intermédiaire 2.4, l'intermédiaire 18.4 remplaçant la 3-(4-aminobenzyl)-thiazolidine. La base libre, obtenue sous forme d'une poudre jaune claire, est directement déprotégée en présence de 10 équivalents d'une solution d'HCl anhydre 4M dans le dioxanne-1,4. Après 15 heures d'agitation, le précipité formé est filtré, les cristaux sont lavés à l'aide d'acétone suivi d'éther éthylique. On obtient le produit attendu sous forme d'une poudre jaune pâle avec un rendement de 53%. Point de fusion : 245-247°C. RMN ¹H (DMSO d6, 400 MHz, δ) : 1,36 (s, 18H, 2 tBu), 2,29 (m, 1H, 1/2 CH₂), 2,71 (m, 1H, 1/2 CH₂), 3,42 (m, 1H, 1/2 CH₂), 3,77 (m, 1H, 1/2 CH₂), 4,57 (m, 1H, CH-N), 5,26 (m, 1H, CH-O), 6,93 (s, 1H, OH), 7,17 (m, 2H, H arom.), 7,37 (m, 1H, H arom.), 7,42 (m, 2H, H arom.), 7,48 (s, 2H, H arom.), 8,17 (m, 2H, H arom.), 8,81 (s large, 1H, NH⁺), 9,03(s large, 1H, NH⁺), 9,78 (s large, 1H, NH⁺), 10,70 (s, 1H, CO-NH), 10,84 (s large, 1H, NH⁺), 11,50 (s large, 1H, NH⁺). IR : νC=O (amide) : 1681 cm⁻¹; νC=N (amidine) : 1652 cm⁻¹.

### Exemple 19 : Chlorhydrate de 1-[(3,4-dihydro-6-hydroxy-2,5,7,8-tétraméthyl-2-H-[1]-benzopyran-2-yl)carbonyl]-4-(S)-{4-[(imino(2-thiényl)méthyl)amino]-phénoxy}-pyrrolidine-2-(S)- carboxylate de méthyle (19) :

### 19.1 4-(S)-(4-nitrophénoxy)-1,2-(S)-pyrrolidinedicarboxylate de 1-(1,1-diméthyléthyle) et 2-méthyle

Le protocole expérimental utilisé est le même que celui décrit pour l'intermédiaire 18.1 le dérivé 4-(S)-{[(4-méthylphényl)sulfonyl]oxy}-1,2-(R)-pyrrolidinedicarboxylate de 1-(1,1-diméthyléthyle) et 2-méthyle étant utilisé à la place du dérivé 4-(R)-{[(4-méthylphényl)sulfonyl]oxy}-1,2-(R)-pyrrolidinedicarboxylate de 1-(1,1-diméthyléthyle) et 2-méthyle. Le produit attendu est obtenu sous forme d'une poudre blanche avec un rendement de 63%. Point de fusion : 155-157°C. RMN ¹H (DMSO d6, 400 MHz, δ) : 1,37 (2 s, 9H, tBu), 2,22 (m, 1H, 1/2 CH₂), 2,62 (m, 1H, 1/2 CH₂), 3,45 (m, 1H, 1/2 CH₂), 3,62 (2 s, 3H, OCH₃), 3,78 (m, 1H, 1/2 CH₂), 4,42 (m, 1H, CH-N), 5,20 (m, 1H, CH-O), 7,07 (m, 2H, H arom.), 8,20 (m, 2H, H arom.).

### 19.2 4-(S)-(4-nitrophénoxy)-pyrrolidine-2-(S)-carboxylate de méthyle :

A une solution de 3,45 g (9,4 mmoles) de l'intermédiaire 19.1 dans 15 ml de dichlorométhane, on ajoute, à 0°C, 10 ml (94 mmoles) d'acide trifluoroacétique dilué par 10 ml de dichlorométhane. Le mélange réactionnel est ensuite agité 2 heures à 23°C et finalement concentré sous vide. Le résidu d'évaporation est dilué par 100 ml de dichlorométhane et la solution organique est lavée successivement par 3 fois 20 ml d'une solution saturée de Na₂CO₃, 2 fois 20 ml d'eau et finalement 20 ml de saumure. Après séchage sur sulfate de magnésium et filtration, la solution organique est concentrée sous vide pour conduire à une huile jaune pâle avec un rendement de 78%.

### 19.3 1-[(3,4-dihydro-6-hydroxy-2,5,7,8-tétraméthyl-2-H-[1]-benzopyran-2-yl)carbonyl]-4-(S)-(4-nitrophénoxy)-pyrrolidine-2-(S)-carboxylate de méthyle :

On ajoute 1,3 g (8,06 mmoles) de 1,1'-carbonyldiimidazole à une solution de 1,83 g (7,33 mmoles) de Trolox dans 20 ml de THF sec. Après une heure d'agitation à 23°C, on additionne goutte-à-goutte une solution de 1,95 g (7,33 mmoles) de l'intermédiaire 19.2 dilué dans 10 ml de THF sec. Le mélange réactionnel est agité à 23°C pendant 15 heures et finalement concentré à sec sous vide. Le résidu est dilué par 100 ml d'acétate d'éthyle et la solution organique est lavée par 2 fois 50 ml d'eau et 50 ml de saumure. Après séchage sur sulfate de magnésium et filtration, la solution organique est concentrée sous vide. Le résidu d'évaporation est purifié sur une colonne de gel de silice (éluant : heptane/acétate d'éthyle : 6/4). Les fractions pures sont collectées et évaporées sous vide pour conduire à une poudre jaune avec un rendement de 61%. Point de fusion : 103-105°C. RMN ¹H (CDCl₃ , 400 MHz, δ): 1,55-2,50 (m, 16H, Trolox), 2,63 (m, 2H, CH₂), 3,60-3,71 (2 s, 3H, OCH₃), 3,85 (m, 2H, CH₂), 4,70-4,88 (2 m, 1H, CH-N), 5,02 (m, 1H, CH-O), 6,82 (m, 2H, H arom.), 8,20 (m, 2H, H arom.).

### 19.4 1-[(3,4-dihydro-6-hydroxy-2,5,7,8-tétraméthyl-2-H-[1]-benzopyran-2-yl)carbonyl]-4-(S)-(4-aminophénoxy)-pyrrolidine-2-(S)-carboxylate de méthyle :

Le protocole utilisé est le même que celui décrit pour l'intermédiaire 14.4, l'intermédiaire 19.3 remplaçant l'intermédiaire 14.3. Le produit attendu est obtenu sous forme d'une poudre blanche avec un rendement de 95%. Point de fusion : 110-112°C.

### 19.5 Chlorhydrate de 1-[(3,4-dihydro-6-hydroxy-2,5,7,8-tétraméthyl-2-H-[1]-benzopyran-2-yl)carbonyl]-4-(S)-{4-[(imino(2-thiényl)méthyl)amino]phénoxy}-pyrrolidine-2-(S)- carboxylate de méthyle (19) :

Le protocole utilisé est le même que celui décrit pour l'intermédiaire 2.4, l'intermédiaire 19.4 remplaçant l'intermédiaire 2.3. La réaction de condensation est effectuée dans le 2-propanol seul. Après salification, on obtient le produit attendu sous forme d'une poudre jaune pâle avec un rendement de 75%. Point de fusion : 203-206°C. RMN ¹H (DMSO d6, 400 MHz, δ): 1,55-2,50 (m, 16H, Trolox), 2,45 (m, 2H, CH₂), 3,45-3,60 (2 s, 3H, OCH₃), 3,70 (m, 2H, CH₂), 4,51 (m, 1H, CH-N), 5,02 (m, 1H, CH-O), 7,00 (m, 2H, H arom.), 7,39 (m, 3H, H arom.), 8,16 (m, 2H, H arom.), 8,80 (s large, 1H, NH⁺), 9,75 (s large, 1H, NH⁺), 11,36 (s large, 1H, NH⁺). IR : νC=O (amide) : 1650 cm⁻¹; ν_{C=N} (amidine) : 1611 cm⁻¹.

### Exempte 20 : Chlorhydrate de 1-[(3,4-dihydro-6-hydroxy-2,5,7,8-tétraméthyl-2H-[1]-benzopyran-2-yl)carbonyl]-3-(S)-{4-[(imino(2-thiényl)méthyl)amino]phénoxy}-pyrrolidine (20) :

### 20.1 3-(R)-{[(4-méthylphényl)sulfonyl]oxy}-1-pyrrolidinecarboxylate de 1,1-diméthyléthyle :

On ajoute 21,6 g (114 mmoles) de chlorure de p-toluènesulfonyle à une solution de 10 g (57 mmoles) de (R)-N-Boc-3-pyrrolidinol (préparé classiquement à partir du (R)-3-pyrrolidinol commercial) et de 13,7 ml (171 mmoles) de pyridine dans 150 ml de dichlorométhane. Après 24 heures d'agitation à 23°C, le mélange réactionnel est lavé par 3 fois 50 ml d'une solution 1M d'HCl. Après décantation, la phase organique est lavée par 50 ml d'eau suivi de 50 ml de saumure et finalement séchée sur sulfate de magnésium, filtrée et concentrée sous vide. Le résidu d'évaporation est purifié rapidement sur une colonne de silice (éluant : heptane/acétate d'éthyle : 8/2) pour conduire à une huile jaune pâle avec un rendement de 67%.

### 20.2 3-(S)-(4-nitrophénoxy)-1-pyrrolidine-carboxylate de 1,1-diméthyléthyle :

Le protocole expérimental utilisé est le même que celui décrit pour l'intermédiaire 18.1, l'intermédiaire 20.1 remplaçant le dérivé 4-(R)-{[(4-méthylphényl)sulfonyl]oxy}-1,2-(R)-pyrrolidinedicarboxylate de 1-(1,1-diméthyléthyle) et 2-méthyle. On obtient le produit attendu sous forme d'une poudre jaune claire avec un rendement de 77%. Point de fusion : 112-114°C. RMN ¹H (CDCl₃, 100 MHz, δ): 1,45 (s, 9H, tBu), 2,20 (m, 2H, CH₂), 3,60 (m, 4H, CH₂-CH₂), 5,00 (m, 1H, CH-O), 6,94 (m, 2H, H arom.), 8,20 (m, 2H, H arom.).

### 20.3 3-(S)-(4-nitrophénoxy)pyrrolidine :

Le protocole expérimental utilisé est le même que celui décrit pour l'intermédiaire 19.2, l'intermédiaire 20.2 remplaçant l'intermédiaire 19.1. On obtient une huile brune avec un rendement quantitatif.

### 20.4 1-[(3,4-dihydro-6-hydroxy-2,5,7,8-tétraméthyl-2H-[1]-benzopyran-2-yl)carbonyl]-3-(S)-(4-nitrophénoxy)pyrrolidine :

Le protocole expérimental utilisé est le même que celui décrit pour l'intermédiaire 19.3, l'intermédiaire 20.3 remplaçant l'intermédiaire 19.2. Le produit attendu est obtenu après chromatographie sur une colonne de silice (éluant : heptane/acétate d'éthyle : 7/3), les fractions pures, après évaporation, donnent une poudre beige avec un rendement de 23%. Point de fusion : 176-178°C. RMN ¹H (CDCl₃, 400 MHz, δ): 1,52-2,60 (m, 16H, Trolox), 2,62 (m, 2H, CH₂), 3,50-4,40 (m, 4H, CH₂-CH₂), 4,80 (m, 1H, CH-O), 6,89 (m, 2H, H arom.), 8,20 (m, 2H, H arom.).

### 20.5 1-[(3,4-dihydro-6-hydroxy-2,5,7,8-tétraméthyl-2H-[1]-benzopyran-2-yl)carbonyl]-3-(S)-(4-aminophénoxy)pyrrolidine :

Le protocole expérimental utilisé est le même que celui décrit pour l'intermédiaire 14.4, l'intermédiaire 20.4 remplaçant l'intermédiaire 14.3. On obtient une poudre blanche avec un rendement de 78%. Point de fusion : 98-100°C.

### 20.6 Chlorhydrate de 1-[(3,4-dihydro-6-hydroxy-2,5,7,8-tétraméthyl-2H-[1]-benzopyran-2-yl)carbonyl]-3-(S)-{4-[(imino(2-thiényl)méthyl)amino]phénoxy}pyrrolidine (20) :

Le protocole utilisé est le même que celui décrit pour l'intermédiaire 2.4, l'intermédiaire 20.5 remplaçant l'intermédiaire 2.3. La réaction de condensation est effectuée dans le 2-propanol seul. Après salification, on obtient le produit attendu sous forme d'une poudre jaune pâle avec un rendement de 85%. Point de fusion : 195-197°C. RMN ¹H (pyridine d5, 400 MHz, δ) : 1,52-2,48 (m, 16H, Trolox), 2,60-3,05 (m, 2H, CH₂), 3,58-4,42 (m, 4H, CH₂-CH₂), 4,59-4,90 (m, 1H, CH-O), 6,65 (m, 1H, H arom.), 6,89 (m, 2H, H arom.), 7,01 (m, 1H, H arom.), 7,15 (m, 1H, H arom.), 7,30 (m, 1H, NH⁺), 7,41 (m, 1H, NH⁺), 7,74 (m, 2H, H arom.), 8,95 (m, 1H, NH⁺). IR : νC=O (amide) : 1650 cm⁻¹ ; νC=N (amidine) : 1610 cm⁻¹

### Exemple 21 : 3-{[(3,4-dihydro-6-hydroxy-2,5,7,8-tétraméthyl-2H-[1]-benzopyran-2-yl)-carbonyl]amino}-1-{4-[(imino(2-thiényl)méthyl)amino]phényl}pyrrolidine (21) :

### 21.1 3-{[(1,1-diméthyléthoxy)carbonyl]amino}-1-(4-nitrophényl)pyrrolidine :

Le protocole expérimental utilisé est le même que celui décrit pour l'intermédiaire 1.1, la 3-(tert-butoxycarbonylamino)pyrrolidine remplaçant l'imidazole. RMN ¹H (CDCl₃, 100 MHz, δ): 1,45 (s, 9H, tBu), 2,20 (m, 2H, CH₂), 3,50 (m, 4H, 2 x CH₂-N), 4,35 (m, 1H, CH-N), 4,75 (m, 1H, NH), 6,45 (m, 2H, H arom.), 8,10 (m, 2H, H arom.).

### 21.2 3-amino-1-(4-nitrophényl)pyrrolidine :

Le protocole expérimental utilisé est le même que celui décrit pour l'intermédiaire 19.2, l'intermédiaire 21.1 remplaçant l'intermédiaire 19.1. RMN ¹H (CDCl₃, 100 MHz, δ): 1,50 (s large, 2H, NH₂), 2,10 (m, 2H, CH₂), 3,10 (m, 1H, CH), 3,50 (m, 4H, 2 x CH₂), 6,40 (m, 2H, H arom.), 8,10 (m, 2H, H arom.).

### 21.3 3-{[(3,4-dihydro-6-hydroxy-2,5,7,8-tétraméthyl-2H-[1]-benzopyran-2-yl)carbonyl]amino}-1-(4-nitrophényl)pyrrolidine :

Le protocole expérimental utilisé est le même que celui décrit pour l'intermédiaire 19.3, l'intermédiaire 21.2 remplaçant l'intermédiaire 19.2. On obtient un solide jaune qui est utilisé directement dans l'étape suivante sans purification supplémentaire. RMN ¹H (CDCl₃, 100 MHz, δ) : 1,50-2,20 (m, 18H, Trolox + CH₂), 3,45 (m, 4H, 2 x CH₂), 4,40 (m, 1H, CH), 4,50 (s large, 1H, NH), 8,15 (m, 2H, H arom.), 8,35 (m, 2H, H arom.).

### 21.4 3-{[(3,4-dihydro-6-hydroxy-2,5,7,8-tétraméthyl-2H-[1]-benzopyran-2-yl)carbonyl]amino}-1-(4-aminophényl)pyrrolidine :

Le protocole expérimental utilisé est le même que celui décrit pour l'intermédiaire 14.4, l'intermédiaire 21.3 remplaçant l'intermédiaire 14.3. RMN ¹H (CDCl₃, 100 MHz, δ) : 1,50-2,50 (m, 18H, Trolox + CH₂), 3,15 (m, 4H, 2 x CH₂), 4,50 (m, 2H, CH + NH), 6,40 (m, 4H, H arom.).

### 21.5 3-{[(3,4-dihydro-6-hydroxy-2,5,7,8-tétraméthyl-2H-[1]-benzopyran-2-yl)carbonyl]amino}-1-{4-[(imino(2-thiényl)méthyl)amino]phényl}pyrrolidine (21) :

Le protocole expérimental utilisé est le même que celui décrit pour l'intermédiaire 2.4, l'intermédiaire 21.4 remplaçant l'intermédiaire 2.3. On obtient le produit attendu sous forme d'une poudre jaune (base libre) avec un rendement de 81%. Point de fusion : 135-138°C. RMN ¹H (DMSO d6, 400 MHz, δ) : 1,39-2,50 (m, 18H, Trolox + CH₂), 2,85-3,43 (m, 4H, 2 x CH₂), 4,37 (m, 1H, CH), 6,23 (s large, 2H, NH₂), 6,46 (m, 2H, H arom.), 6,73 (m, 2H, H arom.), 7,07 (m, 1H, H arom.), 7,17 (d, 1/2H, 1/2 CONH, J = 7,6 Hz), 7,34 (d, 1/2H, 1/2 CONH, J = 7,6 Hz), 7,56 (m, 1H, H arom.), 7,68 (m, 1H, H arom.). IR : νC=O (amide) : 1657 cm⁻¹ ; νC=N (amidine) : 1626 cm⁻¹.

### Exemple 22 : Chlorhydrate de 4-[3,5-bis-(1,1-diméthyléthyl)-4-hydroxyphényl]-N-{4-[(imino(2-thiényl)méthyl)amino]benzoyl}-N-méthyl-1H-imidazole-2-méthanamine (22) :

### 22.1 N-méthyl-N-[(phénylméthoxy)carbonyl]glycinate de {[3,5-bis-(1,1-diméthyléthyl)-4-hydroxyphényl]carbonyl}méthyle :

Cet intermédiaire est classiquement obtenu à partir de la Cbz-Sarcosine et de 1-[3,5-bis(1,1-diméthyléthyl)-4-hydroxyphényl]-2-bromo-éthanone en présence de carbonate de césium dans le DMF. RMN ¹H (CDCl₃, 100 MHz, δ) : 1,46 (s, 18H, 2 tBu), 3,00 (s, 3H, N-CH₃), 4,20 (m, 2H, O-CH₂-Ph), 5,10-5,40 (m, 4H, CH₂-N(CH₃) + CO-CH₂-O-CO), 5,80 (s, 1H, OH), 7,30 (m, 5H, H arom.), 7,70 (s, 2H, H arom.).

### 22.2 4-[3,5-bis-(1,1-diméthyléthyl)-4-hydroxyphényl]-N-méthyl-N-[(phénylméthoxy)-carbonyl]-1H-imidazole-2-méthanamine :

Cet intermédiaire est obtenu, à partir de l'intermédiaire 22.1, en utilisant le même protocole expérimental que celui décrit dans *Tetrahedron Lett.,* 1993, **34,** 1901. On obtient une poudre vert pâle avec un rendement de 81 %. Point de fusion : 200-207°C. RMN ¹H (CDCl₃, 400 MHz, δ) : 1,40 (s, 18H, 2 tBu), 3,00 (s, 3H, N-CH₃), 4,50 (m, 2H, O-CH₂-Ph), 5,10 (s, 2H, CH₂-N-COO), 5,20 (s, 1H, OH), 7,00 (s, 1H, Imidazole), 7,20-7,50 (m, 7H, H arom.), 9,90 (s, 1H, NH).

### 22.3 4-[3,5-bis-(1,1-diméthyléthyl)-4-hydroxyphényl]-N-méthyl-N-[(phénylméthoxy)-carbonyl]-1-{[2-(triméthylsilyl)éthoxy]méthyl}-1H-imidazole-2-méthanamine :

A un mélange de 9,96 ml (56,3 mmoles) de chlorure de 2-(Triméthylsilyl)éthoxyméthyle et de 23 g (51,2 ml) de l'intermédiaire 22.2 dans 200 ml de DMF, on ajoute 7,1 g (51,2 mmoles) de carbonate de potassium par portions. A la fin de l'addition, le mélange réactionnel est agité 3 heures à 50°C. Le solvant est ensuite éliminé sous vide et le résidu est dilué par 200 ml d'acétate d'éthyle. La solution organique est lavée par 2 fois 100 ml de saumure, séchée sur sulfate de magnésium, filtrée et concentrée sous vide. Le résidu d'évaporation est purifié sur une colonne de gel de silice (éluant heptane/acétate d'éthyle : 1/1). Les fractions pures sont évaporées pour conduire à une huile verte avec un rendement de 53%. RMN ¹H (CDCl₃, 400 MHz, δ) : 0,0 (s, 9H, Si(CH₃)₃), 0,9 (m, 2H, CH₂-Si), 1,50 (s, 18H, 2 tBu), 3,00 (s, 3H, N-CH₃), 3,30-3,50 (m, 2H, O-CH₂-CH₂-Si), 4,70 (s, 2H, CH₂-N-COO), 5,10 (s, 2H, O-CH₂-Ph), 5,20 (s, 2H, imidazole-CH₂-OSEM), 5,30 (s, 1H, OH), 7,20 (s, 1H, Imidazole), 7,35 (m, 5H, H arom.), 7,60 (s, 2H, H arom.).

### 22.4 4-[3,5-bis-(1,1-diméthyléthyl)-4-hydroxyphényl]-N-méthyl-1-{[2-(triméthylsilyl)éthoxy]-méthyl}-1H-imidazole-2-méthanamine:

Le protocole expérimental utilisé est le même que celui décrit pour l'intermédiaire 14.4, l'intermédiaire 22.3 remplaçant l'intermédiaire 14.3. On obtient une huile marron avec un rendement de 98%. RMN ¹H (CDCl₃, 100 MHz, δ) : 0,0 (s, 9H, Si(CH₃)₃), 0,9 (m, 2H, CH₂-Si), 1,50 (s, 18H, 2 tBu), 2,50 (s, 3H, N-CH₃), 3,50 (m, 2H, O-CH₂-CH₂-Si), 4,00 (s, 2H, N-CH₂-imidazole), 5,20 (s, 1H, OH), 5,40 (s, 2H, imidazole-CH₂-OSEM), 7,10 (s, 1H, Imidazole), 7,50 (s, 2H, H arom.).

### 22.5 4-[3,5-bis-(1,1-diméthyléthyl)-4-hydroxyphényl]-N-méthyl-N-(4-nitrobenzoyl)-1-{[2-(triméthylsilyl)éthoxy]méthyl}-1H-imidazole-2-méthanamine :

A une solution de 5,34 g (11,9 mmoles) de l'intermédiaire 22.4 et de 2 ml (14,4 mmoles) de triéthylamine dans 50 ml de dichlorométhane, on ajoute goutte-à-goutte une solution de 2,67 g (14,4 mmoles) du chlorure de l'acide 4-nitrobenzoïque dans 50 ml de THF sec. Après 2 heures d'agitation à 23°C, le mélange est dilué par 100 ml de dichlorométhane et la solution organique est lavée par 2 fois 100 ml de saumure. Après séchage sur sulfate de magnésium, la phase organique est filtrée et concentrée sous vide pour conduire à une huile jaune qui est utilisée telle quelle dans l'étape suivante. RMN ¹H (CDCl₃, 400 MHz, δ): 0,0 (s, 9H, Si(CH₃)₃), 0,9 (m, 2H, CH₂-Si), 1,50 (s, 18H, 2 tBu), 3,15 (s, 3H, N-CH₃), 3,50 (m, 2H, O-CH₂-CH₂-Si), 4,80 (s, 2H, N-CH₂-imidazole), 5,20 (s, 2H, imidazole-CH₂-OSEM), 5,30 (s, 1H, OH), 6,90 (m, 2H, H arom.), 7,15 (s, 1H, Imidazole), 7,60 (s, 2H, H arom.), 8,10 (m, 2H, H arom.).

### 22.6 4-[3,5-bis-(1,1-diméthyléthyl)-4-hydroxyphényl]-N-méthyl-N-(4-nitrobenzoyl)-1H-imidazole-2-méthanamine :

L'intermédiaire 22.5 (7,42 g, 12,5 mmoles) est dissous dans 62,4 ml (62,4 mmoles) d'une solution 1M de fluorure de tétrabutylammonium en présence de 1,12g (18,7 mmoles) d'éthylènediamine. Le mélange réactionnel est chauffé à reflux pendant 5 heures et finalement versé directement dans 200 ml de saumure et dilué par 200 ml d'acétate d'éthyle. La phase organique est décantée lavée par 100 ml de saumure et finalement séchée sur sulfate de magnésium, filtrée et concentrée sous vide. Le résidu d'évaporation est purifié sur colonne de silice (éluant : dichlorométhane + 5% d'éthanol). On obtient le produit attendu sous forme d'une mousse rouge avec un rendement de 37%. RMN ¹H (CDCl₃, 400 MHz, δ) : 1,50 (s, 18H, 2 tBu), 3,00 (s, 3H, N-CH₃), 4,70 (s, 2H, N-CH₂-imidazole), 5,20 (s, 1H, OH), 7,10 (s, 1H, Imidazole), 7,40-7,60 (m, 4H, H arom.), 8,30 (m, 2H, H arom.), 10,10 (s large, 1H, NH).

### 22.7 4-[3,5-bis-(1,1-diméthyléthyl)-4-hydroxyphényl]-N-méthyl-N-(4-aminobenzoyl)-1H-imidazole-2-méthanamine :

Le protocole expérimental utilisé est le même que celui décrit pour l'intermédiaire 14.4, l'intermédiaire 22.6 remplaçant l'intermédiaire 14.3. On obtient un solide orange avec un rendement de 52%. Point de fusion : 129-131°C. RMN ¹H (CDCl₃, 400 MHz, δ) : 1,50 (s, 18H, 2 tBu), 3,10 (s, 3H, N-CH₃), 3,90 (s, 2H, N-CH₂-imidazole), 4,70 (s, 2H, NH₂), 5,20 (s, 1H, OH), 6,60 (m, 2H, H arom.), 7,10 (s, 1H, Imidazole), 7,30-7,60 (m, 4H, H arom.), 10,30 (s large, 1H, NH).

### 22.8 Chlorhydrate de 4-[3,5-bis-(1,1-diméthyléthyl)-4-hydroxyphényl]- N-{4-[(imino(2-thiényl)méthyl)amino]benzoyl}-N-méthyl-1H-imidazole-2-méthanamine (22) :

Le protocole expérimental utilisé est le même que celui décrit pour l'intermédiaire 4.3, l'intermédiaire 22.7 remplaçant l'intermédiaire 4.2. On obtient un solide beige clair avec un rendement de 54%. Point de fusion : 250-260°C. RMN ¹H (DMSO d6, 400 MHz, δ) : 1,50 (s, 18H, 2 tBu), 3,20 (s, 3H, N-CH₃), 5,00 (s, 2H, N-CH₂-imidazole), 7,30 (s, 1H, OH), 7,35 (m, 1H, thiophène), 7,50 (m, 4H, H arom.), 7,70 (s, 2H, H arom.), 8,00 (s, 1H, Imidazole), 8,20 (m, 2H, thiophène), 9,20 (s, 1H, NH⁺), 10,00 (s, 1H, NH⁺), 11,8 (s, 1H, NH⁺), 14,8 (s, 1H, NH⁺), 15,2 (s, 1H, NH⁺). IR : νC=O (amide) : 1635 cm⁻¹ ; νC=N (amidine) : 1601 cm⁻¹.

### Exemple 23 : Iodhydrate de N-[3,5-bis-(1,1-diméthyléthyl)-4-hydroxyphényl]-1-{4-[(imino(2-thiényl)méthyl)amino]phényl}-1H-pyrrole-2-carboxamide (23) :

### 23.1 1-(4-nitrophényl)-1H-pyrrole-2-carboxylate d'éthyle :

Dans un tricol refroidi à 0°C, sous atmosphère inerte, on ajoute goutte-à-goutte 0,9 g (7,2 mmoles) de l'ester méthylique de l'acide pyrrole-2-carboxylique (classiquement préparé par estérification de l'acide pyrrole-2-carboxylique commercial) dilué par 10 ml de DMF sec à une suspension de 0,3 g (7,4 mmoles) de NaH à 60% dans 15 ml de DMF sec. Après une heure d'agitation à 23°C, on ajoute goutte-à-goutte une solution de 1,01 g (7,2 mmoles) de 4-fluoronitrobenzène dans 10 ml de DMF sec. Le mélange réactionnel est ensuite chauffé 3 heures à 80°C. Après retour à 23°C, l'ensemble est versé dans 100 ml d'un mélange glace + eau et finalement dilué par 200 ml d'acétate d'éthyle. Après décantation, la phase organique est lavée par 3 fois 100 ml d'eau suivi de 100 ml de saumure. La solution organique est séchée sur sulfate de magnésium, filtrée et concentrée sous vide. Le résidu d'évaporation est purifié sur colonne de silice (éluant : heptane/acétate d'éthyle : 9/1), les fractions pures sont collectées et évaporées sous vide pour conduire à une poudre jaune pâle avec un rendement de 49%.

### 23.2 Acide 1-(4-nitrophényl)-1H-pyrrole-2-carboxylique :

Dans un ballon contenant une solution de 0,87 g (3,5 mmoles) de l'intermédiaire 23.1 dans 20 ml de THF refroidi à 0°C, on ajoute une solution de 0,5 g (7,1 mmoles) de KOH dans 5 ml d'eau. Le mélange réactionnel est agité 24 heures à 23°C et finalement dilué par 100 ml d'acétate d'éthyle. Après décantation, la phase organique est éliminée et la phase aqueuse est refroidie à l'aide d'un bain de glace avant acidification par une solution d'HCl concentrée. Le précipité formé est alors filtré et lavé par 2 fois 20 ml d'eau. Après séchage, on obtient le produit attendu avec un rendement de 66%.

### 23.3 N-[3,5-bis-(1,1-diméthyléthyl)-4-hydroxyphényl]-1-(4-nitrophényl)-1H-pyrrole-2-carboxamide :

Le protocole expérimental utilisé est le même que celui décrit pour l'intermédiaire 14.3, l'intermédiaire 23.2 remplaçant l'intermédiaire 14.2. Le composé attendu est obtenu sous forme d'une poudre verdâtre avec un rendement brut de 25%. Le produit est utilisé tel quel dans l'étape suivante.

### 23.4 N-[3,5-bis-(1,1-diméthyléthyl)-4-hydroxyphényl]-1-(4-aminophényl)-1H-pyrrole-2-carboxamide

Le protocole expérimental utilisé est similaire à celui décrit pour l'intermédiaire 14.4, l'intermédiaire 23.3 remplaçant l'intermédiaire 14.3. La réaction s'effectue dans un mélange dichlorométhane/éthanol (1/1). On obtient une poudre blanche avec un rendement de 61%. Point de fusion : 218-219°C.

### 23.5 Iodhydrate de N-[3,5-bis-(1,1-diméthyléthyl)-4-hydroxyphényl]-1-{4-[(imino(2-thiényl)méthyl)amino]phényl}-1H-pyrrole-2-carboxamide (23) :

Le protocole expérimental utilisé est similaire à celui décrit pour l'intermédiaire 1.3, l'intermédiaire 23.4 remplaçant l'intermédiaire 1.2. On obtient une poudre jaune pâle avec un rendement de 73%. Point de fusion : 271-272°C. RMN ¹H (DMSO d6, 400 MHz, δ) : 1,35 (s, 18H, 2 tBu), 6,36 (s, 1H, OH), 6,78 (s, 1H, H arom.), 7,01 (s, 1H, H arom.), 7,16 (s, 1H, H arom.), 7,45 (m, 7H, H arom.), 8,10 (m, 1H, H arom.), 8,19 (m, 1H, H arom.), 9,16 (s large, 1H, NH⁺), 9,89 (s large, 2H, CONH + NH⁺), 11,39 (s large, 1H, NH⁺). IR : νC=O (amide) : 1633 cm⁻¹ ; νC=N (amidine) : 1609 cm⁻¹.

### Exemple 24 : Iodhydrate de 1-[3,5-bis(1,1-diméthyléthyl)-4-hydroxyphényl]-3-{[4-[[imino(2-thiényl)méthyl]amino]phényl]carbonyl}-2-imidazolidinone (24) :

### 24.1 N-[3,5-bis(1,1-diméthyléthyl)-4-hydroxyphényl]-N'-(2-chloroéthyl)urée :

Dans un ballon contenant une solution de 0,5 g (2 mmoles) de l'intermédiaire 10.2 dans 5 ml de DMF, on ajoute 0,17 ml (2 mmoles) de chloroéthylisocyanate. Le mélange réactionnel est agité 2 heures à 23°C et finalement dilué par 100 ml d'acétate d'éthyle et 25 ml d'eau. Après décantation, la solution organique est lavée par 25 ml d'eau, 2 fois 25 ml de saumure et finalement séchée sur sulfate de magnésium. Après filtration et évaporation, le résidu est repris par de l'isopentane pour conduire finalement au produit attendu, sous forme d'un solide rosé, avec un rendement de 83%. Point de fusion : 169-171°C. RMN ¹H (DMSO d6, 400 MHz, δ): 1,30 (s, 18H, 2 tBu), 3,35 (t, 2H, CH₂-NH, J = 6,0 Hz), 3,60 (t, 2H, CH₂-Cl, J = 6,0 Hz), 6,20 (t, 1H, NH-CH₂, J = 5,6 Hz), 6,60 (s, 1H, OH), 7,10 (s, 2H, H arom.), 8,30 (s, 1H, NH-Ph).

### 24.2 1-[3,5-bis(1,1-diméthyléthyl)-4-hydroxyphényl]-2-imidazolidinone:

A une solution de 0,56 g (1,93 mmole) de l'intermédiaire 24.1 dans 10 ml de DMF sec, on ajoute une solution de 0,22 g (1,93 mmole) de tBuO⁻K⁺ dans 2 ml de DMF sec. Après 3 heures d'agitation à 23°C, le mélange réactionnel est dilué par 50 ml d'eau et 100 ml d'acétate d'éthyle. La phase organique est décantée, lavée successivement par 50 ml d'eau et 50 ml de saumure, séchée sur sulfate de magnésium, filtrée et finalement concentrée sous vide. L'huile marron ainsi obtenue est reprise par de l'éther isopropylique pour conduire à une poudre blanche avec un rendement de 51%. Point de fusion : 205-207°C. RMN ¹H (DMSO d6, 100 MHz, δ) : 1,40 (s, 18H, 2 tBu), 4,60 (m, 2H, CH₂), 4,90 (m, 2H, CH₂), 4,90 (s large, 1H, NH), 5,00 (s, 1H, OH), 7,15 (s, 2H, H arom.).

### 24.3 1-[3,5-bis(1,1-diméthyléthyl)-4-hydroxyphényl]-3-[(4-nitrophényl)carbonyl]-2-imidazolidinone:

A une solution de 1,0 g (3,45 mmoles) de l'intermédiaire 24.2 dans un mélange de 20 ml d'acétonitrile et 10 ml de THF, on ajoute 1,28 g (6,9 mmoles) de chlorure de l'acide 4-nitrobenzoïque par portions suivi de 0,71 g (5,15 mmoles) de carbonate de potassium. Après 3 heures d'agitation à 23°C, le mélange réactionnel est dilué par 100 ml de dichlorométhane et 50 ml de saumure. La phase organique, après décantation, est lavée par 50 ml de saumure et séchée sur sulfate de magnésium. Après filtration et concentration sous vide, le résidu d'évaporation est repris par de l'éther isopropylique pour conduire à un solide jaune avec un rendement de 83% après séchage. Point de fusion > 260°C. RMN ¹H (CDCl₃, 400 MHz, δ) : 1,40 (s, 18H, 2 tBu), 3,95-4,20 (m, 4H, 2 CH₂), 5,20 (s, 1H, OH), 7,20 (s, 2H, H arom.), 7,80 (m, 2H, H arom.), 8,25 (m. 2H, H arom.).

### 24.4 1-[3,5-bis(1,1-diméthyléthyl)-4-hydroxyphényl]-3-[(4-aminophényl)carbonyl]-2-imidazolidinone:

Le protocole expérimental utilisé est similaire à celui décrit pour l'intermédiaire 14.4, l'intermédiaire 24.3 remplaçant l'intermédiaire 14.3. On obtient le produit attendu sous forme d'une poudre blanche avec un rendement de 45%. Point de fusion > 260°C. RMN ¹H (CDCl₃, 400 MHz, δ) : 1,40 (s, 18H, 2 tBu), 3,90-4,00 (m, 4H, 2 CH₂), 5,15 (s, 1H, OH), 6,60 (m, 2H, H arom.), 7,13 (s, 2H, H arom.), 7,60 (m, 2H, H arom.).

### 24.5 Iodhydrate de 1-[3,5-bis(1,1-diméthyléthyl)-4-hydroxyphényl]-3-{[4-[[imino(2-thiényl)méthyl]amino]phényl]carbonyl}-2-imidazolidinone (24) :

Le protocole expérimental utilisé est identique à celui décrit pour l'intermédiaire 1.3, l'intermédiaire 24.4 remplaçant l'intermédiaire1.2. On obtient le produit attendu sous forme d'un solide beige clair avec un rendement de 79%. Point de fusion : 220-260°C. RMN ¹H (DMSO d6, 400 MHz, δ) : 1,30 (s, 18H, 2 tBu), 4,00 (m, 4H, 2 CH₂), 6,95 (s, 1H, OH), 7,20 (s, 2H, H arom.), 7,40 (m, 1H thiophène), 7,50 (m, 2H, H arom.), 7,70 (m, 2H, H arom.), 8,20 (m, 2H, thiophène), 9,20 (s large, 1H, NH⁺), 9,90 (s large, 1H, NH⁺), 11,60 (s large, 1H, NH⁺). IR : νC=O (urée) : 1735 cm⁻¹ ; νC=O (amide) : 1649 cm⁻¹ ; νC=N (amidine) : 1595 cm⁻¹.

### Exemple 25 : Iodhydrate de 3-[3,5-bis(1,1-diméthyléthyl)-4-hydroxyphényl]-4,5-dihydro-N-{4-[(imino(2-thiényl)méthyl)amino]phényl}-5-isoxazoleacétamide (25) :

### 25.1 3,5-bis-(1,1-diméthyléthyl)-N,4-dihydroxy-benzène carboxime :

Cet intermédiaire est préparé selon un protocole expérimental décrit dans *J. Med*. *Chem.,* 1997, **40,** 50-60, à partir du 3,5-di-tert-butyl-4-hydroxybenzaldéhyde commercial. On obtient une mousse rouge avec un rendement quantitatif.

### 25.2 Chlorure de 3,5-bis-(1,1-diméthyléthyl)-N,4-dihydroxy-benzène carboximidoyle :

Le protocole expérimental utilisé est le même que celui décrit dans *Tetrahedron Lett.,* 1996, **37** (26), 4455 à partir de l'intermédiaire 25.1. On obtient un solide beige avec un rendement brut de 77%. Le produit est utilisé directement dans l'étape suivante sans purification supplémentaire.

### 25.3 3-[3,5-bis(1,1-diméthyléthyl)-4-hydroxyphényl]-4,5-dihydro-5-isoxazoleacétate de méthyle :

La réaction de l'intermédiaire 25.2 avec l'ester méthylique de l'acide 3-butènoïque est effectuée dans les mêmes conditions que celles décrites dans *Tetrahedron Lett.,* 1996, **37** (26), 4455. On obtient le composé attendu sous forme d'une huile brune avec un rendement de 49%. RMN ¹H (CDCl₃, 400 MHz, δ) : 1,50 (s, 18H, 2 tBu), 2,60 (dd, 1H, 1/2 CH₂-C=N, J = 16,0 Hz et J = 7,8 Hz), 2,90 (dd, 1H, 1/2 CH₂-C=N, J = 16,0 Hz et J = 5,8 Hz), 3,10 (dd, 1H, 1/2 CH₂-C=O, J = 16,6 Hz et J = 6,9 Hz), 3,60 (dd, 1H, 1/2 CH₂-C=O, J = 16,6 Hz et J = 10,2 Hz), 5,10 (m, 1H, CH), 5,50 (s, 1H, OH), 7,50 (s, 2H, H arom.).

### 25.4 Acide 3-[3,5-bis(1,1-diméthyléthyl)-4-hydroxyphényl]-4,5-dihydro-5-isoxazoleacétique :

Cet intermédiaire est obtenu par saponification de l'intermédiaire 25.3 selon un protocole expérimental décrit dans *J. Med. Chem*., 1997, **40**, 50-60. On obtient un solide blanc avec un rendement de 74%. Point de fusion : 229-231°C. RMN ¹H (CDCl₃, 400 MHz, δ): 1,50 (s, 18H, 2 tBu), 2,70 (dd, 1H, 1/2 CH₂-C=N, J = 16,3 Hz et J = 7,5 Hz), 2,90 (dd, 1H, 1/2 CH₂-C=N, J = 16,3 Hz et J = 6,0 Hz), 3,10 (dd, 1H, 1/2 CH₂-C=O, J = 16,6 Hz et J = 6,9 Hz), 3,50 (dd, 1H, 1/2 CH₂-C=O, J = 16,6 Hz et J = 10,2 Hz), 5,05 (m, 1H, CH), 5,50 (s, 1H, OH), 7,45 (s, 2H, H arom.).

### 25.5 3-[3,5-bis(1,1-diméthyléthyl)-4-hydroxyphényl]-4,5-dihydro-N-(4-nitrophényl)-5-isoxazoleacétamide :

Le protocole expérimental utilisé est le même que celui décrit dans *Org. Prep. Proced. Int*., (1975), **7**, 215 à partir de l'intermédiaire 25.4 et de la 4-nitroaniline. On obtient un solide blanc avec un rendement de 45%. Point de fusion : 149-151°C. RMN ¹H (CDCl₃, 400 MHz, δ) : 1,50 (s, 18H, 2 tBu), 2,70 (m, 1H, 1/2 CH₂-C=N), 2,85 (dd, 1H, 1/2 CH₂-C=N, J = 15,1 Hz et J = 7,5 Hz), 3,20 (dd, 1H, 1/2 CH₂-C=O, J = 16,7 Hz et J = 7,0 Hz), 3,70 (dd, 1H, 1/2 CH₂-C=O, J = 16,7 Hz et J = 10,1 Hz), 5,05 (m, 1H, CH), 5,50 (s, 1H, OH), 7,45 (s, 2H, H arom.), 7,70 (m, 2H, H arom.), 8,20 (m, 2H, H arom.), 8,50 (s, 1H, NH-CO).

### 25.6 3-[3,5-bis(1,1-diméthyléthyl)-4-hydroxyphényl]-4,5-dihydro-N-(4-aminophényl)-5-isoxazoleacétamide:

Le protocole expérimental utilisé est le même que celui décrit pour l'intermédiaire 17.5, l'intermédiaire 25.5 remplaçant l'intermédiaire 17.4. On obtient une huile incolore avec un rendement de 80%. RMN ¹H (CDCl₃, 400 MHz, δ) : 1,40 (s, 18H, 2 tBu), 2,60 (dd, 1H, 1/2 CH₂-C=N, J = 15,0 Hz et J = 5,7 Hz), 2,80 (dd, 1H, 1/2 CH₂-C=N, J = 15,0 Hz et J = 6,7 Hz), 3,15 (dd, 1H, 1/2 CH₂-C=O, J = 16,7 Hz et J = 7,2 Hz), 3,50 (dd, 1H, 1/2 CH₂-C=O, J = 16,7 Hz et J = 10,1 Hz), 3,70 (2H, NH₂), 5,10 (m, 1H, CH), 5,60 (s, 1H, OH), 6,60 (m, 2H, H arom.), 7,20 (m, 2H, H arom.), 7,50 (s, 2H, H arom.), 8,10 (s, 1H, NH-CO).

### 25.7 Iodhydrate de 3-[3,5-bis(1,1-diméthyléthyl)-4-hydroxyphényl]-4,5-dihydro-N-{4-[(imino(2-thiényl)méthyl)amino]phényl}-5-isoxazoleacétamide (25) :

Le protocole expérimental utilisé est identique à celui décrit pour l'intermédiaire 1.3, l'intermédiaire 25.6 remplaçant l'intermédiaire 1.2. On obtient le produit attendu sous forme d'une poudre jaune pâle avec un rendement de 72%. Point de fusion > 260°C. RMN ¹H (DMSO d6, 400 MHz, δ): 1,40 (s, 18H, 2 tBu), 2,70 (m, 2H, CH₂-C=N), 3,20 (dd, 1H, 1/2 CH₂-C=O, J = 16,8 Hz et J = 6,8 Hz), 3,60 (dd, 1H, 1/2 CH₂-C=O, J = 16,8 Hz et J = 10,2 Hz), 5,00 (m, 1H, CH), 7,35 (m, 6H, H arom. + OH), 7,80 (m, 2H, H arom.), 8,20 (m, 2H, thiophène), 8,70 (s large, 1H, NH⁺), 9,70 (s large, 1H, NH⁺), 10,30 (s, 1H, NH-CO), 11,20 (s large, 1H, NH⁺). IR: νC=O (amide) : 1650 cm⁻¹; νC=N (amidine) : 1603 cm⁻¹.

### Exemple 26 : Chlorhydrate de 4-[3,5-bis(1,1-diméthyléthyl)-4-hydroxyphényl]-N-{4-[(imino(2-thiényl)méthyl)amino]phényl}-N-méthyl-2-thiazoleméthanamine (26) :

### 26.1 2-{[(1,1-diméthyléthoxy)carbonyl]méthyl}amino-éthanethioamide :

Le protocole expérimental utilisé est identique à celui décrit pour l'intermédiaire 17.1, la N-Boc sarcosinamide (obtenue classiquement à partir de la sarcosinamide commerciale et de BocOBoc) est utilisée comme produit de départ à la place de la 4-nitrobenzamide. On obtient une pâte blanche qui est utilisée directement dans l'étape suivante.

### 26.2 4-[3,5-bis(1,1-diméthyléthyl)-4-hydroxyphényl]-N-[(1,1-diméthyléthoxy)carbonyl]-N-méthyl-2-thiazoleméthanamine :

Le protocole expérimental utilisé est le même que celui décrit dans *J. Org. Chem*., (1995), **60**, 5638-5642, à partir de l'intermédiaire 26.1 et de la 1-[3,5-bis(1,1-diméthyléthyl)-4-hydroxyphényl]-2-bromo-éthanone. On obtient une huile marron. RMN ¹H (CDCl₃, 400 MHz, δ) : 1,50 (m, 27H, 3 tBu), 3,00 (s, 3H, N-CH₃), 4,70 (s, 2H, CH₂), 5,30 (s, 1H, OH), 7,25 (s, 1H, thiazole), 7,70 (s, 2H, H arom.).

### 26.3 4-[3,5-bis(1,1-diméthyléthyl)-4-hydroxyphényl]-N-méthyl-2-thiazoleméthanamine:

A une solution de 2,5 g (5,8 mmoles) de l'intermédiaire 26.2 et de 2 ml (1,6 mmole) de triéthylsilane dans 50 ml de dichlorométhane, on ajoute goutte-à-goutte, à 0°C, 2,3 ml (29 mmoles) de TFA. Après une heure d'agitation, le mélange réactionnel est concentré sous vide et le résidu est dilué dans 100 ml d'acétate d'éthyle et 50 ml d'une solution saturée de NaHCO₃. Après agitation et décantation, la phase organique est séchée sur sulfate de magnésium, filtrée et concentrée sous vide. Le résidu est repris par de l'heptane pour donner un solide blanc, après séchage, avec un rendement de 73%. Point de fusion : 136°C. RMN ¹H (CDCl₃, 400 MHz, δ) : 1,50 (s, 18H, 2 tBu), 2,60 (s, 3H, N-CH₃), 4,20 (s, 2H, CH₂), 5,30 (s, 1H, OH), 7,20 (s, 1H, thiazole), 7,70 (s, 2H, H arom.).

### 26.4 4-[3,5-bis(1,1-diméthyléthyl)-4-hydroxyphényl]-N-méthyl-N-(4-nitrophényl)-2-thiazoleméthanamine :

Le protocole expérimental utilisé est le même que celui décrit pour l'intermédiaire 1.1, l'intermédiaire 26.3 remplaçant l'imidazole. On obtient un solide jaune avec un rendement de 23%. Point de fusion : 199-201°C. RMN ¹H (DMSO d6, 400 MHz, δ) : 1,40 (s, 18H, 2 tBu), 3,25 (s, 3H, N-CH₃), 5,10 (s, 2H, CH₂), 6,95 (m, 2H, H arom.), 7,10 (s, 1H, OH), 7,60 (s, 2H, H arom.), 7,80 (s, 1H, thiazole), 8,05 (m, 2H, H arom.).

### 26.5 4-[3,5-bis(1,1-diméthyléthyl)-4-hydroxyphényl]-N-méthyl-N-(4-aminophényl)-2-thiazoleméthanamine:

Le protocole expérimental utilisé est le même que celui décrit pour l'intermédiaire 17.5, l'intermédiaire 26.4 remplaçant l'intermédiaire 17.4. On obtient le produit attendu sous forme d'une mousse beige avec un rendement de 71%. RMN ¹H (DMSO d6, 400 MHz, δ) : 1,40 (s, 18H, 2 tBu), 2,90 (s, 3H, N-CH₃), 4,50 (s large, 2H, NH₂), 4,60 (s, 2H, CH₂), 6,50 (m, 2H, H arom.), 6,60 (m, 2H, H arom.), 7,10 (s, 1H, OH), 7,60 (s, 2H, H arom.), 7,70 (s, 1H, thiazole).

### 26.6 Chlorhydrate de 4-[3,5-bis(1,1-diméthyléthyl)-4-hydroxyphényl]-N-{4-[(imino(2-thiényl)méthyl)amino]phényl}-N-méthyl-2-thiazoleméthanamine (26) :

Le protocole expérimental utilisé est le même que celui décrit pour l'intermédiaire 4.3, l'intermédiaire 26.5 remplaçant l'intermédiaire 4.2. On obtient une poudre blanche avec un rendement de 67%. Point de fusion : 157-160°C. RMN ¹H (DMSO d6, 400 MHz, δ) : 1,50 (s, 18H, 2 tBu), 3,15 (s, 3H, N-CH₃), 5,00 (s, 2H, CH₂), 6,95 (m, 2H, H arom.), 7,15 (s, 1H, OH), 7,20 (m, 2H, H arom.), 7,40 (m, 1H, thiophène), 7,65 (s, 2H, H arom.), 7,75 (s, 1H, thiazole), 8,15 (m, 2H, thiophène), 8,70 (s large, 1H, NH⁺), 9,70 (s large, 1H, NH⁺), 11,30 (s large, 1H, NH⁺). IR : νC=O (amide) : 1648 cm⁻¹ ; νC=N (amidine) : 1611 cm⁻¹.

### Exemple 27 : Chlorhydrate de 4-[3,5-bis(1,1-diméthyléthyl)-4-hydroxyphényl]-N-{4-[(imino(2-thiényl)méthyl)amino]phényl}-N-méthyl-1H-imidazole-2-méthanamine (27) :

### 27.1 4-[3,5-bis(1,1-diméthyléthyl)-4-hydroxyphényl]-N-méthyl-N-(4-nitrophényl)-1-{[2-(triméthylsilyl)éthoxy]méthyl}-1H-imidazole-2-méthanamine :

Le protocole expérimental utilisé est le même que celui décrit pour l'intermédiaire 1.1, l'intermédiaire 22.4 remplaçant l'imidazole. On obtient un solide jaune avec un rendement de 53%. Point de fusion : 149-151°C. RMN ¹H (CDCl₃, 400 MHz, δ): 0,0 (s, 9H, Si(CH₃)₃), 0,9 (t, 2H, CH₂-Si, J = 8,4 Hz), 1,50 (s, 18H, 2 tBu), 3,15 (s, 3H, N-CH₃), 3,50 (t, 2H, O-CH₂-CH₂-Si, J = 8,4 Hz), 4,80 (s, 2H, N-CH₂-imidazole), 5,20 (s, 2H, imidazole-CH₂-OSEM), 5,25 (s, 1H, OH), 6,90 (m, 2H, H arom.), 7,10 (s, 1H, Imidazole), 7,60 (s, 2H, H arom.), 8,15 (m, 2H, H arom.).

### 27.2 4-[3,5-bis(1,1-diméthyléthyl)-4-hydroxyphényl]-N-méthyl-N-(4-nitrophényl)-1H-imidazole-2-méthanamine :

Le protocole expérimental utilisé est le même que celui décrit pour l'intermédiaire 22.6, l'intermédiaire 27.1 remplaçant l'intermédiaire 22.5. On obtient un solide jaune avec un rendement de 44%. Point de fusion : 209-211°C. RMN ¹H (CDCl₃, 400 MHz, δ) : 1,40 (s, 18H, 2 tBu), 3,20 (s, 3H, N-CH₃), 4,70 (s, 2H, CH₂), 6,80-7,10 (m, 3H, H arom.), 7,20-7,60 (m, 3H, H arom. + OH), 8,10 (m, 2H, H arom.), 12,00 (s, 1H, NH).

### 27.3 4-[3,5-bis(1,1-diméthyléthyl)-4-hydroxyphényl]-N-méthyl-N-(4-aminophényl)-1H-imidazole-2-méthanamine:

Le protocole expérimental utilisé est le même que celui décrit pour l'intermédiaire 14.4, l'intermédiaire 27.2 remplaçant l'intermédiaire 14.3. On obtient une mousse beige avec un rendement de 67%. RMN ¹H (CDCl₃, 400 MHz, δ) : 1,40 (s, 18H, 2 tBu), 2,80 (s, 3H, N-CH₃), 4,20 (s, 2H, CH₂), 4,30-4,70 (m, 3H, NH₂ + NH imidazole), 5,00 (s, 1H, OH), 6,50 (m, 2H, H arom.), 6,70 (m, 2H, H arom.), 6,80 (s, 1H, imidazole), 7,40 (s, 2H, H arom.).

### 27.4 Chlorhydrate de 4-[3,5-bis(1,1-diméthyléthyl)-4-hydroxyphényl]-N-{4-[(imino(2-thiényl)méthyl)amino]phényl}-N-méthyl-1H-imidazole-2-méthanamine (27) :

Le protocole expérimental utilisé est le même que celui décrit pour l'intermédiaire 4.3, l'intermédiaire 27.3 remplaçant l'intermédiaire 4.2. On obtient une poudre jaune avec un rendement de 86%. Point de fusion : 195-200°C. RMN ¹H (DMSO d6, 400 MHz, δ) : 1,50 (s, 18H, 2 tBu), 3,20 (s, 3H, N-CH₃), 5,00 (s, 2H, CH₂), 7,00 (m, 2H, H arom.), 7,20 (m, 2H, H arom.), 7,40 (m, 2H, thiophène + OH), 7,60 (s, 2H, H arom.), 7,90 (s, 1H, imidazole), 8,20 (m, 2H, thiophène), 8,70 (s large, 1H, NH⁺), 9,70 (s large, 1H, NH⁺), 11,40 (s large, 1H, NH⁺), 14,60 (s large, 1H, NH⁺), 15,60 (s large, 1H, NH⁺). IR : νC=O (amide) : 1646 cm⁻¹ ; νC=N (amidine) : 1612 cm⁻¹.

### Exemple 28 : 3-[3,5-bis(1,1-diméthyléthyl)-4-hydroxyphényl]-4,5-dihydro-5-{2-{4-[(imino(2-thiényl)méthyl)amino]phénoxy}éthyl}isoxazole (28) :

### 28.1 3-[3,5-bis(1,1-diméthyléthyl)-4-hydroxyphényl]-4,5-dihydro-5-isoxazoleéthanol :

A une solution de 0,69 g (2,1 mmoles) de l'intermédiaire 25.3 dans 15 ml de THF sec, refroidie à 0°C, on ajoute, par petites portions, 0,09 g (2,4 mmoles) de LiAlH₄. Après une heure d'agitation à 23°C, le mélange réactionnel est refroidi à l'aide d'un bain de glace et l'excès d'hydrure est détruit par addition d'eau (5 ml). Le produit est extrait à l'aide de 2 fois 25 ml d'éther éthylique. La phase organique est lavée par 2 fois 10 ml de saumure, séchée sur sulfate de magnésium, filtrée et concentrée sous vide. Le résidu est purifié sur silice (éluant : heptane/acétate d'éthyle : 1/1). On obtient une mousse blanche avec un rendement de 58%. RMN ¹H (DMSO d6, 100 MHz, δ): 1,40 (s, 18H, 2 tBu), 1,60-1,80 (m, 2H, CH₂-CH₂-O), 3,05 (m, 1H, 1/2 CH₂ isoxazoline), 3,40 (m, 1H, 1/2 CH₂ isoxazoline), 3,50 (m, 2H, CH₂-CH₂-O), 4,60 (s, 1H, OH), 4,70 (m, 1H, CH isoxazoline), 7,40 (s large, 3H, H arom. + OH).

### 28.2 3-[3,5-bis(1,1-diméthyléthyl)-4-hydroxyphényl]-4,5-dihydro-5-[2-(4-nitrophénoxy)éthyl]isoxazole :

Un mélange composé de 0,37 g (1,58 mmole) de l'intermédiaire 28.1, 0,5 ml d'Aliquat 336, 0,18 g (1,27 mmole) de 4-fluoronitrobenzène et 0,071 g (1,27 mmole) de KOH dans 2 ml de toluène est chauffé à 80°C pendant 2 heures. Après retour à 23°C, le mélange réactionnel est partagé entre 50 ml de dichlorométhane et 20 ml d'eau. Après décantation, la phase organique est lavée par 20 ml d'eau suivi de 20 ml de saumure. La solution organique est ensuite séchée sur sulfate de magnésium, filtrée et concentrée sous vide. Le résidu d'évaporation est purifié sur colonne de silice (éluant : heptane/acétate d'éthyle : gradient 10/0 jusqu'à 0/10). On obtient une poudre blanche avec un rendement de 60%. Point de fusion : 151-153°C. RMN ¹H (CDCl₃ , 400 MHz, δ) : 1.50 (s, 18H, 2 tBu), 2,15 (m, 2H, CH₂-CH₂-O), 3,10 (dd, 1H, 1/2 CH₂ isoxazoline, J = 16,3 Hz et J = 6,65 Hz), 3,50 (dd, 1H, 1/2 CH₂ isoxazoline, J = 16,3 Hz et J = 10,4 Hz), 4,10-4,30 (m, 2H, CH₂-CH₂-O), 5,00 (m, 1H, CH isoxazoline), 5,50 (s, 1H, OH), 6,90 (m, 2H, H arom.), 7,50 (s, 2H, H arom.), 8,20 (m, 2H, H arom.).

### 28.3 3-[3,5-bis(1,1-diméthyléthyl)-4-hydroxyphényl]-4,5-dihydro-5-[2-(4-aminophénoxy)éthyl]isoxazole:

Le protocole expérimental utilisé est le même que celui décrit pour l'intermédiaire 17.5, l'intermédiaire 28.2 remplaçant l'intermédiaire 17.4. On obtient une poudre blanche avec un rendement de 60%. Point de fusion : 129-131°C. RMN ¹H (DMSO d6 , 400 MHz, δ) : 1,35 (s, 18H, 2 tBu), 2,00 (m, 2H, CH₂-CH₂-O), 3,15 (dd, 1H, 1/2 CH₂ isoxazoline, J = 16,7 Hz et J = 7,5 Hz), 3,40 (dd, 1H, 1/2 CH₂ isoxazoline, J = 16,7 Hz et J = 10,5 Hz), 3,90 (m, 2H, CH₂-CH₂-O), 4,60 (s, 2H, NH₂), 4,70 (m, 1H, CH isoxazoline), 6,50 (m, 2H, H arom.), 6,70 (m, 2H, H arom.), 7,40 (s, 3H, H arom. + OH).

### 28.4 3-[3,5-bis(1,1-diméthyléthyl)-4-hydroxyphényl]-4,5-dihydro-5-{2-{4-[(imino(2-thiényl)méthyl)amino]phénoxy}éthyl}isoxazole (28) :

Le protocole expérimental utilisé est le même que celui décrit pour l'intermédiaire 4.3, l'intermédiaire 28.3 remplaçant l'intermédiaire 4.2. On obtient un solide blanc avec un rendement de 32%. Point de fusion : 240-245°C. RMN ¹H (DMSO d6 , 400 MHz, δ) : 1,40 (s, 18H, 2 tBu), 2,15 (m, 2H, CH₂-CH₂-O), 3,20 (dd, 1H, 1/2 CH₂ isoxazoline, J = 16,65 Hz et J = 7,35 Hz), 3,50 (dd, 1H, 1/2 CH₂ isoxazoline, J = 16,65 Hz et J = 10,3 Hz), 4,20 (s large, 2H, CH₂-CH₂-O), 4,90 (m, 1H, CH isoxazoline), 7,20 (m, 2H, H arom.), 7,40 (m, 6H, H arom. + OH), 8,20 (m, 2H, thiophène), 8,80 (s large, 1H, NH⁺), 9,80 (s large, 1H, NH⁺), 11,40 (s large, 1H, NH⁺). IR : νC=O (amide) : 1655 cm⁻¹ ; νC=N (amidine) : 1618 cm⁻¹.

### Exemple 29 : Chlorhydrate de 1-{[3,5-bis(1,1-diméthyléthyl)-4-hydroxyphényl]amino}-carbonyl}-3-{4-[(imino(2-thiényl)méthyl)amino]phénoxy}azétidine (29) :

### 29.1 1-(diphénylméthyl)-3-(4-nitrophénoxy)azétidine :

A une suspension de 0,06 g (2,3 mmoles) de NaH dans 20 ml de THF sec, sous atmosphère d'argon, on ajoute 0,5 g (2 mmoles) de 1-(diphénylméthyl)-3-hydroxyazétidine. Après une heure d'agitation à 23°C, on ajoute goutte-à-goutte au mélange réactionnel une solution de 0,29 g (2,1 mmoles) de 4-fluoronitrobenzène dans 5 ml de THF sec. L'agitation est maintenue pendant 2 heures supplémentaires à 23°C et l'ensemble est finalement versé dans 25 ml d'eau. Le produit est extrait par 2 fois 25 ml d'acétate d'éthyle, la phase organique est ensuite lavée par 2 fois 25 ml de saumure, séchée sur sulfate de magnésium, filtrée et concentrée sous vide. Le produit est purifié sur une colonne de silice (éluant : 12% d'acétate d'éthyle dans l'heptane). Les fractions pures sont évaporées pour conduire à une huile incolore avec un rendement de 40%. RMN ¹H (CDCl₃, 400 MHz, δ) : 3,20 (m, 2H, azétidine), 4,50 (s, 1H, CH-(Ph)₂), 4,80 (m, 2H, azétidine), 4,90 (m, 1H, CH-O), 6,80 (m, 2H, H arom.), 7,20-7,50 (m, 10H, H arom.), 8,20 (m, 2H, H arom.).

### 29.2 1-(diphénylméthyl)-3-(4-aminophénoxy)azétidine :

Le protocole expérimental utilisé est le même que celui décrit pour l'intermédiaire 17.5, l'intermédiaire 29.1 remplaçant l'intermédiaire 17.4. On obtient une huile incolore avec un rendement de 75%. RMN ¹H (CDCl₃, 400 MHz, δ) : 3,10 (m, 2H, azétidine), 3,40 (s large, 2H, NH₂), 4,40 (s, 1H, CH-(Ph)₂), 4,70 (m, 2H, azétidine), 4,75 (m, 1H, CH-O), 6,60 (s, 4H, H arom.), 7,10-7,40 (m, 10H, H arom.).

### 29.3 1-(diphénylméthyl)-3-{4-[(1,1-diméthyléthoxy)carbonyl]aminophénoxy}azétidine :

La protection de l'amine est classiquement effectuée par BocOBoc en présence de triéthylamine dans le dichlorométhane. On obtient un solide blanc avec un rendement de 77%. Point de fusion : 149-151°C. RMN ¹H (DMSO d6, 400 MHz, δ) : 1,40 (s, 9H, tBu), 2,90 (s large, 2H, azétidine), 3,60 (s large, 2H, azétidine), 4,50 (s, 1H, CH-(Ph)₂), 4,70 (m, 1H, CH-O), 6,70 (m, 2H, H arom.), 7,10-7,60 (m, 12H, H arom.), 9,10 (s, 1H, NH).

### 29.4 3-{4-[(1,1-diméthyléthoxy)carbonyl]aminophénoxy}azétidine :

Le protocole expérimental utilisé est le même que celui décrit pour l'intermédiaire 14.4 sauf pour le catalyseur d'hydrogénation qui est remplacé par Pd(OH)₂. On obtient un solide blanc avec un rendement de 78%. Point de fusion 184-186°C. RMN ¹H (DMSO d6, 400 MHz, δ) : 1,50 (s, 9H, tBu), 3,50 (m, 2H, azétidine), 3,70 (m, 2H, azétidine), 4,90 (m, 1H, CH-O), 6,70 (m, 2H, H arom.), 7,30 (m, 2H, H arom.), 9,10 (s, 1H, NH).

### 29.5 1-{[3,5-bis(1,1-diméthyléthyl)-4-hydroxyphényl]amino}carbonyl}-3-{4-[(1,1-diméthyléthoxy)carbonyl]aminophénoxy}azétidine :

Une solution de 0,6 g (2,7 mmoles) de l'intermédiaire 10.2 dans 10 ml de dichlorométhane est ajoutée goutte-à-goutte, en une heure, à une solution de 0,27 g (0,9 mmole) de triphosgène dans 6 ml de dichlorométhane. Après 5 minutes d'agitation à 23°C, une solution de 0,72 g (2,7 mmoles) de l'intermédiaire 29.4 et de 0,52 ml (3 mmoles) de diisopropyléthylamine dans 6 ml de dichlorométhane est ajoutée en une seule fois. Le mélange réactionnel est agité pendant 2 heures à 23°C et finalement évaporé à sec sous vide. Le résidu est dilué dans 50 ml d'acétate d'éthyle et cette solution organique est lavée par 2 fois 25 ml d'eau suivi de 25 ml de saumure. Après séchage sur sulfate de magnésium et filtration, la solution organique est concentrée sous vide. Le résidu est purifié sur colonne de silice (éluant : heptane/acétate d'éthyle : 7/3). On obtient un solide blanc avec un rendement de 61 %. Point de fusion : 224-226°C. RMN ¹H (DMSO d6, 400 MHz, δ) : 1,35 (s, 18H, 2 tBu), 1,45 (s, 9H, tBu), 3,80 (m, 2H, azétidine), 4,30 (m, 2H, azétidine), 4,90 (m, 1H, CH-O), 6,60 (s, 1H, OH), 6,70 (m, 2H, H arom.), 7,20 (s, 2H, H arom.), 7,35 (m, 2H, H arom.), 8,20 (s, 1H, NH urée), 9,10 (s, 1 H, NH).

### 29.6 1-{[3,5-bis(1,1-diméthyléthyl)-4-hydroxyphényl]amino}carbonyl}-3-(4-aminophénoxy)azétidine :

Le protocole expérimental utilisé est le même que celui décrit pour l'intermédiaire 26.3, l'intermédiaire 29.5 remplaçant l'intermédiaire 26.2. On obtient un solide blanc avec un rendement de 93%. Point de fusion : 225-227°C. RMN ¹H (DMSO d6, 400 MHz, δ) : 1,30 (s, 18H, 2 tBu), 3,80 (m, 2H, azétidine), 4,30 (m, 2H, azétidine), 4,70 (s large, 2H, NH₂), 4,85 (m, 1H, CH-O), 6,40-6,70 (m, 5H, H arom. + OH), 7,25 (s, 2H, H arom.), 8,20 (s, 1H, NH urée).

### 29.7 Chlorhydrate de 1-{[3,5-bis(1,1-diméthyléthyl)-4-hydroxyphényl]amino}carbonyl}-3-{4-[(imino(2-thiényl)méthyl)amino]phénoxy}azétidine (29) :

Le protocole expérimental utilisé est le même que celui décrit pour l'intermédiaire 4.3, l'intermédiaire 29.6 remplaçant l'intermédiaire 4.2. On obtient un solide blanc avec un rendement de 16%. Point de fusion : 235-240°C. RMN ¹H (DMSO d6, 400 MHz, δ): 1,30 (s, 18H, 2 tBu), 3,90 (m, 2H, azétidine), 4,40 (m, 2H, azétidine), 5,10 (m, 1H, CH-O), 6,60 (s, 1H, OH), 6,90-7,50 (m, 7H, H arom.), 8,20 (m, 2H, thiophène), 8,30 (s, 1H, NH urée), 8,80 (s, 1H, NH⁺), 9,80 (s, 1H, NH⁺), 11,50 (s, 1H, NH⁺). IR : νC=O (urée): 1660 cm⁻¹ ; νC=N (amidine) : 1640 cm⁻¹.

### Exemple 30 : Chlorhydrate de 1-(2-hydroxy-5-méthoxybenzoyl)-3-{4-[(imino(2-thiényl)méthyl)amino]phénoxy}azétidine (30) :

### 30.1 1-(2-hydroxy-5-méthoxybenzoyl)-3-{4-[(1,1-diméthyléthoxy)carbonyl]-aminophénoxy}azétidine :

La condensation de l'acide 2-hydroxy 5-méthoxybenzoÏque et de l'intermédiaire 29.4 est effectuée dans les mêmes conditions expérimentales que celles décrites pour l'intermédiaire 8.1. On obtient un solide blanc avec un rendement de 62%. Point de fusion : 152-153°C. RMN ¹H (DMSO d6, 400 MHz, δ) : 1,50 (s, 9H, tBu), 3,70 (s, 3H, OCH₃), 4,00-4,80 (m, 4H, azétidine), 5,00 (m, 1H, CH-O), 6,70-6,90 (m, 5H, H arom.), 7,30 (m, 2H, H arom.), 9,1 (s, 1H, OH), 10,65 (s, 1H, NH).

### 30.2 1-(2-hydroxy-5-méthoxybenzoyl)-3-aminophénoxy-azétidine :

Le protocole expérimental utilisé est le même que celui décrit pour l'intermédiaire 26.3, l'intermédiaire 30.1 remplaçant l'intermédiaire 26.2. On obtient une huile jaune avec un rendement de 90%. RMN ¹H (DMSO d6, 400 MHz, δ) : 3,25 (s large, 2H, NH₂), 3,80 (s, 3H, OCH₃), 4,20-4,90 (m, 4H, azétidine), 4,95 (m, 1H, CH-O), 6,60-7,00 (m, 7H, H arom.), 11,35 (s large, 1H, OH).

### 30.3 Chlorhydrate de 1-(2-hydroxy-5-méthoxybenzoyl)-3-{4-[(imino(2-thiényl)méthyl)-amino]phénoxy}azétidine (30) :

Le protocole expérimental utilisé est le même que celui décrit pour l'intermédiaire 4.3, l'intermédiaire 30.2 remplaçant l'intermédiaire 4.2. On obtient une poudre blanche avec un rendement de 44%. Point de fusion : 165-166°C. RMN ¹H (DMSO d6, 400 MHz, δ) : 3,70 (s, 3H, OCH₃), 4,00-4,80 (m, 4H, azétidine), 5,15 (m, 1H, CH-O), 6,80-7,10 (m, 5H, H arom.), 7,40 (m, 3H, H arom.), 8,20 (m, 2H, thiophène), 8,75 (s large, 1H, NH⁺), 9,80 (s large, 1H, NH⁺), 10,60 (s, 1H, OH), 11,50 (s large, 1H, NH⁺). IR: νC=O (amide) : 1655 cm⁻¹ ; νC=N (amidine) : 1612 cm⁻¹.

### Exempte 31 : Chlorhydrate de 1-[(3,4-dihydro-6-hydroxy-2,5,7,8-tetraméthyl-2H-[1]-benzopyran-2-yl)carbonyl]-4-[4-[(imino(2-thiényl)méthyl)amino]phénoxy}-pipéridine (31) :

### 31.1 4-(4-nitrophénoxy)-1-pipéridinecarboxylate de 1,1-diméthyléthyle :

Dans un tricol sous atmosphère inerte, refroidi par un bain de glace, on ajoute goutte-à-goutte une solution de 2,01 g (10 mmoles) de N-Boc-4-hydroxypipéridine (préparé classiquement à partir de la 4-hydroxypipéridine commerciale) dans 10 ml de THF sec à une solution de 1,23 g (11 mmoles) de tBuO⁻K⁺ dans 10 ml de THF sec. Après une demi-heure d'agitation à 0°C, on ajoute goutte-à-goutte une solution de 1,06 ml (10 mmoles) de 4-fluoronitrobenzène dans 10 ml de THF sec. Le mélange réactionnel est agité pendant 5 heures à 23°C et finalement versé dans 25 ml d'un mélange eau + glace. Le produit est extrait à l'aide de 50 ml d'acétate d'éthyle. Après décantation, la phase organique est lavée par 2 fois 25 ml d'eau et 25 ml de saumure. Séchage de la solution organique sur sulfate de magnésium, filtration et concentration du filtrat sous vide conduisent à un résidu qui est purifié sur une colonne de silice (éluant : heptane/acétate d'éthyle : 8/2). Les fractions pures sont collectées et évaporées sous vide. On obtient le produit attendu sous forme d'une poudre jaune pâle avec un rendement de 47%. Point de fusion : 97-98°C.

### 31.2 4-(4-nitrophénoxy)pipéridine :

Le protocole expérimental utilisé est le même que celui décrit pour l'intermédiaire 19.2, l'intermédiaire 31.1 remplaçant l'intermédiaire 19.1. On obtient une huile jaune avec un rendement de 87%. RMN ¹H (CDCl₃, 100 MHz, δ) : 1,58 (s, 1H, NH), 1,59-2,19 (m, 4H, CH₂-CH₂), 2,65-3,30 (m, 4H, CH₂-CH₂), 4,51 (m, 1H, CH-O), 6,98 (m, 2H, H arom.), 8,21 (m, 2H, H arom.).

### 31.3 1-[(3,4-dihydro-6-hydroxy-2,5,7,8-tetraméthyl-2H-[1]-benzopyran-2-yl)carbonyl]-4-(4-nitrophényl)pipéridine :

Le protocole expérimental utilisé est le même que celui décrit pour l'intermédiaire 19.3, l'intermédiaire 31.2 remplaçant l'intermédiaire 19.2. On obtient une poudre jaune pâle avec un rendement brut de 83%. Le produit est suffisamment pur pour être investi directement dans l'étape suivante.

### 31.4 1-[(3,4-dihydro-6-hydroxy-2,5,7,8-tetraméthyl-2H-[1]-benzopyran-2-yl)carbonyl]-4-(4-aminophényl)pipéridine :

Le protocole expérimental utilisé est similaire à celui décrit pour l'intermédiaire 14.4, l'intermédiaire 31.3 remplaçant l'intermédiaire 14.3. La réaction s'effectue dans un mélange dichlorométhane/éthanol (1/1). On obtient une poudre blanche avec un rendement de 77%. Point de fusion : 153-154°C. RMN ¹H (CDCl₃ + D₂O, 400 MHz, δ) : 1,60-2,18 (m, 18H, CH₂ + Trolox), 2,52-2,81 (m, 2H, CH₂), 3,41-4,28 (m, 5H, 2 x CH₂ + CH-O), 6,63 (m, 2H, H arom.), 6,74 (m, 2H, H arom.).

### 31.5 Chlorhydrate de 1-[(3,4-dihydro-6-hydroxy-2,5,7,8-tetraméthyl-2H-[1]-benzopyran-2-yl)carbonyl]-4-[4-[(imino(2-thiényl)méthyl)amino]phénoxy}pipéridine (31) :

Le protocole utilisé est le même que celui décrit pour l'intermédiaire 2.4, l'intermédiaire 31.4 remplaçant l'intermédiaire 2.3. La réaction de condensation est effectuée dans le 2-propanol seul. Après salification, on obtient le produit attendu sous forme d'une poudre jaune avec un rendement de 25%. Point de fusion : se décompose à partir de 170°C. RMN ¹H (DMSO d6, 400 MHz, δ) : 1,50-2,10 (m, 18H, CH₂ + Trolox), 2,40-2,65 (m, 2H, CH₂), 3,13-4,37 (m, 4H, 2 x CH₂), 4,64 (m, 1H, CH-O), 7,11 (m, 2H, H arom.), 7,35 (m, 2H, H arom.), 7,57 (s, 1H, H arom.), 8,17 (m, 2H, H arom.), 8,74 (s large, 1H, NH⁺), 9,76 (s large, 1H, NH⁺), 11,42 (s large, 1H, NH⁺). IR : νC=N (amidine) : 1611 cm⁻¹.

### Exemple 32 : Chlorhydrate de 1-[(3,4-dihydro-6-hydroxy-2,5,7,8-tetraméthyl-2H-[1]-benzopyran-2-yl)carbonyl]-3-{4-[(imino(2-thiényl)méthyl)amino]-phénoxy}azétidine (32) :

### 32.1 1-[(3,4-dihydro-6-hydroxy-2,5,7,8-tetraméthyl-2H-[1]-benzopyran-2-yl)carbonyl]-3-{4-[(1,1-diméthyléthoxy)carbonyl]aminophénoxy}azétidine :

La condensation du Trolox et de l'intermédiaire 29.4 est effectuée dans les mêmes conditions expérimentales que celles décrites pour l'intermédiaire 8.1. On obtient un solide blanc avec un rendement de 98%. Point de fusion : 182-183°C. RMN ¹H (CDCl₃ , 400 MHz, δ) : 1,50 (s, 9H, tBu), 1,60-2,60 (m, 16H, Trolox), 3,90-4,90 (m, 5H, azétidine), 6,40 (s, 1H, OH), 6,65 (m, 2H, H arom.), 7,20-7,30 (m, 3H, H arom. + NH).

### 32.2 1-[(3,4-dihydro-6-hydroxy-2,5,7,8-tetraméthyl-2H-[1]-benzopyran-2-yl)carbonyl]-3-aminophénoxy-azétidine :

Le protocole expérimental utilisé est le même que celui décrit pour l'intermédiaire 26.3, l'intermédiaire 32.1 remplaçant l'intermédiaire 26.2. On obtient une mousse blanche avec un rendement de 43%. RMN ¹H (CDCl₃, 400 MHz, δ) : 1,60-2,60 (m, 16H, Trolox), 3,50 (s large, 2H, NH₂), 3,90-4,90 (m, 5H, azétidine), 6,50-6,70 (m, 4H, H arom.).

### 32.3 Chlorhydrate de 1-[(3,4-dihydro-6-hydroxy-2,5,7,8-tetraméthyl-2H-[1]-benzopyran-2-yl)carbonyl]-3-{4-[(imino(2-thiényl)méthyl)amino]phénoxy}azétidine (32) :

Le protocole expérimental utilisé est le même que celui décrit pour l'intermédiaire 4.3, l'intermédiaire 32.2 remplaçant l'intermédiaire 4.2. On obtient une poudre blanche avec un rendement de 56%. Point de fusion : 190-195°C. RMN ¹H (DMSO d6, 400 MHz, δ) : 1,60-2,50 (m, 16H, Trolox), 3,60-5,00 (m, 5H, azétidine), 6,90 (m, 2H, H arom.), 7,30 (m, 3H, H arom.), 8,15 (m, 2H, thiophène), 8,80 (s large, 1H, NH⁺), 9,80 (s large, 1H, NH⁺), 11,50 (s large, 1H, NH⁺). IR : νC=O (amide) : 1647 cm⁻¹ ; νC=N (amidine) : 1611 cm⁻¹.

### Etude pharmacologique des produits de l'invention :

### Etude des effets sur la NO synthase constitutive neuronale de cervelet de rat :

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (*Proc. Natl*. *Acad. Sci. USA,* (1990) **87:** 682-685). Des cervelets de rats Sprague-Dawley (300g - Charles River) sont rapidement prélevés, disséqués à 4°C et homogénéisés dans un volume de tampon d'extraction (HEPES 50 mM, EDTA 1 mM, pH 7,4, pepstatin A 10 mg/ml, leupeptine 10 mg/ml). Les homogénats sont ensuite centrifugés à 21000 g pendant 15 min à 4° C. Le dosage se fait dans des tubes à essai en verre dans lesquels sont distribués 100 µl de tampon d'incubation contenant 100 mM d'HEPES (pH 7,4), 2 mM d'EDTA, 2,5 mM de CaCl₂, 2 mM de dithiotréitol, 2 mM de NADPH réduit et 10 µg/ml de calmoduline. On ajoute 25 µl d'une solution contenant 100 nM de [³H]L-arginine (Activité spécifique : 56,4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES; pH 5,5 ; 2 mM d'EDTA). Après passage des échantillons sur une colonne de 1 ml de résine DOWEX, la radioactivité est quantifiée par un spectromètre à scintillation liquide. Les composés des exemples 1, 6, 7 et 8 décrits ci-dessus présentent une CI₅₀ inférieure à 3,5 µM. Le composé de l'exemple 3 présente quant à lui une CI₅₀ inférieure à 5 µM.

### Etude des effets sur la péroxidation lipidique du cortex cérébral de rat :

L'activité inhibitrice des produits de l'invention est déterminée par la mesure de leur effets sur Le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, *Meth. Enzymol.* (1990) **186** : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4° C. Le culot est conservé à -80° C. Le jour de l'expérience le culot est remis en suspension à la concentration de 1 g/ 15 ml et centrifugé à 515 g pendant 10 minutes à 4° C. Le surnageant est utilisé immédiatement pour la détermination de la péroxidation lipidique. L'homogénat de cortex cérébral de rat (500 µl) est incubé à 37° C pendant 15 minutes en présence des composés à tester ou du solvant (10 µl). La réaction de péroxidation lipidique est initiée par l'ajout de 50 µl de FeCl₂ à 1 mM, d'EDTA à 1 mM et d'acide ascorbique à 4 mM. Après 30 minutes d'incubation à 37° C la réaction est arrêtée par l'ajout de 50 µl d'une solution de di-tertio-butyl toluène hydroxylé (BHT, 0,2 %). Le MDA est quantifié à l'aide d'un test colorimétrique, en faisant réagir un réactif chromogène (R) le N-méthyl-2-phenylindole (650 µl) avec 200 µl de l'homogénat pendant 1 heure à 45° C. La condensation d'une molécule de MDA avec deux molécules de réactif R produit un chromophore stable dont la longueur d'onde d'absorbance maximale est égale à 586 nm. (Caldwell et coll. *European J. Pharmacol*. (1995) **285**, 203-206). Les composés des exemples 3, 11, 12, 13, 14 et 15 décrits ci-dessus présentent une CI₅₀ inférieure à 30 µM.

## Revendications

1. Composé **caractérisé en ce qu'**il répond à la formule générale **(I)** 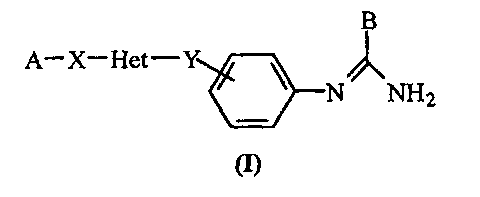 dans laquelle :
A est un aromatique correspondant aux structures : 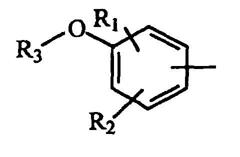
dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
ou 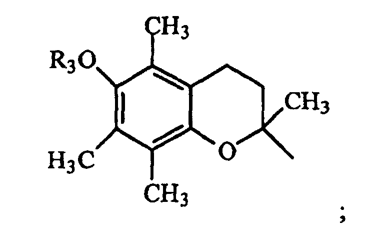
B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine ;
ou sel d'addition à un acide dudit composé.

2. Composé selon la revendication 1, **caractérisé en ce que** :
A est un aromatique correspondant à la structure : 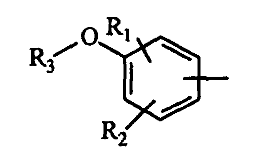
dans laquelle :
R₁ et R₂ représentent, indépendamment un radical alkyle linéaire ou ramifié ayant de 1 à 6 atomes de carbone ou un radical alkoxy linéaire ou ramifié ayant de 1 à 6 atomes de carbone,
R₃ représente un atome d'hydrogène ou un radical alkyle linéaire ou ramifié ayant de 1 à 6 atomes de carbone;
B représente un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène ;
X représente -NH-CO-X'-, -CH=, -CO- ou une liaison,
X' représentant -(CH₂)ₙ- avec n entier de 0 à 6 ;
Y représente -Y'-, -Y'-NH-CO-, -Y'-CO-, -Y'-O-, -Y'-O-Y'-, -Y'-N(R₃)-Y'- ou une liaison, Y' représentant -(CH₂)ₙ- avec n entier de 0 à 6 ;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine ;
ou sel d'addition à un acide dudit composé.

3. Composé selon la revendication 2, **caractérisé en ce que** B représente un cycle thiophène, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène ;
ou sel d'addition à un acide dudit composé.

4. Composé selon l'une des revendications 1 à 3, **caractérisé en ce qu'**il s'agit d'un des composés suivants :
- iodhydrate de N-(3,5-di-t-butyl-4-hydroxyphényl)-5-[4-{imino(2-thiényl)-méthylamino}phényl]-2-furanne carboxamide;
- chlorhydrate de 3-(3,5-di-t-butyl-4-hydroxyphényl)-1-[4-{imino(2-thiényl)-méthylamino}phényl]-2,5-imidazolidinedione;
- chlorhydrate de 2-(3,5-di-t-butyl-4-hydroxyphényl)-3-[4-{imino(2-thiényl)-méthylamino}phényl]-4-thiazolidinone;
- fumarate de 5-[(3,5-di-t-butyl-4-hydroxyphényl)méthylène]-1-méthyl-3-[4-{imino(2-thiényl)méthylamino}phényl]-2,4-imidazolidinedione;
- chlorhydrate de 2-(*S*)-4-(*S*)-N-[4-hydroxy-3,5-bis-(1,1-diméthyléthyl)-phényl]-4- {4-[(imino(2-thiényl)méthyl)amino]phénoxy}-prolinamide;
- fumarate de N-[4-hydroxy-3,5-bis-(1,1-diméthyléthyl)phényl]-2-(*R,S*)-{4-[(imino(2-thiényl)méthyl)amino]phényl}-4-(*R*)-thiazolidine carboxamide;
- iodhydrate de N-[3,5-bis(1,1-diméthyléthyl)-4-hydroxyphényl]-2-{4-[(imino(2-thiényl)méthyl)amino]phényl}-4-thiazolecarboxamide
- dichlorhydrate de N-[3,5-bis(1,1-diméthyléthyl)-4-hydroxyphényl]-4-(S)-{4-[(imino(2-thiényl)méthyl)amino]phénoxy}-pyrrolidine-2-(R)-carboxamide
- chlorhydrate de 1-[(3,4-dihydro-6-hydroxy-2,5,7,8-tétraméthyl-2-H-[1]-benzopyran-2-yl)carbonyl]-4-(S)-{4-[(imino(2-thiényl)méthyl)amino]-phénoxy}-pyrrolidine-2-(S)-carboxylate de méthyle
- chlorhydrate de 1-[(3,4-dihydro-6-hydroxy-2,5,7,8-tétraméthyl-2H-[1]-benzopyran-2-yl)carbonyl]-3-(S)-{4-[(imino(2-thiényl)méthyl)amino]phénoxy}-pyrrolidine
- 3-{[(3,4-dihydro-6-hydroxy-2,5,7,8-tétraméthyl-2H-[1]-benzopyran-2-yl)-carbonyl]amino}-1-{4-[(imino(2-thiényl)méthyl)amino]phényl}pyrrolidine
- chlorhydrate de 4-[3,5-bis-(1,1-diméthyléthyl)-4-hydroxyphényl]-N-{4-[(imino(2-thiényl)méthyl)amino]benzoyl}-N-méthyl-1H-imidazole-2-méthanamine
- iodhydrate de N-[3,5-bis-(1,1-diméthyléthyl)-4-hydroxyphényl]-1-{4-[(imino(2-thiényl)méthyl)amino]phényl}-1H-pyrrole-2-carboxamide
- iodhydrate de 1-[3,5-bis(1,1-diméthyléthyl)-4-hydroxyphényl]-3-{[4-[[imino(2-thiényl)méthyl]amino]phényl]carbonyl}-2-imidazolidinone
- iodhydrate de 3-[3,5-bis(1,1-diméthyléthyl)-4-hydroxyphényl]-4,5-dihydro-N-{4-[(imino(2-thiényl)méthyl)amino]phényl}-5-isoxazoleacétamide
- chlorhydrate de 4-[3,5-bis(1,1-diméthyléthyl)-4-hydroxyphényl]-N-{4-[(imino(2-thiényl)méthyl)amino]phényl}-N-méthyl-2-thiazoleméthanamine
- chlorhydrate de 4-[3,5-bis(1,1-diméthyléthyl)-4-hydroxyphényl]-N-{4-[(imino(2-thiényl)méthyl)amino]phényl}-N-méthyl-1H-imidazole-2-méthanamine
- 3-[3,5-bis(1,1-diméthyléthyl)-4-hydroxyphényl]-4,5-dihydro-5-{2-{4-[(imino(2-thiényl)méthyl)amino]phénoxy}éthyl}isoxazole
- chlorhydrate de 1-{[3,5-bis(1,1-diméthyléthyl)-4-hydroxyphényl]amino}-carbonyl}-3-{4-[(imino(2-thiényl)méthyl)amino]phénoxy}azétidine
- chlorhydrate de 1-(2-hydroxy-5-méthoxybenzoyl)-3-{4-[(imino(2-thiényl)méthyl)amino]phénoxy}azétidine
- chlorhydrate de 1-[(3,4-dihydro-6-hydroxy-2,5,7,8-tetraméthyl-2H-[1]-benzopyran-2-yl)carbonyl]-4-[4-[(imino(2-thiényl)méthyl)amino]phénoxy}-pipéridine
- chlorhydrate de 1-[(3,4-dihydro-6-hydroxy-2,5,7,8-tetraméthyl-2H-[1]-benzopyran-2-yl)carbonyl]-3-{4-[(imino(2-thiényl)méthyl)amino]-phénoxy}azétidine
ou d'un de leurs sels ou énantiomères.

5. Composé selon l'une quelconque des revendications 1 à 4, **caractérisé en ce qu'**il s'agit d'un des composés suivants :
- chlorhydrate de 3-(3,5-di-t-butyl-4-hydroxyphényl)-1-[4-{imino(2-thiényl)-méthylamino}phényl]-2,5-imidazolidinedione;
- chlorhydrate de 2-(3,5-di-t-butyl-4-hydroxyphényl)-3-[4-{imino(2-thiényl)-méthylamino}phényl]-4-thiazolidinone;
- fumarate de 5-[(3,5-di-t-butyl-4-hydroxyphényl)méthylène]-1-méthyl-3-[4-{imino(2-thiényl)méthylamino}phényl]-2,4-imidazolidinedione;
- chlorhydrate de 2-(*S*)-4-(*S*)-N-[4-hydroxy-3,5-bis-(1,1-diméthyléthyl)-phényl]-4-{4-[(imino(2-thiényl)méthyl)amino]phénoxy}-prolinamide;
- chlorhydrate de N-[4-hydroxy-3,5-bis-(1,1-diméthyl)éthyl-phényl]-2-{4-[(imino(2-thiényl)méthyl)amino]phényl}-4-thiazole carboxamide ;
ou d'un de leurs sels ou énantiomères.

6. A titre de produits industriels nouveaux, les composés de formules générales **(V), (VI) et (VII)** 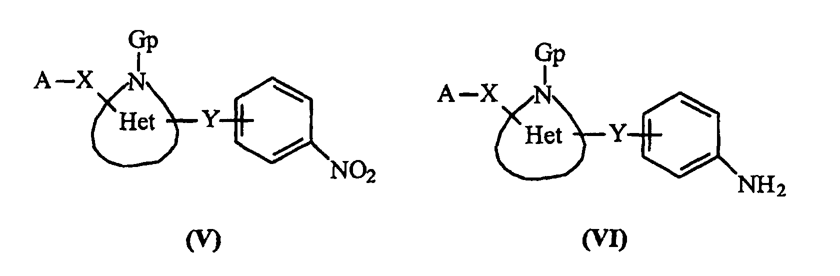 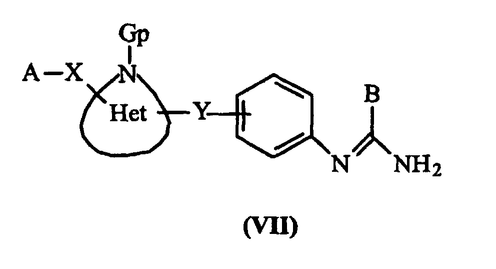 pour lesquels
A est un aromatique correspondant aux structures : 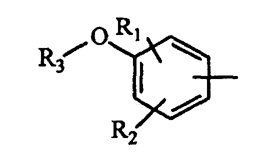 dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
ou 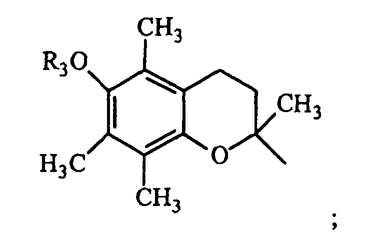 B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétàne, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine ;
Gₚ représente un groupe protecteur de fonction amine clivable en milieu acide anhydre.

7. Procédé de préparation de composés de formule générale **(I)** selon la revendication 1, **caractérisé en ce qu'**il consiste en la condensation, de préférence dans un mélange d'isopropanol et de DMF et à température ambiante, de dérivés d'aniline de formule générale **(III)** 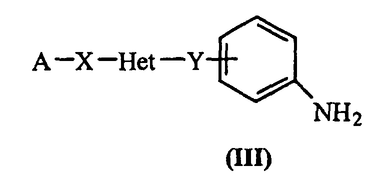 sur des dérivés S-alkyl thioimidate de formule générale **(IV)** 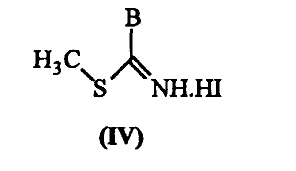 pour conduire aux composés finaux de formule générale **(I),**
les composés de formule générale **(I), (III)** et **(IV)** étant tels que
A est un aromatique correspondant aux structures : 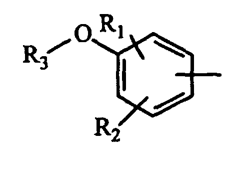 dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
ou 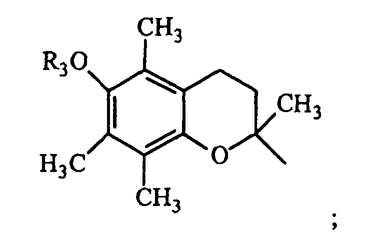 B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyl ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine.

8. Procédé de préparation de composés de formule générale **(I)** selon la revendication 1 dont l'hétérocycle Het comprend au moins un atome d'azote, **caractérisé en ce qu'**il comprend les deux étapes suivantes :
- la condensation, de préférence dans un mélange d'isopropanol et de DMF et à température ambiante, d'un composé de formule générale **(VI)** 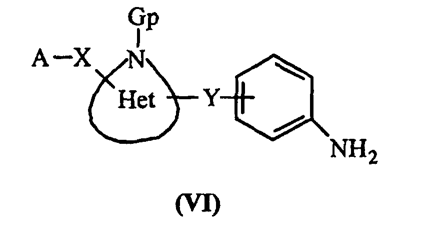 avec un composé de formule générale **(IV)** 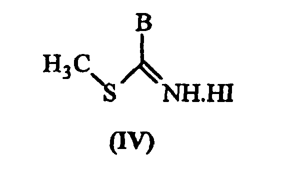 pour donner un composé de formule générale **(VII)** 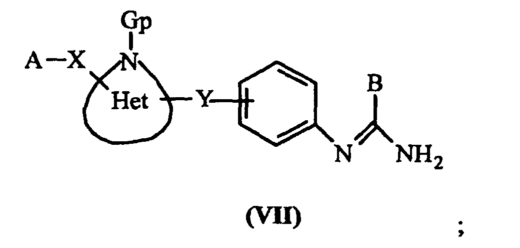
- le clivage du groupe protecteur Gₚ du composé de formule générale **(VII)** défini ci-dessus pour obtenir le composé de formule générale **(I),**
les composés de formule générale **(I), (IV), (VI)** et **(VII)** étant tels que
A est un aromatique correspondant aux structures : 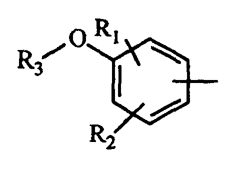
dans laquelle :
R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
ou 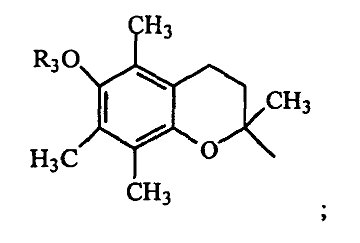 B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un alkyle ayant de 1 à 6 atomes de carbone linéaire ou ramifié, un radical alkoxy ayant de 1 à 6 atomes de carbone ou un halogène;
X représente -CO-N(R₃)-X'-, -NH-CO-X'-, -CH=, -CO- ou une liaison,
X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine, à l'exception des hétérocycles suivants : pipérazines, homopipérazines, 4-aminopipéridine ;
Gₚ représente un groupe protecteur de fonction amine de préférence clivable en milieu acide anhydre, tel que par exemple les carbamates de t-butyle, trichloroéthyle ou de triméthylsilyléthyle ou encore le groupe trityle.

9. A titre de médicament, un composé de formule générale **(I)** selon l'une quelconque des revendications 1 à 5, ou un sel pharmaceutiquement acceptable de ce composé.

10. Composition pharmaceutique contenant à titre de principe actif au moins un composé selon la revendication 9.

11. Utilisation d'un composé de formule générale **(I)** selon l'une quelconque des revendications 1 à 5, ou d'un sel pharmaceutiquement acceptable de ce composé, pour fabriquer un médicament destiné à inhiber la NO synthase.

12. Utilisation d'un composé de formule générale **(I)** selon l'une quelconque des revendications 1 à 5, ou d'un sel pharmaceutiquement acceptable de ce composé, pour fabriquer un médicament destiné à inhiber la peroxydation lipidique.

13. Utilisation d'un composé de formule générale **(I)** selon l'une quelconque des revendications 1 à 5, ou d'un sel pharmaceutiquement acceptable de ce composé, pour fabriquer un médicament ayant à la fois une activité d'inhibition de la NO synthase et d'inhibition de la peroxydation lipidique.

14. Utilisation d'un composé de formule générale **(I)** selon l'une quelconque des revendications 1 à 5, ou d'un sel pharmaceutiquement acceptable de ce composé, pour fabriquer un médicament destiné à traiter les troubles cardio-vasculaires et cérébro-vasculaires.

15. Utilisation d'un composé de formule générale **(I)** selon l'une quelconque des revendications 1 à 5, ou d'un sel pharmaceutiquement acceptable de ce composé, pour fabriquer un médicament destiné à traiter les troubles du système nerveux central ou périphérique.

## Patentansprüche

1. Verbindung, **dadurch gekennzeichnet, daß** sie der allgemeinen Formel (I) entspricht 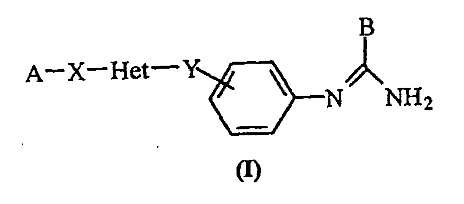 in der:
A ein aromatischer Rest ist, der den Strukturen 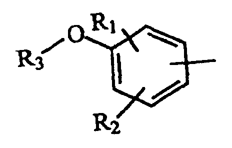 worin
R₁ und R₂ unabhängig voneinander ein Wasserstoffatom, ein Halogenatom, die OH-Gruppe, einen linearen oder verzweigten Alkylrest mit 1 bis 6 Kohlenstoffatomen, einen linearen oder verzweigten Alkoxyrest mit 1 bis 6 Kohlenstoffatomen darstellen,
R₃ ein Wasserstoffatom, einen linearen oder verzweigten Alkylrest mit 1 bis 6 Kohlenstoffatomen oder einen Rest -COR₄ darstellt, wobei R₄ einen Alkylrest mit 1 bis 6 Kohlenstoffatomen darstellt,
oder 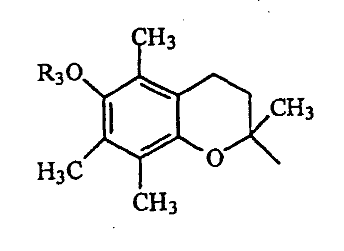 entspricht,
B einen linearen oder verzweigten Alkylrest mit 1 bis 6 Kohlenstoffatomen, Phenyl, Pyridinyl oder einen Heterocyclus mit 5 Kettengliedern darstellt, der 1 bis 4 Heteroatome enthält, die aus O, S, N ausgewählt sind, und insbesondere: Thiophen, Furan, Pyrrol oder Thiazol, deren Kohlenstoffatome ggf. durch eine oder mehrere Gruppen substituiert sind, die aus einem linearen oder verzweigten Alkyl mit 1 bis 6 Kohlenstoffatomen, einem Alkoxyrest mit 1 bis 6 Kohlenstoffatomen oder einem Halogenatom ausgewählt sind;
X -CO-N(R₃)-X'-, -NH-CO-X'-, -CH=, -CO- oder eine Bindung darstellt, wobei X' -(CH₂)ₙ- mit n einer ganzen Zahl von 0 bis 6 darstellt;
Y -Y'-, -CO-NH-Y', -Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- oder eine Bindung darstellt, wobei Y' -(CH₂)ₙ- mit n einer ganzen Zahl von 0 bis 6 darstellt;
Het einen Heterocyclus mit 1 bis 5 Heteroatomen darstellt, die aus O, N, S ausgewählt sind und durch einen oder mehrere Substituenten X'-OR₃, X'-NR₃, X'-S-R₃ substituiert sein können, wie z.B.:
Oxetan, Pyrrol, Pyrrolidin, Furan, Tetrahydrofuran, Thiophen, Tetrahydrothiophen, Sulfolan, Imidazol, Imidazolin, Dihydroimidazol-2-on, Dihydroimidazol-2-thion, Oxazol, Isoxazol, Oxazolin, Isoxazolin, Oxazolidin, Oxazolidinon, Thiazol, Thiazolin, Thiazolidin, Thiazolidinon, Hydantoin, 1,2,4-Triazol, 1,3,4-Oxadiazol, 1,3,4-Thiadiazol, 1,1-Dioxid-1,2,5-thiadiazolidin, 1,2,4-Triazol-3-on, Tetrazol, Tetrahydropyridin, Azetidin, mit Ausnahme der folgenden Heterocyclen: Piperazine, Homopiperazine, 4-Aminopiperidin;
oder ein Säureadditionssalz dieser Verbindung.

2. Verbindung nach Anspruch 1, **dadurch gekennzeichnet, daß**:
A ein aromatischer Rest ist, der der Struktur 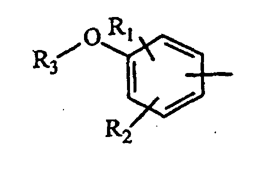 entspricht, in der:
R₁ und R₂ unabhängig voneinander einen linearen oder verzweigten Alkylrest mit 1 bis 6 Kohlenstoffatomen oder einen linearen oder verzweigten Alkoxyrest mit 1 bis 6 Kohlenstoffatomen darstellen,
R₃ ein Wasserstoffatom oder einen linearen oder verzweigten Alkylrest mit 1 bis 6 Kohlenstoffatomen darstellt;
B einen Heterocyclus mit 5 Kettengliedern darstellt, der 1 bis 4 Heteroatome enthält, die aus O, S, N ausgewählt sind, und insbesondere: Thiophen, Furan, Pyrrol oder Thiazol, deren Kohlenstoffatome ggf. durch eine oder mehrere Gruppen substituiert sind, die aus einem linearen oder verzweigten Alkyl mit 1 bis 6 Kohlenstoffatomen, einem Alkoxyrest mit 1 bis 6 Kohlenstoffatomen oder einem Halogenatom ausgewählt sind;
X -NH-CO-X'-, -CH=, -CO- oder eine Bindung darstellt, wobei X' -(CH₂)ₙ- mit n einer ganzen Zahl von 0 bis 6 darstellt;
Y -Y'-, -Y'-NH-CO-, -Y'-CO-,-Y'-O, -Y'-O-Y'-, -Y'-N(R₃)-Y'- oder eine Bindung darstellt, wobei Y' -(CH₂)ₙ- mit n einer ganzen Zahl von 0 bis 6 darstellt;
Het einen Heterocyclus mit 1 bis 5 Heteroatomen darstellt, die aus O, N, S ausgewählt sind und durch einen oder mehrere Substituenten X'-OR₃, X'-NR₃, X'-S-R₃ substituiert sein können, wie z.B.:
Oxetan, Pyrrol, Pyrrolidin, Furan, Tetrahydrofuran, Thiophen, Tetrahydrothiophen, Sulfolan, Imidazol, Imidazolin, Dihydroimidazol-2-on, Dihydroimidazol-2-thion, Oxazol, Isoxazol, Oxazolin, Isoxazolin, Oxazolidin, Oxazolidinon, Thiazol, Thiazolin, Thiazolidin, Thiazolidinon, Hydantoin, 1,2,4-Triazol, 1,3,4-Oxadiazol, 1,3,4-Thiadiazol, 1,1-Dioxid-1,2,5-thiadiazolidin, 1,2,4-Triazol-3-on, Tetrazol, Tetrahydropyridin, Azetidin, mit Ausnahme der folgenden Heterocyclen: Piperazine, Homopiperazine, 4-Aminopiperidin;
oder ein Säureadditionssalz dieser Verbindung.

3. Verbindung nach Anspruch 2, **dadurch gekennzeichnet, daß** B einen Thiophencyclus darstellt, dessen Kohlenstoffatome ggf. durch eine oder mehrere Gruppen substituiert sind, die aus einem linearen oder verzweigten Alkyl mit 1 bis 6 Kohlenstoffatomen, einem Alkoxyrest mit 1 bis 6 Kohlenstoffatomen oder einem Halogenatom ausgewählt sind;
oder ein Säureadditionssalz dieser Verbindung.

4. Verbindung nach einem der Ansprüche 1 bis 3, **dadurch gekennzeichnet, daß** es sich um eine der folgenden Verbindungen handelt:
- N-(3,5-Di-t-butyl-4-hydroxyphenyl)-5-[4-{imino(2-thienyl)methylamino}phenyl]-2-furancarboxamidiodhydrat;
- 3-(3,5-Di-t-butyl-4-hydroxyphenyl)-1-[4-{imino(2-thienyl)methylamino}phenyl]-2,5-imidazolidindionchlorhydrat;
- 2-(3,5-Di-t-butyl-4-hydroxyphenyl)-3-[4-{imino(2-thienyl)methylamino}phenyl]-4-thiazolidinonchlorhydrat;
- 5-[(3,5-Di-t-butyl-4-hydroxyphenyl)methylen]-1-methyl-3-[4-{imino(2-thienyl)methylamino}phenyl]-2,4-imidazolidindionfumarat;
- 2-(*S*)-4-(*S*)-N-[4-Hydroxy-3,5-bis-(1,1-dimethylethyl)-phenyl]-4-{4-[(imino(2-thienyl)methyl)amino]phenoxy}-prolinamidchlorhydrat;
- N-[4-Hydroxy-3,5-bis-(1,1-dimethylethyl)phenyl]-2-(*R*,*S*)-{4-[(imino(2-thienyl)methyl)amino]phenyl}-4-(*R*)-thiazolidincarboxamidfumarat;
- N-[3,5-Bis(1,1-dimethylethyl)-4-hydroxyphenyl]-2-{4-[(imino(2-thienyl)methyl)amino]phenyl}-4-thiazolcarboxamidiodhydrat;
- N-[3,5-Bis(1,1-dimethylethyl)-4-hydroxyphenyl]-4-(*S*)-{4-[(imino(2-thienyl)methyl)amino]phenoxy}-pyrrolidin-2-(*R*)-carboxamiddichlorhydrat;
- 1-[(3,4-Dihydro-6-hydroxy-2,5,7,8-tetramethyl-2-H-[1]-benzopyran-2-yl)carbonyl]-4-(*S*)-{4-[(imino(2-thienyl)-methyl)amino]phenoxy}-pyrrolidin-2-(*S*)-methylcarboxylatchlorhydrat;
- 1-[(3,4-Dihydro-6-hydroxy-2,5,7,8-tetramethyl-2H-[1]-benzopyran-2-yl)carbonyl]-3-(*S*)-{4-[(imino(2-thienyl)-methyl)amino]phenoxy}-pyrrolidinchlorhydrat;
- 3-{[(3,4-Dihydro-6-hydroxy-2,5,7,8-tetramethyl-2H-[1]-benzopyran-2-yl)carbonyl]amino}-1-{4-[(imino(2-thienyl)-methyl)amino]phenyl}pyrrolidin;
- 4-[3,5-Bis-(1,1-dimethylethyl)-4-hydroxyphenyl]-N-{4-[(imino(2-thienyl)methyl)amino]benzoyl}-N-methyl-1H-imidazol-2-methanaminchlorhydrat;
- N-[3,5-Bis-(1,1-dimethylethyl)-4-hydroxyphenyl]-1-{4-[(imino(2-thienyl)methyl)amino]phenyl}-1H-pyrrol-2-carboxamidiodhydrat;
- 1-[3,5-Bis-(1,1-dimethylethyl)-4-hydroxyphenyl]-3-{[4-[[imino(2-thienyl)methyl]amino]phenyl]carbonyl}-2-imidazolidinoniodhydrat;
- 3-[3,5-Bis-(1,1-dimethylethyl)-4-hydroxyphenyl]-4,5-dihydro-N-{4-[(imino(2-thienyl)methyl)amino]phenyl}-5-isoxazolacetamidiodhydrat;
- 4-[3,5-Bis-(1,1-dimethylethyl)-4-hydroxyphenyl]-N-{4-[(imino(2-thienyl)methyl)amino]phenyl}-N-methyl-2-thiazolmethanaminchlorhydrat;
- 4-[3,5-Bis(1,1-dimethylethyl)-4-hydroxyphenyl]-N-{4-[(imino(2-thienyl)methyl)amino]phenyl}-N-methyl-1H-imidazol-2-methanaminchlorhydrat;
- 3-[3,5-Bis(1,1-dimethylethyl)-4-hydroxyphenyl]-4,5-dihydro-5-{2-{4-[(imino(2-thienyl)methyl)amino]-phenoxxy}ethyl}isoxazol;
- 1-{[3,5-Bis(1,1-dimethylethyl)-4-hydroxyphenyl]amino}-carbonyl}-3-{4-[(imino(2-thienyl)methyl)amino]phenoxy}-azetidinchlorhydrat;
- 1-(2-Hydroxy-5-methoxybenzoyl)-3-{4-[(imino(2-thienyl)-methyl)amino]phenoxy}azetidinchlorhydrat;
- 1-[(3,4-Dihydro-6-hydroxy-2,5,7,8-tetramethyl-2H-[1]-benzopyran-2-yl)carbonyl]-4-{4-[(imino(2-thienyl)methyl)-amino]phenoxy}-piperidinchlorhydrat;
- 1-E(3,4-Dihydro-6-hydroxy-2,5,7,8-tetramethyl-2H-[1]-benzopyran-2-yl)carbonyl]-3-{4-[(imino(2-thienyl)methyl)-amino]phenoxy}-azetidinchlorhydrat;
oder eines ihrer Salze oder Enantiomere.

5. Verbindung nach einem der Ansprüche 1 bis 4, **dadurch gekennzeichnet, daß** es sich um eine der folgenden Verbindungen handelt:
- 3-(3,5-Di-t-butyl-4-hydroxyphenyl)-1-[4-{imino(2-thienyl)methylamino}phenyl]-2,5-imidazolidindionchlorhydrat;
- 2-(3,5-Di-t-butyl-4-hydroxyphenyl)-3-[4-{imino(2-thienyl)methylamino}phenyl]-4-thiazolidinonchlorhydrat;
- 5-[(3,5-Di-t-butyl-4-hydroxyphenyl)methylen]-1-methyl-3-[4-{imino(2-thienyl)methylamino}phenyl]-2,4-imidazolidindionfumarat;
- 2-(*S*)-4-(*S*)-N-[4-hydroxy-3,5-bis-(1,1-dimethylethyl)-phenyl]-4-{4-[(imino(2-thienyl)methyl)amino]phenoxy}-prolinamidchlorhydrat;
- N-[4-Hydroxy-3,5-bis-(1,1-dimethyl)ethylphenyl]-2-{4-[(imino(2-thienyl)methyl)amino]phenyl}-4-thiazolcarboxamidchlorhydrat;
oder eines ihrer Salze oder Enantiomere.

6. Die Verbindungen der allgemeinen Formeln (V), (VI) und (VII) in Form eines neuen Industrieprodukts 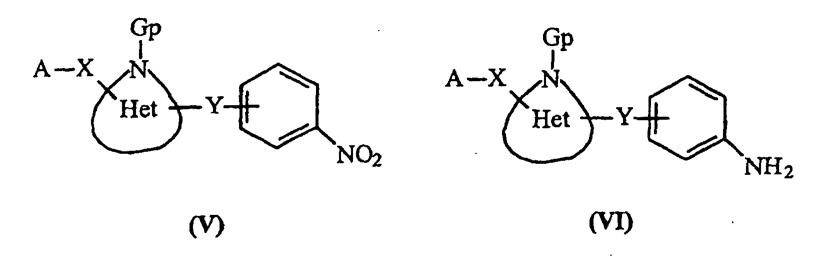 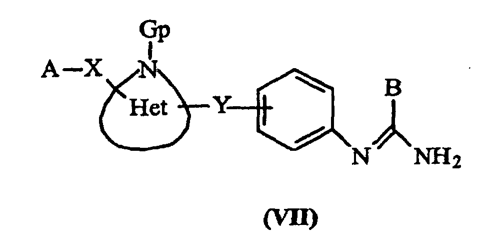 bei denen
A ein aromatischer Rest ist, der den Strukturen 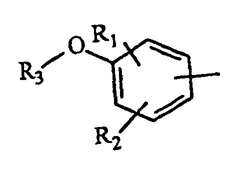 worin:
R₁ und R₂ unabhängig voneinander ein Wasserstoffatom, ein Halogenatom, die OH-Gruppe, einen linearen oder verzweigten Alkylrest mit 1 bis 6 Kohlenstoffatomen, einen linearen oder verzweigten Alkoxyrest mit 1 bis 6 Kohlenstoffatomen darstellen,
R₃ ein Wasserstoffatom, einen linearen oder verzweigten Alkylrest mit 1 bis 6 Kohlenstoffatomen oder einen Rest -COR₄ darstellt, wobei R₄ einen Alkylrest mit 1 bis 6 Kohlenstoffatomen darstellt,
oder 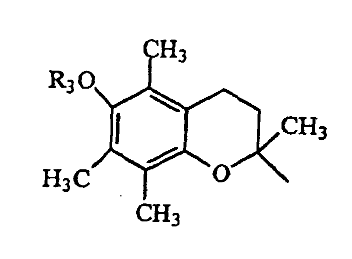 entspricht;
B einen linearen oder verzweigten Alkylrest mit 1 bis 6 Kohlenstoffatomen, Phenyl, Pyridinyl oder einen Heterocyclus mit 5 Kettengliedern darstellt, der 1 bis 4 Heteroatome enthält, die aus O, S, N ausgewählt sind, und insbesondere: Thiophen, Furan, Pyrrol oder Thiazol, deren Kohlenstoffatome ggf. durch eine oder mehrere Gruppen substituiert sind, die aus einem linearen oder verzweigten Alkyl mit 1 bis 6 Kohlenstoffatomen, einem Alkoxyrest mit 1 bis 6 Kohlenstoffatomen oder einem Halogenatom ausgewählt sind;
X -CO-N(R₃)-X'-, -NH-CO-X'-, -CH=, -CO- oder eine Bindung darstellt, wobei X' -(CH₂)ₙ- mit n einer ganzen Zahl von 0 bis 6 darstellt;
Y -Y'-, -CO-NH-Y', -Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- oder eine Bindung darstellt, wobei Y' -(CH₂)ₙ- mit n einer ganzen Zahl von 0 bis 6 darstellt;
Het einen Heterocyclus mit 1 bis 5 Heteroatomen darstellt, die aus O, N, S ausgewählt sind und durch einen oder mehrere Substituenten X'-OR₃, X'-NR₃, X'-S-R₃ substituiert sein können, wie z.B.:
Oxetan, Pyrrol, Pyrrolidin, Furan, Tetrahydrofuran, Thiophen, Tetrahydrothiophen, Sulfolan, Imidazol, Imidazolin, Dihydroimidazol-2-on, Dihydroimidazol-2-thion, Oxazol, Isoxazol, Oxazolin, Isoxazolin, Oxazolidin, Oxazolidinon, Thiazol, Thiazolin, Thiazolidin, Thiazolidinon, Hydantoin, 1,2,4-Triazol, 1,3,4-Oxadiazol, 1,3,4-Thiadiazol, 1,1-Dioxid-1,2,5-thiadiazolidin, 1,2,4-Triazol-3-on, Tetrazol, Tetrahydropyridin, Azetidin, mit Ausnahme der folgenden Heterocyclen: Piperazine, Homopiperazine, 4-Aminopiperidin;
G_{P} eine in wasserfreiem saurem Medium abspaltbare Schutzgruppe für die Aminfunktion ist.

7. Verfahren zur Herstellung von Verbindungen der allgemeinen Formel (I) nach Anspruch 1, **dadurch gekennzeichnet, daß** es in der Kondensierung von Anilinderivaten der allgemeinen Formel (III) 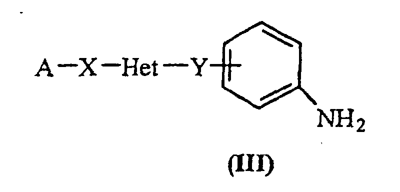 vorzugsweise in einer Mischung von Isopropanol und DMF und bei Raumtemperatur an S-Alkylthioimidat-Derivate der allgemeinen Formel (IV) 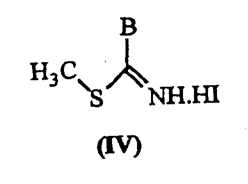 besteht, um zu den Endverbindungen der allgemeinen Formel (I) zu führen,
wobei die Verbindungen der allgemeinen Formel (I), (III) und (IV) so beschaffen sind, daß
A ein aromatischer Rest ist, der den Strukturen 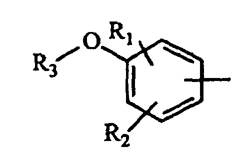 worin
R₁ und R₂ unabhängig voneinander ein Wasserstoffatom, ein Halogenatom, die OH-Gruppe, einen linearen oder verzweigten Alkylrest mit 1 bis 6 Kohlenstoffatomen, einen linearen oder verzweigten Alkoxyrest mit 1 bis 6 Kohlenstoffatomen darstellen,
R₃ ein Wasserstoffatom, einen linearen oder verzweigten Alkylrest mit 1 bis 6 Kohlenstoffatomen oder einen Rest -COR₄ darstellt, wobei R₄ einen Alkylrest mit 1 bis 6 Kohlenstoffatomen darstellt,
oder 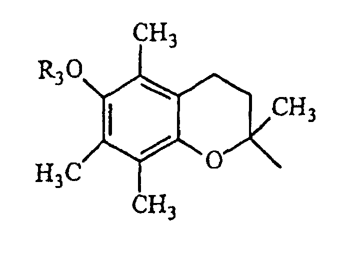 entspricht;
B einen linearen oder verzweigten Alkylrest mit 1 bis 6 Kohlenstoffatomen, Phenyl, Pyridinyl oder einen Heterocyclus mit 5 Kettengliedern darstellt, der 1 bis 4 Heteroatome enthält, die aus O, S, N ausgewählt sind, und insbesondere: Thiophen, Furan, Pyrrol oder Thiazol, deren Kohlenstoffatome ggf. durch eine oder mehrere Gruppen substituiert sind, die aus einem linearen oder verzweigten Alkyl mit 1 bis 6 Kohlenstoffatomen, einem Alkoxyrest mit 1 bis 6 Kohlenstoffatomen oder einem Halogenatom ausgewählt sind;
X -CO-N(R₃)-X'-, -NH-CO-X'-, -CH=, -CO- oder eine Bindung darstellt, wobei X' -(CH₂)ₙ- mit n einer ganzen Zahl von 0 bis 6 darstellt;
Y -Y'-, -CO-NH-Y', -Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- oder eine Bindung darstellt, wobei Y' -(CH₂)ₙ- mit n einer ganzen Zahl von 0 bis 6 darstellt;
Het einen Heterocyclus mit 1 bis 5 Heteroatomen darstellt, die aus O, N, S ausgewählt sind und durch einen oder mehrere Substituenten X'-OR₃, X'-NR₃, X'-S-R₃ substituiert sein können, wie z.B.:
Oxetan, Pyrrol, Pyrrolidin, Furan, Tetrahydrofuran, Thiophen, Tetrahydrothiophen, Sulfolan, Imidazol, Imidazolin, Dihydroimidazol-2-on, Dihydroimidazol-2-thion, Oxazol, Isoxazol, Oxazolin, Isoxazolin, Oxazolidin, Oxazolidinon, Thiazol, Thiazolin, Thiazolidin, Thiazolidinon, Hydantoin, 1,2,4-Triazol, 1,3,4-Oxadiazol, 1,3,4-Thiadiazol, 1,1-Dioxid-1,2,5-thiadiazolidin, 1,2,4-Triazol-3-on, Tetrazol, Tetrahydropyridin, Azetidin, mit Ausnahme der folgenden Heterocyclen: Piperazine, Homopiperazine, 4-Aminopiperidin.

8. Verfahren zur Herstellung von Verbindungen der allgemeinen Formel (I) nach Anspruch 1, deren Heterocyclus Het mindestens ein Stickstoffatom aufweist,
**dadurch gekennzeichnet, daß** es die beiden folgenden Schritte umfasst:
- Kondensation einer Verbindung der allgemeinen Formel (VI) 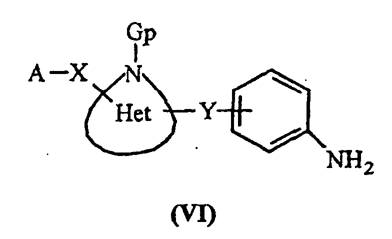 vorzugsweise in einer Mischung von Isopropanol und DMF und bei Raumtemperatur mit einer Verbindung der allgemeinen Formel (IV) 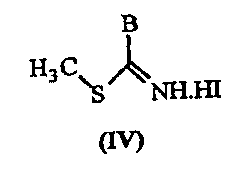 um eine Verbindung der allgemeinen Formel (VII) zu ergeben 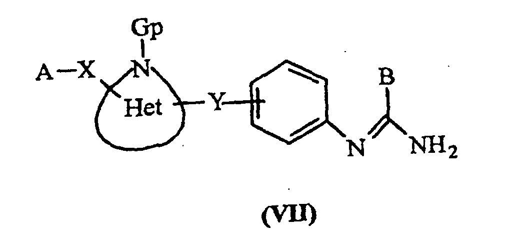
- Abspaltung der Schutzgruppe GP der oben definierten Verbindung der allgemeinen Formel (VII), um die Verbindung der allgemeinen Formel (I) zu erhalten,
wobei die Verbindungen der allgemeinen Formel (I), (IV), (VI) und (VII) so beschaffen sind, daß
A ein aromatischer Rest ist, der den Strukturen 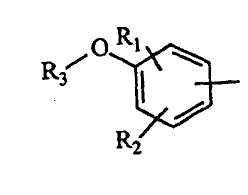 worin:
R₁ und R₂ unabhängig voneinander ein Wasserstoffatom, ein Halogenatom, die OH-Gruppe, einen linearen oder verzweigten Alkylrest mit 1 bis 6 Kohlenstoffatomen, einen linearen oder verzweigten Alkoxyrest mit 1 bis 6 Kohlenstoffatomen darstellen,
R₃ ein Wasserstoffatom, einen linearen oder verzweigten Alkylrest mit 1 bis 6 Kohlenstoffatomen oder einen Rest -COR₄ darstellt, wobei R₄ einen Alkylrest mit 1 bis 6 Kohlenstoffatomen darstellt,
oder 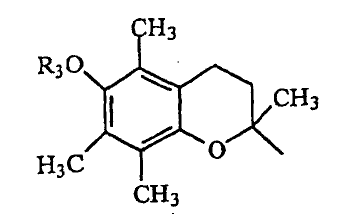 entspricht;
B einen linearen oder verzweigten Alkylrest mit 1 bis 6 Kohlenstoffatomen, Phenyl, Pyridinyl oder einen Heterocyclus mit 5 Kettengliedern darstellt, der 1 bis 4 Heteroatome enthält, die aus O, S, N ausgewählt sind, und insbesondere: Thiophen, Furan, Pyrrol oder Thiazol, deren Kohlenstoffatome ggf. durch eine oder mehrere Gruppen substituiert sind, die aus einem linearen oder verzweigten Alkyl mit 1 bis 6 Kohlenstoffatomen, einem Alkoxyrest mit 1 bis 6 Kohlenstoffatomen oder einem Halogenatom ausgewählt sind;
X -CO-N(R₃)-X'-, -NH-CO-X'-, -CH=, -CO- oder eine Bindung darstellt, wobei X' -(CH₂)ₙ- mit n einer ganzen Zahl von 0 bis 6 darstellt;
Y -Y'-, -CO-NH-Y', -Y'-NH-CO-, -CO-Y'-, -Y'-CO, -N(R₃)-Y'-, -Y'-N(R₃)-, Y'-CH₂-N (R₃) -CO-, -O-Y' -, -Y'-O-, -S-Y'-, -Y'-S-, -Y'-O-Y'-, -Y'-N(R₃)-Y'- oder eine Bindung darstellt, wobei Y' -(CH₂)ₙ- mit n einer ganzen Zahl von 0 bis 6 darstellt;
Het einen Heterocyclus mit 1 bis 5 Heteroatomen darstellt, die aus O, N, S ausgewählt sind und durch einen oder mehrere Substituenten X'-OR₃, X'-NR₃, X'-S-R₃ substituiert sein können, wie z.B.:
Oxetan, Pyrrol, Pyrrolidin, Furan, Tetrahydrofuran, Thiophen, Tetrahydrothiophen, Sulfolan, Imidazol, Imidazolin, Dihydroimidazol-2-on, Dihydroimidazol-2-thion, Oxazol, Isoxazol, Oxazolin, Isoxazolin, Oxazolidin, Oxazolidinon, Thiazol, Thiazolin, Thiazolidin, Thiazolidinon, Hydantoin, 1,2,4-Triazol, 1,3,4-Oxadiazol, 1,3,4-Thiadiazol, 1,1-Dioxid-1,2,5-thiadiazolidin, 1,2,4-Triazol-3-on, Tetrazol, Tetrahydropyridin, Azetidin, mit Ausnahme der folgenden Heterocyclen: Piperazine, Homopiperazine, 4-Aminopiperidin;
G_{P} eine in wasserfreiem saurem Medium abspaltbare Schutzgruppe für die Aminfunktion ist, wie z.B. die Carbamate von t-Butyl, Trichlorethyl oder Trimethylsilylethyl oder auch der Tritylgruppe.

9. Eine Verbindung der allgemeinen Formel (I) nach einem der Ansprüche 1 bis 5 oder ein pharmazeutisch verträgliches Salz dieser Verbindung in Form eines Medikaments.

10. Pharmazeutische Zusammensetzung, die als Wirkstoff mindestens eine Verbindung nach Anspruch 9 enthält.

11. Verwendung einer Verbindung der allgemeinen Formel (I) nach einem der Ansprüche 1 bis 5 oder eines pharmazeutisch verträglichen Salzes dieser Verbindung für die Herstellung eines Medikaments zur Hemmung der NO-Synthase.

12. Verwendung einer Verbindung der allgemeinen Formel (I) nach einem der Ansprüche 1 bis 5 oder eines pharmazeutisch verträglichen Salzes dieser Verbindung für die Herstellung eines Medikaments zur Hemmung der Lipidperoxidation.

13. Verwendung einer Verbindung der allgemeinen Formel (I) nach einem der Ansprüche 1 bis 5 oder eines pharmazeutisch verträglichen Salzes dieser Verbindung für die Herstellung eines Medikaments, das gleichzeitig eine die NO-Synthase hemmende Wirkung und eine die Lipidperoxidation hemmende Wirkung hat.

14. Verwendung einer Verbindung der allgemeinen Formel (I) nach einem der Ansprüche 1 bis 5 oder eines pharmazeutisch verträglichen Salzes dieser Verbindung für die Herstellung eines Medikaments zur Behandlung von kardiovaskulären und zerebrovaskulären Störungen.

15. Verwendung einer Verbindung der allgemeinen Formel (I) nach einem der Ansprüche 1 bis 5 oder eines pharmazeutisch verträglichen Salzes dieser Verbindung für die Herstellung eines Medikaments zur Behandlung von Störungen des zentralen oder peripheren Nervensystems.

## Claims

1. Product **characterized in that** it corresponds to general formula **(I)** 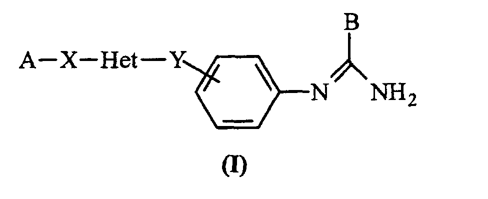 in which:
A is an aromatic corresponding to the structures: 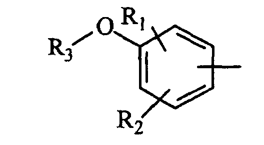 in which R₁ and R₂ represent, independently, a hydrogen atom, a halogen, the OH group, a linear or branched alkyl radical having from 1 to 6 carbon atoms, a linear or branched alkoxy radical having from 1 to 6 carbon atoms,
R₃ represents a hydrogen atom, a linear or branched alkyl radical having from 1 to 6 carbon atoms or a -COR₄ radical, R₄ representing an alkyl radical having from 1 to 6 carbon atoms,
or 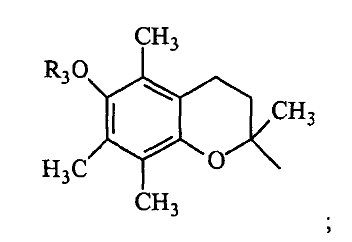
B represents a linear or branched alkyl radical having from 1 to 6 carbon atoms, phenyl, pyridinyl or a heterocycle with 5 members containing from 1 to 4 heteroatoms chosen from O, S, N and more particularly: thiophene, furan, pyrrole or thiazole, the carbons of which are optionally substituted by one or more groups chosen from a linear or branched alkyl radical having from 1 to 6 carbon atoms; an alkoxy radical having from 1 to 6 carbon atoms or a halogen;
X represents -CO-N(R₃)-X'-, -NH-CO-X'-, -CH=, -CO- or a bond,
X' representing -(CH₂)ₙ- with n an integer from 0 to 6;
Y represents -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- or a bond, Y' representing -(CH₂)ₙ- with n an integer from 0 to 6;
Het represents a heterocycle containing from 1 to 5 heteroatoms chosen from O, N, S which can be substituted by one or more substituents X'-OR₃, X'-NR₃, X'-S-R₃ and such as for example:
oxetane, pyrrole, pyrrolidine, furan, tetrahydrofuran, thiophene, tetrahydrothiophene, sulpholane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoine, 1,2,4-triazole, 1,3,4-oxadiazole, 1,3,4-thiadiazole, 1,1-dioxyde-1,2,5-thiadiazolidine, 1,2,4-triazole-3-one, tetrazole, tetrahydropyridine, azetidine, with the exeception of the following heterocycles: piperazines, homopiperazines, 4-aminopiperidine;
or addition salt with acid thereof.

2. Product according to claim 1, **characterized in that**:
A is an aromatic corresponding to the structure: 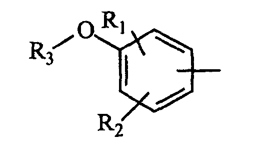
in which:
R₁ and R₂ represent, independently a linear or branched alkyl radical having from 1 to 6 carbon atoms or a linear or branched alkoxy radical having from 1 to 6 carbon atoms,
R₃ represents a hydrogen atom or a linear or branched alkyl radical having from 1 to 6 carbon atoms;
B represents a heterocycle with 5 members containing from 1 to 4 heteroatoms chosen from O, S, N and more particularly: thiophene, furan, pyrrole or thiazole, the carbons of which are optionally substituted by one or more groups chosen from a linear or branched alkyl having from 1 to 6 carbon atoms, an alkoxy radical having from 1 to 6 carbon atoms or a halogen;
X represents -NH-CO-X'-, -CH=, -CO- or a bond,
X' representing -(CH₂)ₙ- with n an integer from 0 to 6;
Y represents -Y'-, -Y'-NH-CO-, -Y'-CO-, -Y'-O-, -Y'-O-Y'-, -Y'-N(R₃)-Y'- or a bond, Y' representing -(CH₂)ₙ- with n an integer from 0 to 6;
Het represents a heterocycle containing from 1 to 5 heteroatoms chosen from O, N, S which can be substituted by one or more substituents X'-OR₃, X'-NR₃, X'-S-R₃ and such as for example:
oxetane, pyrrole, pyrrolidine, furan, tetrahydrofuran, thiophene, tetrahydrothiophene, sulpholane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoin, 1,2,4-triazole, 1,3,4-oxadiazole, 1,3,4-thiadiazole, 1,1-dioxyde-1,2,5-thiadiazolidine, 1,2,4-triazole-3-one, tetrazole, tetrahydropyridine, azetidine, with the exception of the following heterocycles: piperazines, homopiperazines, 4-aminopiperidine
or an addition salt with acid thereof.

3. Product according to claim 2, **characterized in that** B represents a thiophene ring, the carbons of which are optionally substituted by one or more groups chosen from a linear or branched alkyl having from 1 to 6 carbon atoms, an alkoxy radical having from 1 to 6 carbon atoms or a halogen ;
or addition salt with acid thereof.

4. Product according to one of claims 1 to 3, **characterized in that** it is one of the following compounds:
- N-(3,5-di-t-butyl-4-hydroxyphenyl)-5-[4-{imino(2-thienyl)-methylamino} phenyl]-2-furan carboxamide hydroiodide;
- 3-(3,5-di-t-butyl-4-hydroxyphenyl)-1-[4-{imino(2-thienyl)-methylamino} phenyl]-2,5-imidazolidinedione hydrochloride;
- 2-(3,5-di-t-butyl-4-hydroxyphenyl)-3-[4-{imino(2-thienyl)-methylamino} phenyl]-4-thiazolidinone hydrochloride;
- 5-[(3,5-di-t-butyl-4-hydroxyphenyl)methylene]-1-methyl-3-[4-{imino(2-thienyl) methylamino}phenyl]-2,4-imidazolidinedione fumarate;
- 2-(*S*)-4-(*S*)-N-[4-hydroxy-3,5-bis-(1,1-dimethylethyl)-phenyl]-4-{4-[(imino(2-thienyl)methyl)amino]phenoxy}-prolinamide hydrochloride;
- N-[4-hydroxy-3,5-bis-(1,1-dimethylethyl)phenyl]-2-(*R,S*)-{4-[(imino(2-thienyl) methyl)amino]phenyl}-4-(*R*)-thiazolidine carboxamide fumarate;
- N-[3,5-bis(1,1-dimethylethyl)-4-hydroxyphenyl]-2-{4-[(imino(2-thienyl) methyl)amino]phenyl}-4-thiazolecarboxamide hydroiodide
- N-[3,5-bis(1,1-dimethylethyl)-4-hydroxyphenyl]-4-(S)-{4-[(imino(2-thienyl)methyl)amino]phenoxy}-pyrrolidine-2-(R)-carboxamide dihydrochloride
- methyl 1-[(3,4-dihydro-6-hydroxy-2,5,7,8-tetramethyl-2-H-[1]-benzopyran-2-yl)carbonyl]-4-(S)-{4-[(imino(2-thienyl)methyl)amino]-phenoxy}-pyrrolidine-2-(S)-carboxylate hydrochloride
- 1-[(3,4-dihydro-6-hydroxy-2,5,7,8-tetramethyl-2H-[1]-benzopyran-2-yl) carbonyl]-3-(S)-{4-[(imino(2-thienyl)methyl)amino]phenoxy}-pyrrolidine hydrochloride
- 3-{[(3,4-dihydro-6-hydroxy-2,5,7,8-tetramethyl-2H-[1]-benzopyran-2-yl)-carbonyl]amino}-1-{4-[(imino(2-thienyl)methyl)amino]phenyl}pyrrolidine
- 4-[3,5-bis-(1,1-dimethylethyl)-4-hydroxyphenyl]-N-{4-[(imino(2-thienyl) methyl)amino]benzoyl}-N-methyl-1H-imidazole-2-methanamine hydrochloride
- N-[3,5-bis-(1,1-dimethylethyl)-4-hydroxyphenyl]-1-{4-[(imino(2-thienyl) methyl)amino]phenyl}-1H-pyrrole-2-carboxamide hydroiodide
- 1-[3,5-bis(1,1-dimethylethyl)-4-hydroxyphenyl]-3-{[4-[[imino(2-thienyl) methyl]amino]phenyl]carbonyl}-2-imidazolidinone hydroiodide
- 3-[3,5-bis(1,1-dimethylethyl)-4-hydroxyphenyl]-4,5-dihydro-N-{4-[(imino(2-thienyl)methyl)amino]phenyl}-5-isoxazoleacetamide hydroiodide
- 4-[3,5-bis(1,1-dimethylethyl)-4-hydroxyphenyl]-N-{4-[(imino(2-thienyl) methyl)amino]phenyl}-N-methyl-2-thiazolemethanamine hydrochloride
- 4-[3,5-bis(1,1-dimethylethyl)-4-hydroxyphenyl]-N-{4-[(imino(2-thienyl) methyl)amino]phenyl}-N-methyl-1H-imidazole-2-methanamine hydrochloride
- 3-[3,5-bis(1,1-dimethylethyl)-4-hydroxyphenyl]-4,5-dihydro-5-{2-{4-[(imino(2-thienyl)methyl)amino]phenoxy}ethyl}isoxazole
- 1-{[3,5-bis(1,1-dimethylethyl)-4-hydroxyphenyl]amino}-carbonyl}-3-{4-[(imino(2-thienyl)methyl)amino]phenoxy}azetidine hydrochloride
- 1-(2-hydroxy-5-methoxybenzoyl)-3-{4-[(imino(2-thienyl)methyl)amino] phenoxy}azetidine hydrochloride
- 1-[(3,4-dihydro-6-hydroxy-2,5,7,8-tetramethyl-2H-[1]-benzopyran-2-yl) carbonyl]-4-[4-[(imino(2-thienyl)methyl)amino]phenoxy}-piperidine hydrochloride
- 1-[(3,4-dihydro-6-hydroxy-2,5,7,8-tetramethyl-2H-[1]-benzopyran-2-yl) carbonyl]-3-{4-[(imino(2-thienyl)methyl)amino]-phenoxy}azetidine hydrochloride
or their salts or enantiomers.

5. Product according to any one of claims 1 to 4, **characterized in that** it is one of the following compounds:
- 3-(3,5-di-t-butyl-4-hydroxyphenyl)-1-[4-{imino(2-thienyl)-methylamino} phenyl]-2,5-imidazolidinedione hydrochloride;
- 2-(3,5-di-t-butyl-4-hydroxyphenyl)-3-[4-{imino(2-thienyl)-methylamino} phenyl]-4-thiazolidinone hydrochloride;
- 5-[(3,5-di-t-butyl-4-hydroxyphenyl)methylene]-1-methyl-3-[4-{imino(2-thienyl)methylamino}phenyl]-2,4-imidazolidinedione fumarate;
- 2-(*S*)-4-(*S*)-N-[4-hydroxy-3,5-bis-(1,1-dimethylethyl)-phenyl]-4-{4-[(imino(2-thienyl)methyl)amino]phenoxy}-prolinamide hydrochloride;
- N-[4-hydroxy-3,5-bis-(1,1-dimethyl)ethyl-phenyl]-2-{4-[(imino(2-thienyl) methyl)amino]phenyl}-4-thiazole carboxamide hydrochloride;
or their salts or enantiomers.

6. As new industrial products, the compounds of general formula **(V), (VI)** and **(VII)** 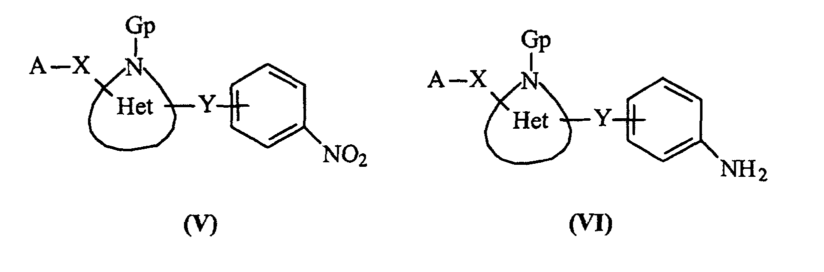 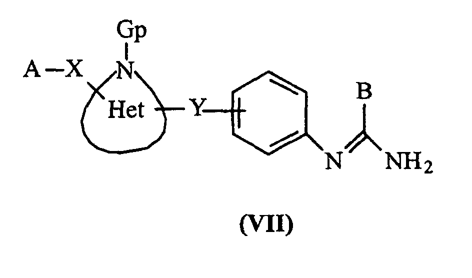 in which
A is an aromatic corresponding to structures : 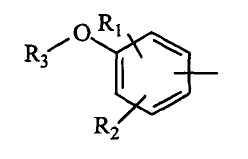 in which:
R₁ and R₂ represent, independently, a hydrogen atom, a halogen, the OH group, a linear or branched alkyl radical having from 1 to 6 carbon atoms, a linear or branched alkoxy radical having from 1 to 6 carbon atoms,
R₃ represents a hydrogen atom, a linear or branched alkyl radical having from 1 to 6 carbon atoms or a -COR₄ radical, R₄ representing an alkyl radical having from 1 to 6 carbon atoms,
or 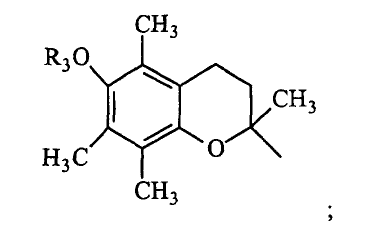 B represents a linear or branched alkyl radical having from 1 to 6 carbon atoms, phenyl, pyridinyl or a heterocycle with 5 members containing from 1 to 4 heteroatoms chosen from O, S, N and more particularly: thiophene, furan, pyrrole or thiazole, the carbons of which are optionally substituted by one or more groups chosen from a linear or branched alkyl having from 1 to 6 carbon atoms, an alkoxy radical having from 1 to 6 carbon atoms or a halogen;
X represents -CO-N(R₃)-X'-, -NH-CO-X'-, -CH=, -CO- or a bond,
X' representing -(CH₂)ₙ- with n an integer from 0 to 6;
Y represents -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- or a bond, Y' representing -(CH₂)ₙ- with n an integer from 0 to 6;
Het represents a heterocycle containing from 1 to 5 heteroatoms chosen from O, N, S which can be substituted by one or more substituents X'-OR₃, X'-NR₃, X'-S-R₃ and such as for example:
oxetane, pyrrole, pyrrolidine, furan, tetrahydrofuran, thiophene, tetrahydrothiophene, sulpholane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoin, 1,2,4-triazole, 1,3,4-oxadiazole, 1,3,4-thiadiazole, 1,1-dioxyde-1,2,5-thiadiazolidine, 1,2,4-triazole-3-one, tetrazole, tetrahydropyridine, azetidine, with the exception of the following heterocycles: piperazines, homopiperazines, 4-aminopiperidine;
Gₚ represents a protective group of the amine function cleavable in an anhydrous acid medium.

7. Preparation process for the compounds of general formula **(I)** according to claim 1, **characterized in that** it consists of the condensation, preferably in a mixture of isopropanol and DMF and at ambient temperature, of the aniline derivatives of general formula **(III)** 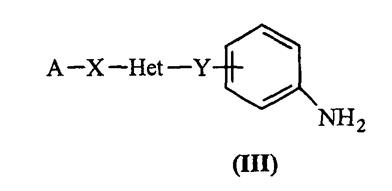 on the S-alkyl thioimidate derivatives of general formula **(IV)** 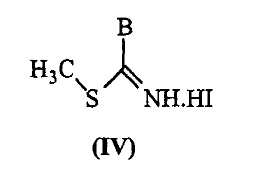 in order to produce the final compounds of general formula **(I)**,
the compounds of general formula **(I), (III)** and **(IV)** such that
A is an aromatic corresponding to structures : 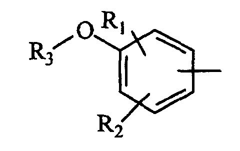 R₁ and R₂ represent, independently, a hydrogen atom, a halogen, the OH group, a linear or branched alkyl radical having from 1 to 6 carbon atoms, a linear or branched alkoxy radical having from 1 to 6 carbon atoms,
R₃ represents a hydrogen atom, a linear or branched alkyl radical having from 1 to 6 carbon atoms or a -COR₄ radical, R₄ representing an alkyl radical having from 1 to 6 carbon atoms,
or 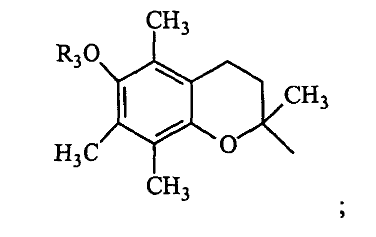 B represents a linear or branched alkyl radical having from 1 to 6 carbon atoms, phenyl, pyridinyl or a heterocycle with 5 members containing from 1 to 4 heteroatoms chosen from O, S, N and more particularly: thiophene, furan, pyrrole or thiazole, the carbons of which are optionally substituted by one or more groups chosen from a linear or branched alkyl having from 1 to 6 carbon atoms, an alkoxy radical having from 1 to 6 carbon atoms or a halogen;
X represents -CO-N(R₃)-X'-, -NH-CO-X'-, -CH=, -CO- or a bond,
X' representing -(CH₂)ₙ- with n an integer from 0 to 6;
Y represents -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- or a bond, Y' representing -(CH₂)ₙ- with n an integer from 0 to 6;
Het represents a heterocycle containing from 1 to 5 heteroatoms chosen from O, N, S which can be substituted by one or more substituents X'-OR₃, X'-NR₃, X'-S-R₃ and such as for example:
oxetane, pyrrole, pyrrolidine, furan, tetrahydrofuran, thiophene, tetrahydrothiophene, sulpholane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoin, 1,2,4-triazole, 1,3,4-oxadiazole, 1,3,4-thiadiazole, 1,1-dioxyde-1,2,5-thiadiazolidine, 1,2,4-triazole-3-one, tetrazole, tetrahydropyridine, azetidine, with the exception of the following heterocycles: piperazines, homopiperazines, 4-aminopiperidine;

8. Preparation process for the compounds of general formula **(I)** according to claim 1 heterocycle Het of which contains at least one nitrogen atom, **characterized in that** it includes the following two stages:
- the condensation, preferably in an isopropanol and DMF mixture and at ambient temperature, of a compound of general formula **(VI)** 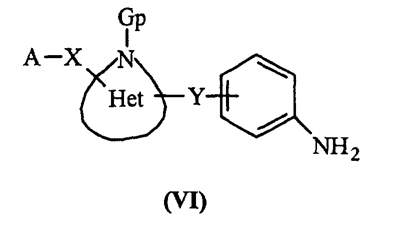 with a compound of general formula **(IV)** 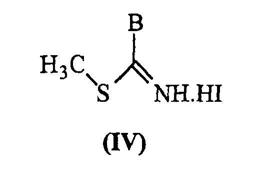 in order to produce a compound of general formula **(VII)** 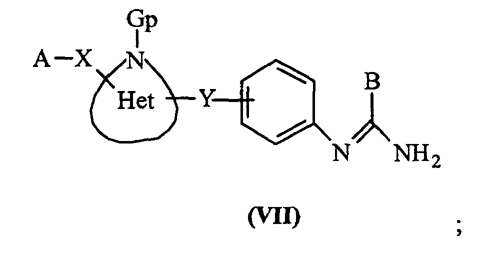
- the cleavage of the protective group Gₚ of the compound of general formula **(VII)** defined above in order to obtain the compound of general formula **(I),**
the compounds of general formula **(I), (IV), (VI)** and **(VII)** being such that
A is an aromatic corresponding to structures : 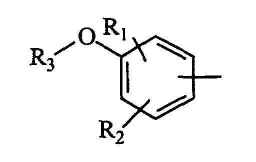
in which :
R₁ and R₂ represent, independently, a hydrogen atom, a halogen, the OH group, a linear or branched alkyl radical having from 1 to 6 carbon atoms, a linear or branched alkoxy radical having from 1 to 6 carbon atoms,
R₃ represents a hydrogen atom, a linear or branched alkyl radical having from 1 to 6 carbon atoms or a -COR₄ radical R₄ representing an alkyl radical having 1 to 6 carbon atoms,
or 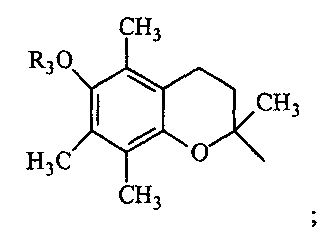 B represents a linear or branched alkyl radical having from 1 to 6 carbon atoms, phenyl, pyridinyl or a heterocycle with 5 members containing from 1 to 4 heteroatoms chosen from O, S, N and more particularly: thiophene, furan, pyrrole or thiazole, the carbons of which are optionally substituted by one or more groups chosen from a linear or branched alkyl having from 1 to 6 carbon atoms, an alkoxy radical having from 1 to 6 carbon atoms or a halogen;
X represents -CO-N(R₃)-X'-, -NH-CO-X'-, -CH=, -CO- or a bond,
X' representing -(CH₂)ₙ- with n an integer from 0 to 6;
Y represents -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- or a bond, Y' representing -(CH₂)ₙ- with n an integer from 0 to 6;
Het represents a heterocycle containing from 1 to 5 heteroatoms chosen from O, N, S which can be substituted by one or more substituents X'-OR₃, X'-NR₃, X'-S-R₃ and such as for example:
oxetane, pyrrole, pyrrolidine, furan, tetrahydrofuran, thiophene, tetrahydrothiophene, sulpholane, imidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoin, 1,2,4-triazole, 1,3,4-oxadiazole, 1,3,4-thiadiazole, 1,1-dioxyde-1,2,5-thiadiazolidine, 1,2,4-triazole-3-one, tetrazole, tetrahydropyridine, azetidine, with the exception of the following heterocycles: piperazines, homopiperazines, 4-aminopiperidine;
Gₚ represents a protective group of the amine function preferably cleavable in an anhydrous acid medium, such as for example the carbamates of t-butyl, trichloroethyl or trimethylsilylthyl or also the trityl group.

9. As a medicament, a product of general formula **(I)** according to any one of claims 1 to 5, or a pharmaceutically acceptable salt of this product.

10. Pharmaceutical composition containing as active ingredient at least one product according to claim 9.

11. Use of a product of general formula **(I)** according to any one of claims 1 to 5, or a pharmaceutically acceptable salt of this product, to manufacture a medicament intended to inhibit NO synthase.

12. Use of a product of general formula **(I)** according to any one of claims 1 to 5, or a pharmaceutically acceptable salt of this product, to manufacture a medicament intended to inhibit lipidic peroxidation.

13. Use of a product of general formula **(I)** according to any one of claims 1 to 5, or a pharmaceutically acceptable salt of this product, to manufacture a medicament having both an activity on the inhibition of NO synthase and the inhibition of lipidic peroxidation.

14. Use of a product of general formula **(I)** according to any one of claims 1 to 5, or a pharmaceutically acceptable salt of this product, to manufacture a medicament intended to treat cardio-vascular and cerebro-vascular disorders.

15. Use of a product of general formula **(I)** according to any one of claims 1 to 5, or a pharmaceutically acceptable salt of this product, to manufacture a medicament intended to treat disorders of the central or peripheral nervous system.
